# EUROPEAN PATENT APPLICATION

(11) **EP 4 435 115 A1**
(43) Date of publication of application: **25.09.2024**
(21) Application number: 23382281.6
(22) Date of filing: 24.03.2023
(51) Int. Cl.: C12Q 1/6844

(54) **IMPROVED PADLOCK PROBES FOR NUCLEIC ACID DETECTION BY HYPERBRANCHED ROLLING CIRCLE AMPLIFICATION**

(71) Applicant: Fundación del Sector Público Estatal Centro Nacional de Investigaciones Oncológicas Carlos III (F.S.P. CNIO), 28029 Madrid (ES)
(72) Inventor: CORTÉS LEDESMA, Felipe, 28029 Madrid (ES); GARCÍA EXPÓSITO, Laura, 28029 Madrid (ES); LÓPEZ DE ALBA, Ernesto, Madrid 28029 (ES); MUÑOZ BARRERA, Marta, 28029 Madrid (ES); GÓMEZ MARÍN, Carlos, 28029 Madrid (ES)
(74) Representative: Hoffmann Eitle

(57) **Abstract**

The present invention relates to a HRCA method comprising the steps of a) providing a nucleic acid structure formed by the hybridization of at least one padlock with a target nucleic acid, b) circularising the hybridised padlock, c) subjecting the ligated circularized padlock probe to rolling circle amplification (RCA), and optionally a MDA reaction using short primers, and d) detecting the amplification product from step c) preferably with a sequencespecific detection method, thereby detecting the target nucleic acid.

## Description

### TECHNICAL FIELD

The present invention relates to the field of molecular biology. Particularly, the present invention relates to the field of amplification and detection methods using Hyperbranched Rolling Circle Amplification.

### BACKGROUND ART

Rolling circle amplification (RCA) is an enzymatic process where a short DNA or RNA primer is amplified to form a long single stranded DNA or RNA using a circular single stranded nucleic acid template and DNA or RNA polymerases with strand displacement ability. This method is used for amplification of DNA or RNA target molecules, where specially designed padlock probes hybridize to the target nucleic acid, whereby the two ends of the probe become juxtaposed and are joined by a ligase, forming the circular single stranded nucleic acid that will be the template for the amplification. The addition of a polymerase and nucleotides starts the polymerisation, that can be carried out under isothermal conditions. As the circular single stranded nucleic acid template is endless, the resultant product of the RCA is a long single stranded nucleic acid molecule composed of tandem repeats, or monomers, that are complementary to said nucleic acid template (i.e., a concatemer).

The RCA reaction thus produces a linear amplification of DNA, as each circular template grows at a given speed for a certain amount of time. To increase yield and achieve exponential amplification, several approaches have been investigated. One of them is the Hyperbranched Rolling Circle Amplification (HRCA), where primers that anneal, each to the RCA product and to the reverse complementary sequence are added, and also extended in a further amplification step, which can also be carried out under isothermal conditions (Multiple Displacement Amplification; MDA). In this way, the original RCA creates more template that can be amplified and subsequently detected. Methods to detect nucleic acid are widely known, and include the use of DNA dyes, beacons probes, electrochemical assays and CRISPR-Cas based methods.

Since its first introduction, HRCA using padlock probes has been proven to be a robust DNA amplification technique for detecting pathogens and other applications, allowing rapid detection of nucleic-acid sequences with high specificity. This method has been adopted as the basis for amplifying and detecting linear and circular nucleic acid molecules in laboratory settings.

Despite the growth of research on point-of-care (POC) tests, most devices never leave the laboratory. This is especially relevant in the case of diagnostic tests, as the COVID-19 pandemic has highlighted the importance of developing rapid testing and diagnosis for efficient epidemic control. Hence, it is desired that methods such as RCA and HRCA are modified and optimized to be moved from the bench to the field in low-cost, point of care diagnostics. However, this represents a challenge as several hurdles need to be overcome. On the one hand, unspecific amplifications resulting for circularised padlock probes that did not hybridize with the target nucleic acid give rise to background noise that mask positive signals. On the other hand, unspecific hybridizations at the low temperatures required in POC testing also result in target-independent amplification. The detection method should therefore efficiently discriminate this background amplification from specific target-dependent amplification. Importantly, while the method should be adapted to be rapid, portable, simple, and low cost, this adaptation should not be detrimental on the specificity and accuracy of the method.

The present invention focuses on padlock design and an improved HRCA method and provides a new method for the rapid, low cost, but specific and efficient, detection of target nucleic acids.

### BRIEF DESCRIPTION OF DRAWINGS

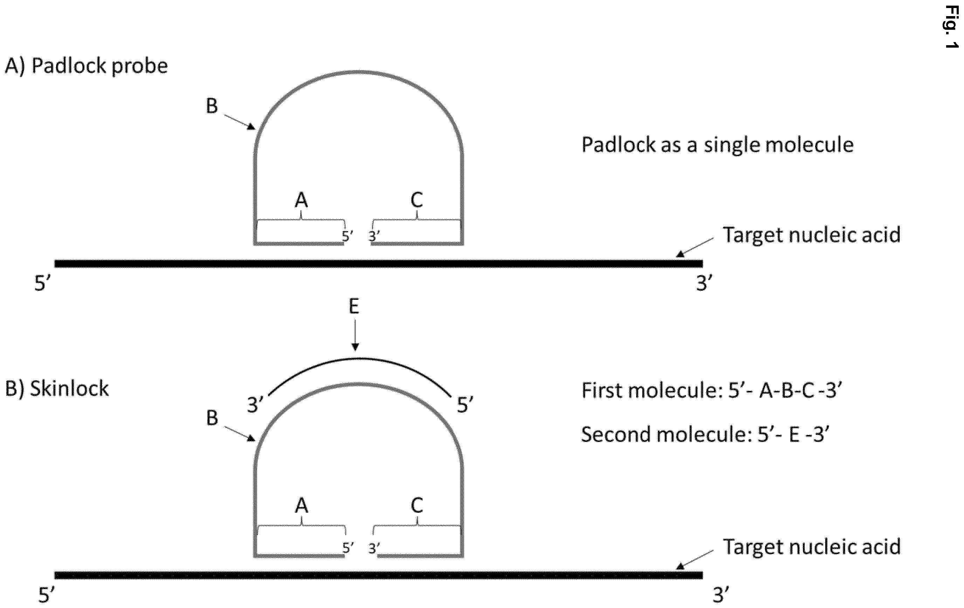

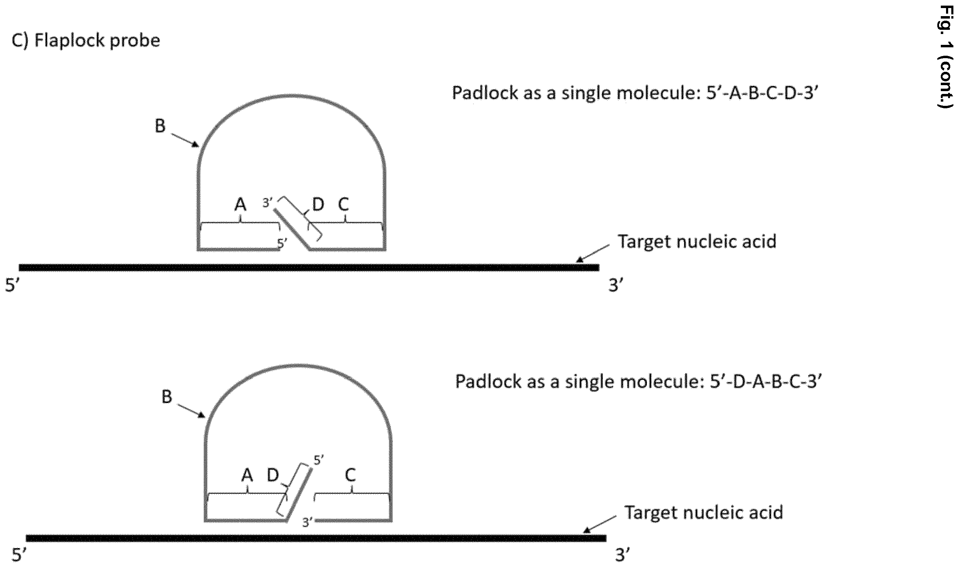

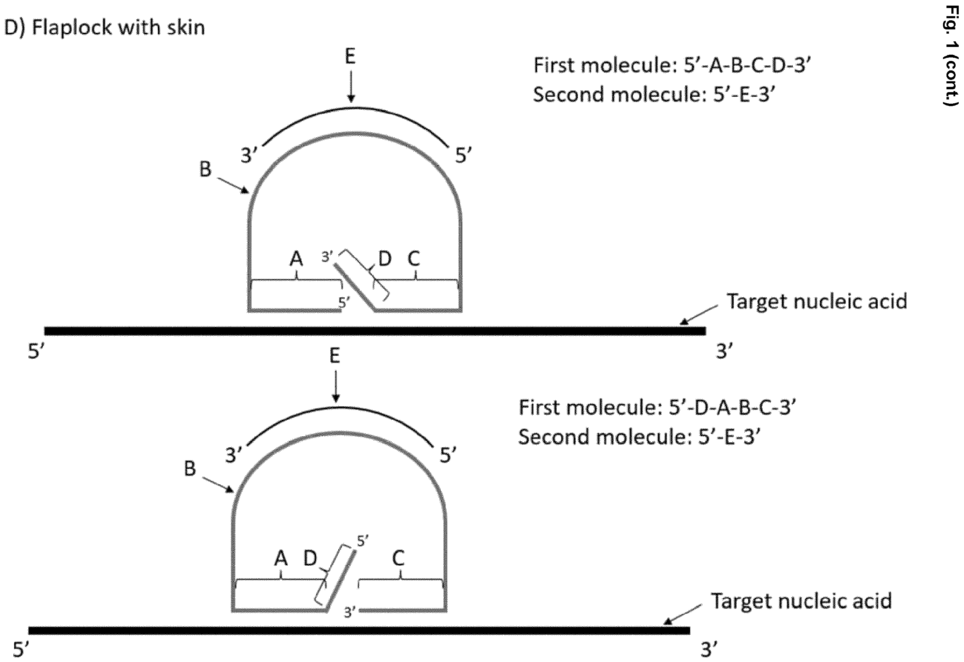

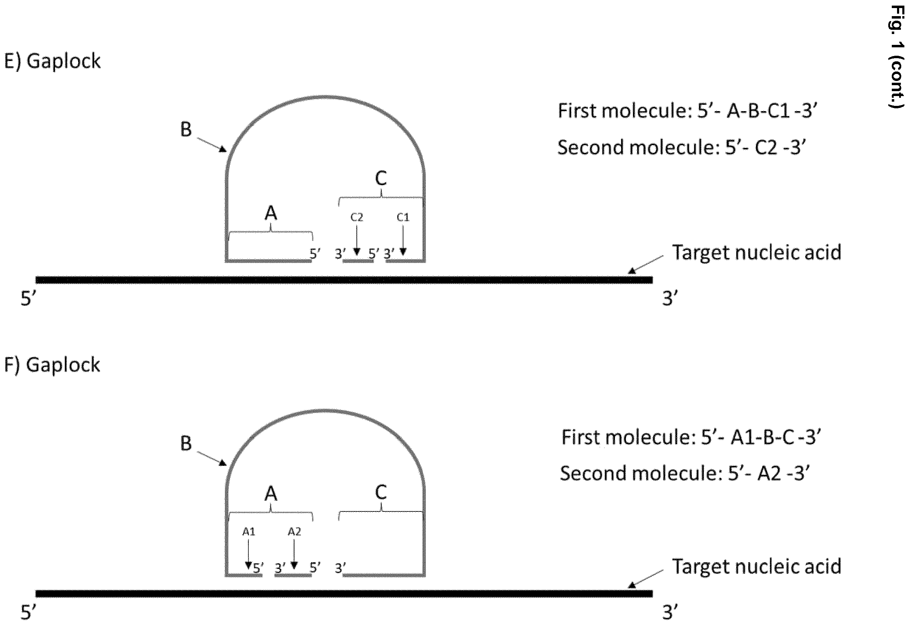

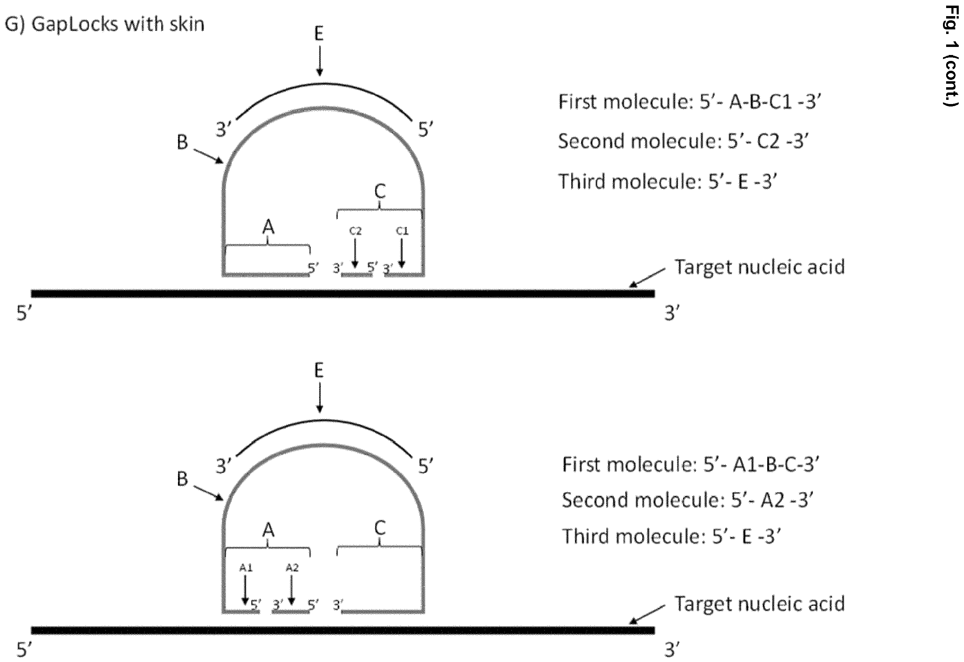

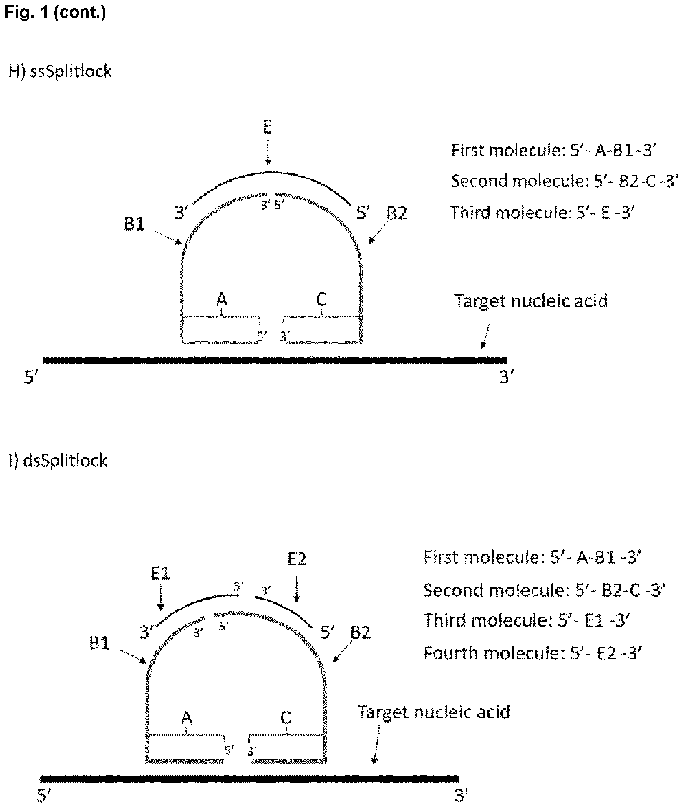

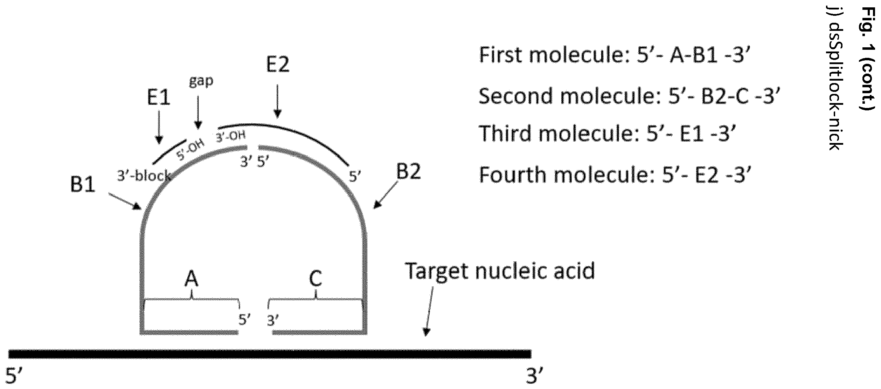

**Fig. 1****: (A to J) Preferred padlocks of the present invention.**

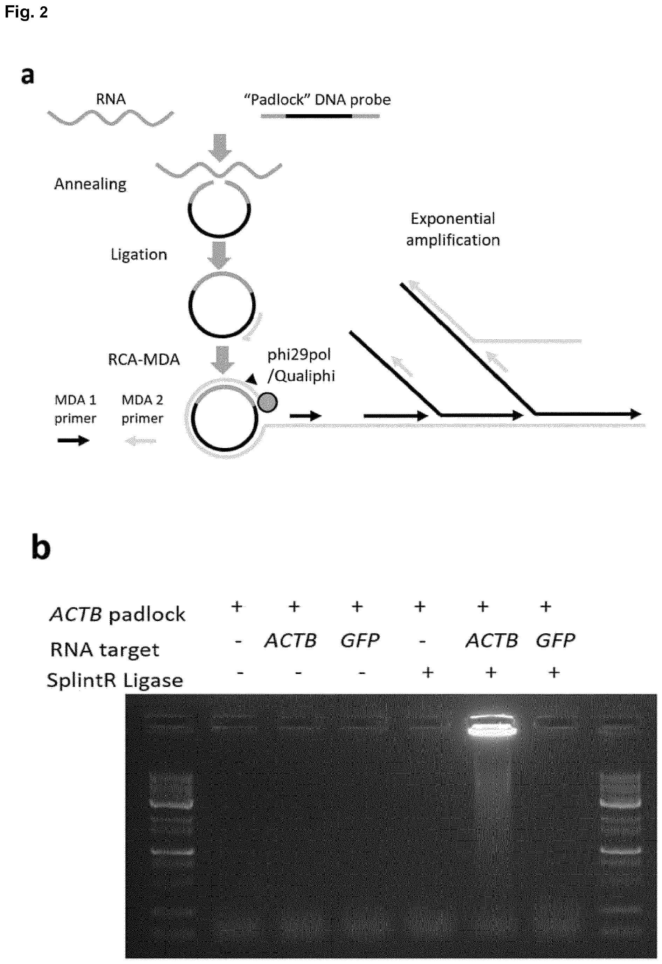

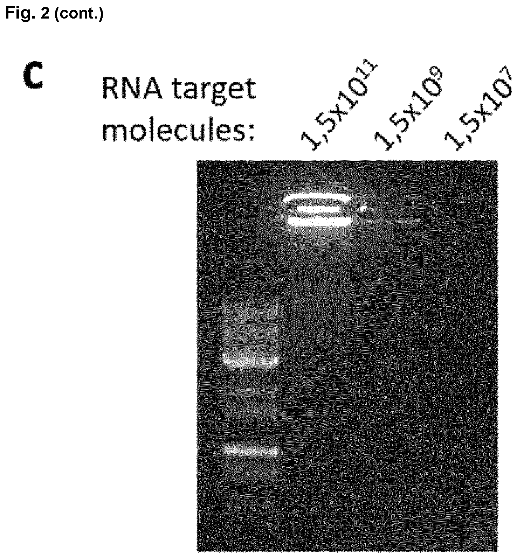

**Fig. 2****: HRCA-mediated RNA detection with padlock probes and HRCA. (a)** Scheme of the basic method, which uses padlock probes to specifically generate circles in the presence of target RNA and amplify this product by RCA-MDA, a.k.a. hyperbranched RCA (HRCA). RCA reactions can also be primed by the target RNA. The differential advantage of using phi29pol/Qualiphi is the capacity to conduct reactions at room temperature. **(b)** Proof-of-principle experiment. A padlock targeting the human *ACTB* gene was incubated with the indicated synthetic RNA as described in example 1 in the presence and absence of SplintR Ligase. Amplified material was subjected to electrophoresis in a 0.8% agarose gel and visualized with Gelred staining. **(c)** Target titration. The experiment was performed as described in Example 2. The human ACTB padlock probe was incubated in the presence of the indicated amounts of synthetic ACTB target RNA. Amplified material was subjected to electrophoresis in a 0.8% agarose gel and visualized with Gelred staining.

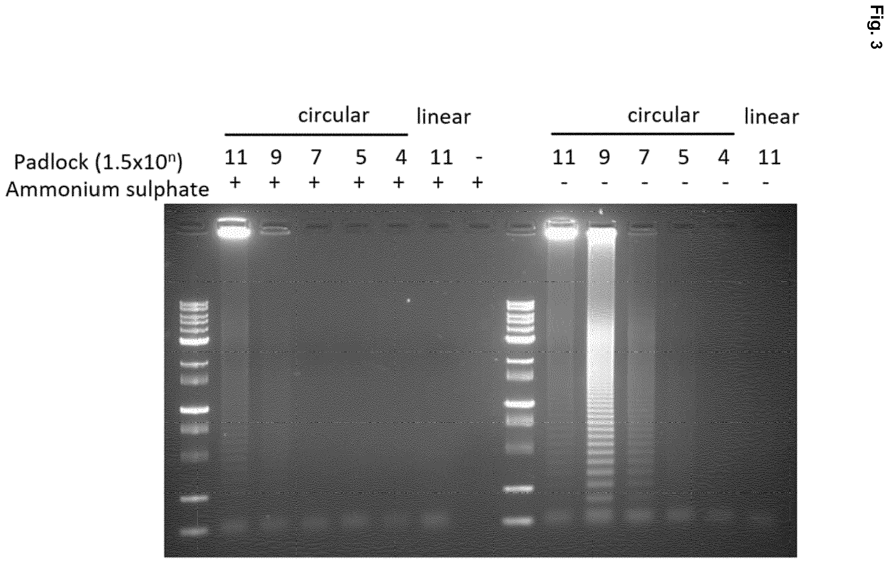

**Fig. 3****: Optimization of HRCA conditions: buffer.** The experiment was performed as described in Example 3. The indicated amount of pre-ligated (circular) or linear ACTB padlock probe was subject to an HRCA reaction. Amplified material was subjected to electrophoresis in a 0.8% agarose gel and visualized with Gelred staining.

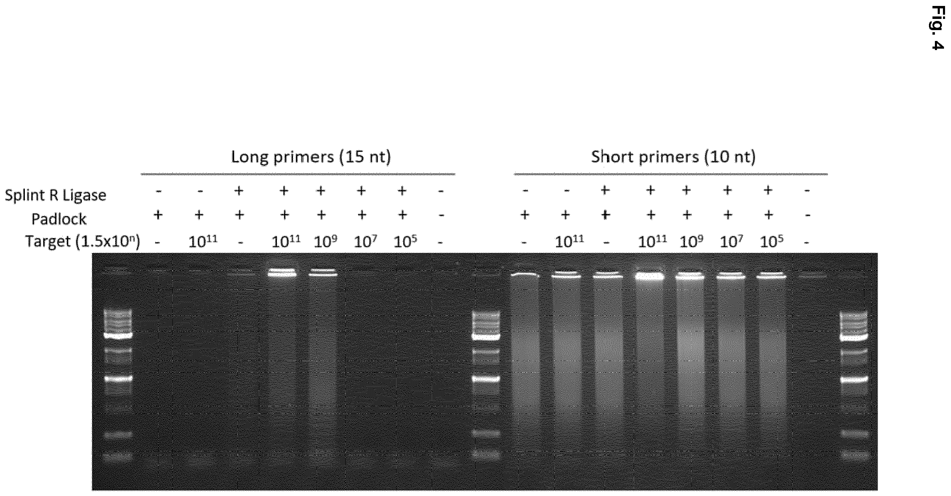

**Fig. 4****: Optimization of HRCA conditions: primers.** The experiment was performed as described in Example 4. The HRCA reactions included the indicated amount ACTB RNA with and without the presence of ACTB padlock and/or SplintR ligase, as indicated. Amplified material was subjected to electrophoresis in a 0.8% agarose gel and visualized with Gelred staining.

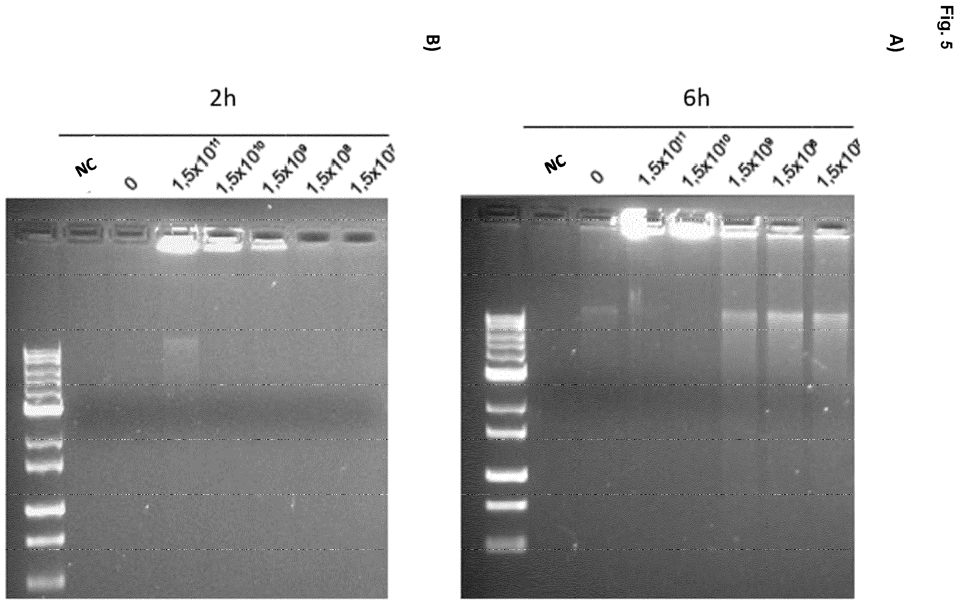

**Fig. 5****: HRCA with wild-type phi29pol.** The experiment was performed as described in Example 5. The HRCA reactions included the indicated amount ACTB RNA. A negative control (NC) without ACTB padlock was included. Amplification was carried out for 2 (B) and 6h (A) as indicated. Amplified material was subjected to electrophoresis in a 0.8% agarose gel and visualized with Gelred staining.

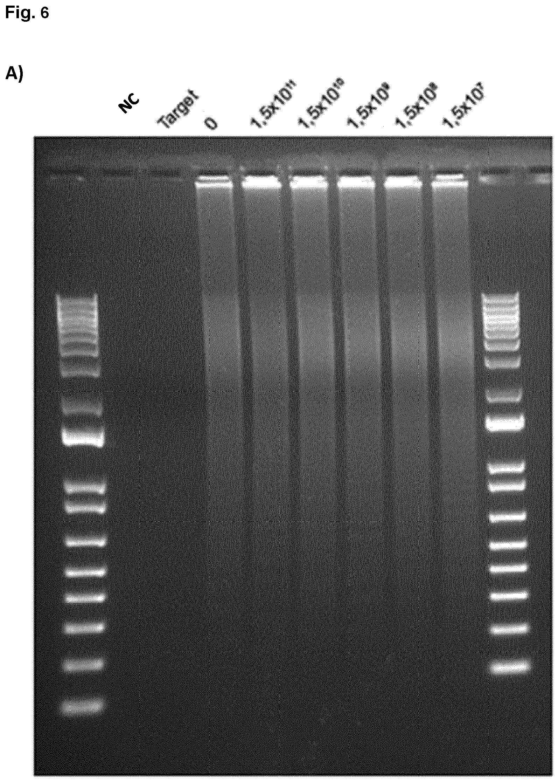

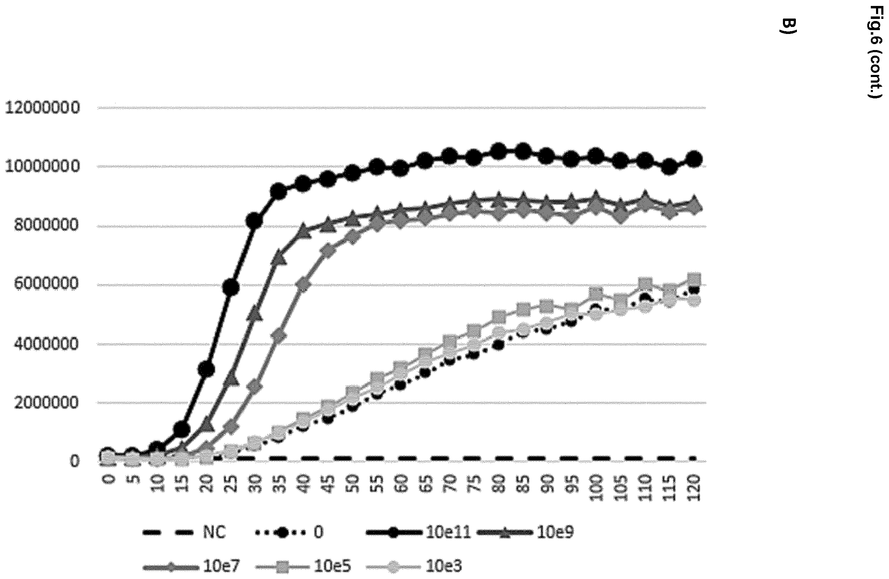

**Fig. 6****: HRCA coupled with CRISPR-Cas12a detection,** (a) HRCA-CRISPR-Cas12a detection performed as described in Example 6. Reactions contain the indicated amount of target RNA. Negative controls without padlock, but containing the maximum amount of target (target), and without padlock or target (NC, negative control) are included. Total amplified material, as detected by agarose gel electrophoresis (A), and as specifically detected by CRISPR-Cas12a of the reporter substrate (B), are shown.

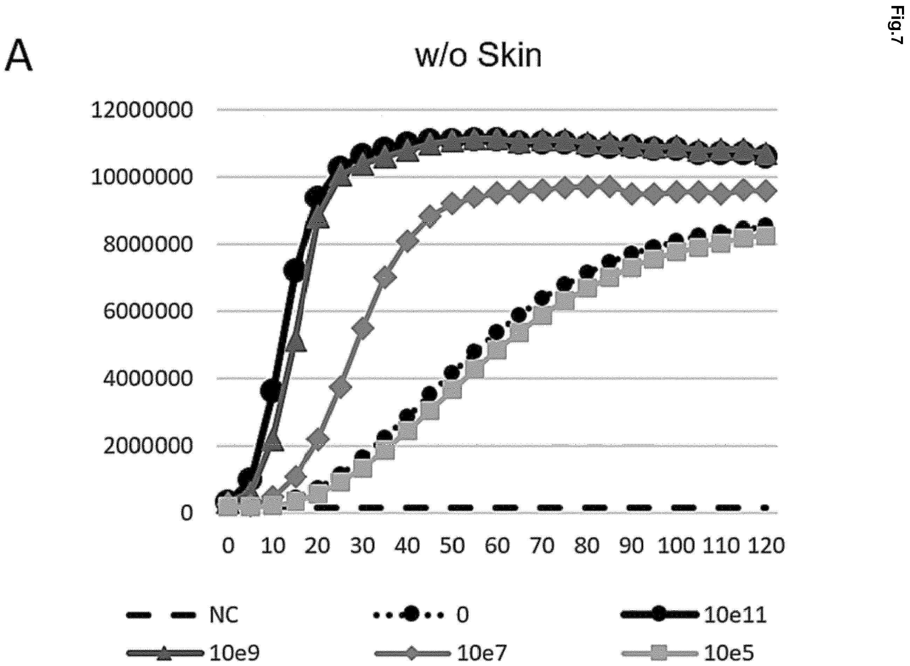

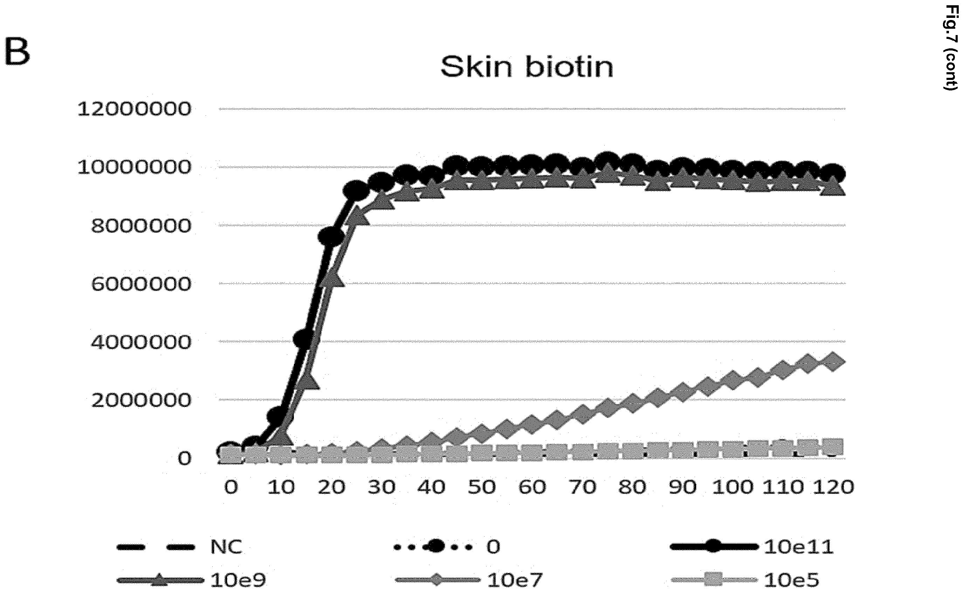

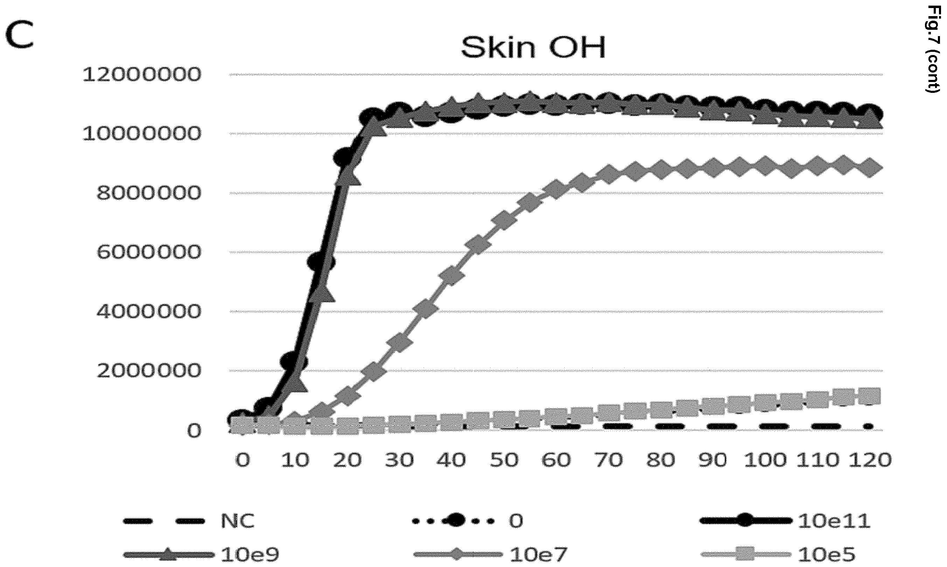

**Fig. 7****: Improved padlocks for HRCA CRISPR-Cas12a: Skin.** The HRCA reactions were carried out as described in Example 7 with the indicated amounts of target RNA, and S gene padlocks not containing (A), or containing a skin with a blocking 3' biotin (B) or a 3' OH (C). A negative control without padlock or target (NC, negative control) was included.

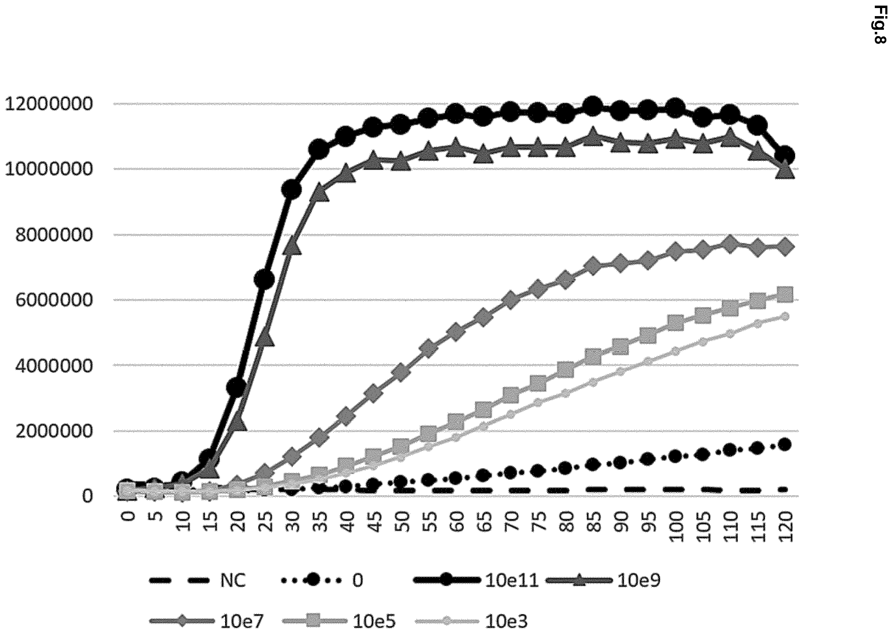

**Fig. 8****: Improved padlocks for HRCA CRISPR-Cas12a: FLAP and Exol**. The HRCA reactions were carried out as described in Example 8, including a treatment with Exol. The indicated amounts of target RNA were incubated with S gene padlocks containing a poly-A extension on the 3' end. A negative control without padlock or target (NC) was included.

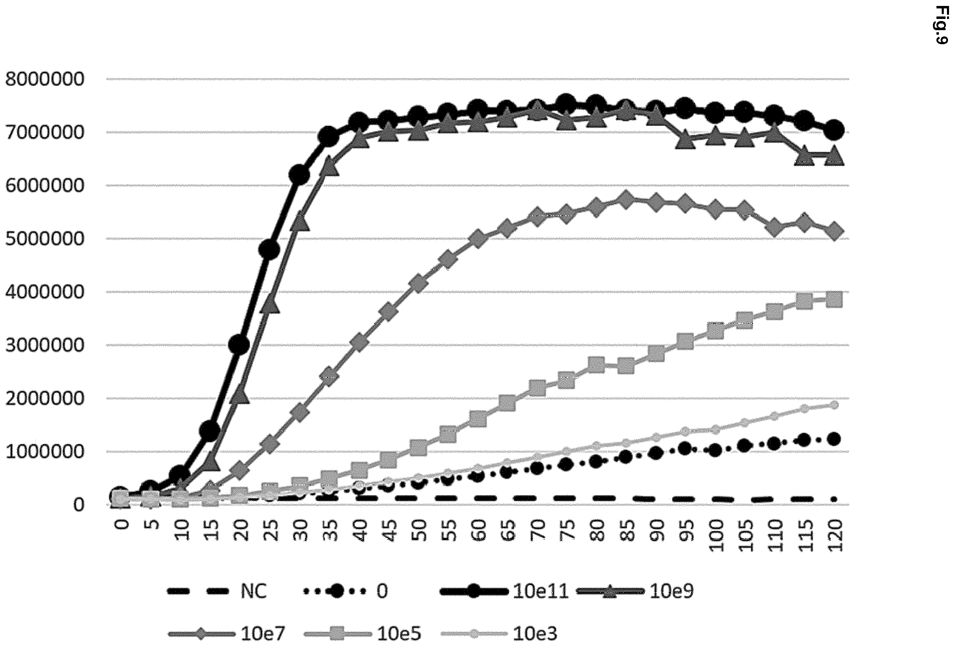

**Fig. 9****: Improved padlocks for HRCA CRISPR-Cas12a: FLAP.** The HRCA reactions were carried out as described in Example 9. The indicated amounts of target RNA were incubated with S gene padlocks. A negative control without padlock or target (NC) was included.

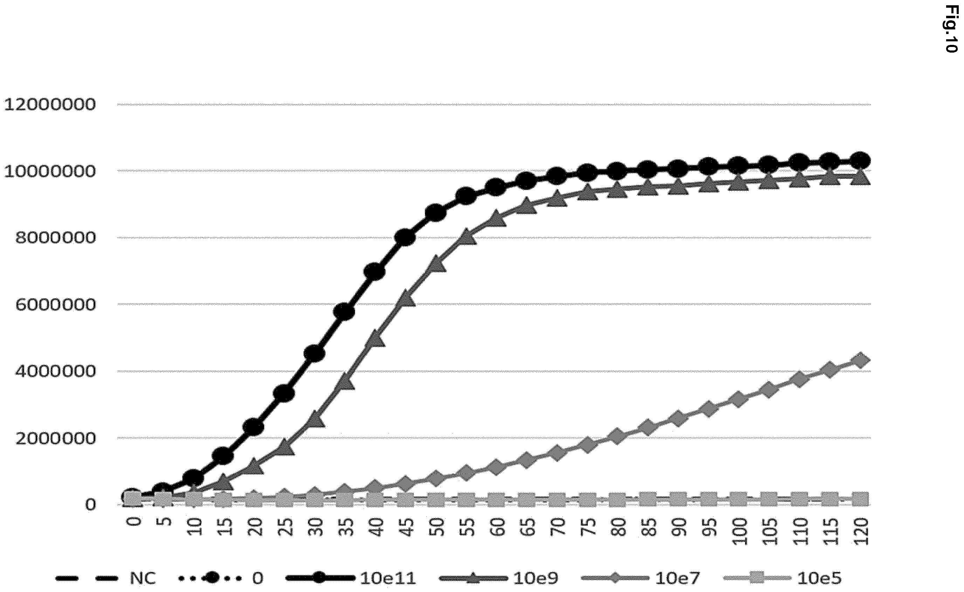

**Fig. 10****: Improved padlocks for HRCA CRISPR-Cas12a: GAP.** The HRCA reactions were carried out as described in Example 10 and depicted on the left. The indicated amounts of target RNA were incubated with S gene padlocks containing a gap in the breakpoint junction, which was filled-in by an additional oligonucleotide. A negative control without padlock or target (NC) was included.

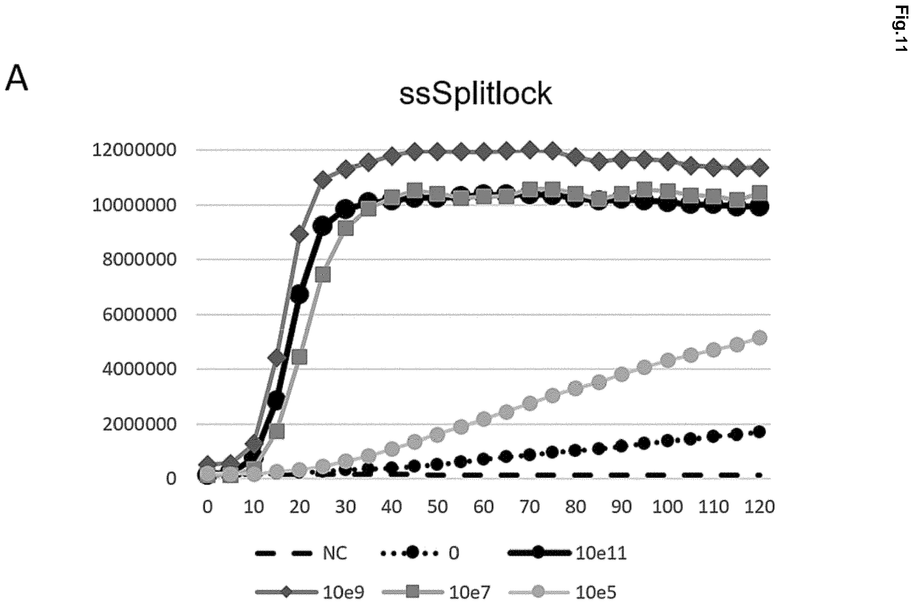

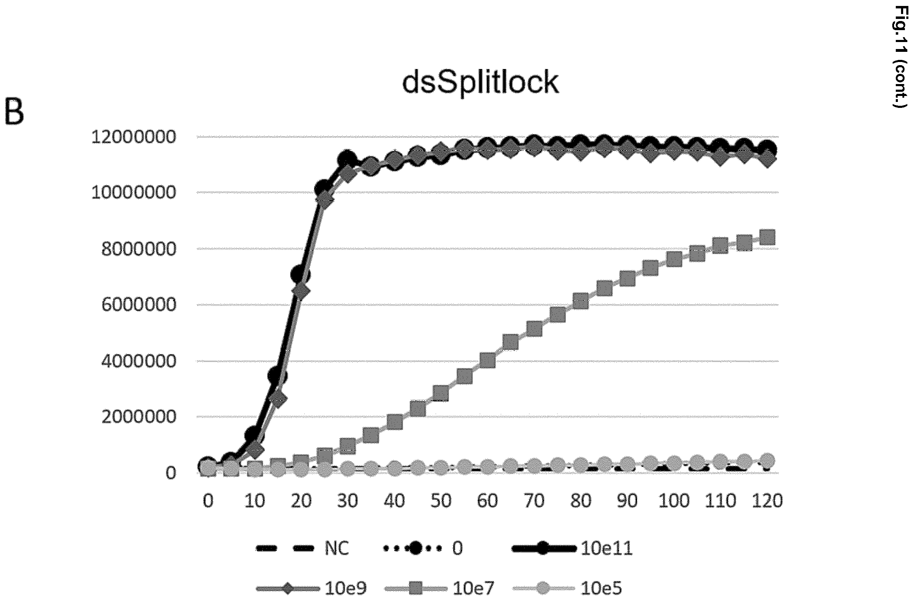

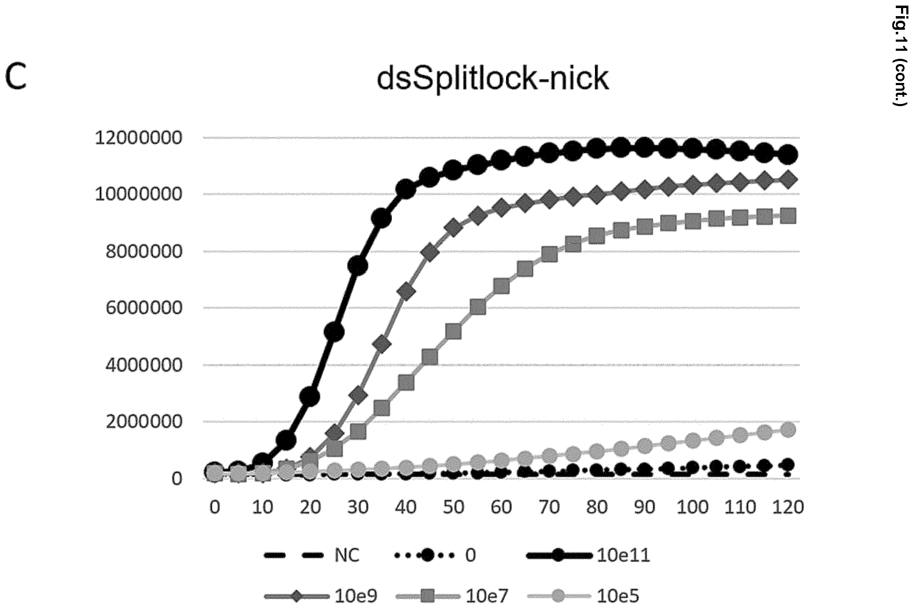

**Fig. 11****: Improvement of HRCA CRISPR-Cas12a: padlock in two arms.** Results of the HRCA reactions with ssDNA **(a)** and dsDNA **(b)** and dsDNA-nick **(c)** split padlock probes as described in Example 11. The indicated amounts of target RNA and a negative control without padlock or target (NC) was tested.

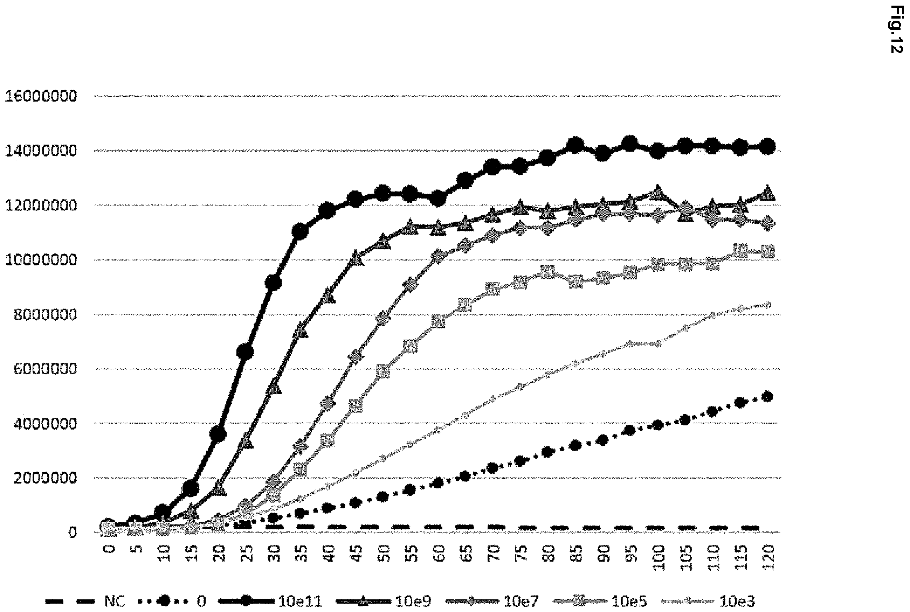

**Fig.12****: Improvement of HRCA CRISPR-Cas12a: combination of FLAP and skin.** The experiment was performed as described in Example 12 and depicted on the left. The results with the indicated amounts of target RNA and a negative control without padlock or target (NC) are shown.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

It is noted that the term "about", as used herein, refers to +/- 30%, preferably +/- 20%, preferably +/- 15%, more preferably +/- 10%, of the indicated referred value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of', though less preferred.

When used herein "consisting of" excludes any element, step, or ingredient not specified in the claim element. When used herein, "consisting essentially of does not exclude materials or steps that do not materially affect the basic and novel characteristics of the claim.

"Percent (%) amino acid sequence identity" with respect to proteins or polypeptides described herein is defined as the percentage of amino acid residues in a candidate sequence that are identical with the amino acid residues in the reference sequence (i.e., the protein or polypeptide from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared.

Preferably, the "percentage of identity" as used herein is decided in the context of a local alignment, i.e., it is based on the alignment of regions of local similarity between nucleobase sequences, contrary to a global alignment, which aims to align two sequences across their entire span. Thus, in the context of the present invention, percentage identity is calculated preferably only based on the local alignment comparison algorithm.

The term "reverse complementary", "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage. Please note that the term "complementary" as used in the present invention also encompasses the term "substantially complementary". A region is "substantially complementary" to a target region when the percentage of complementarity between both regions is of at least 60%, at least 70%, at least 80%, at least 85%, at least 90%, at least 93%, at least 95%, at least 96%, at least 97%, at least 98%, at least 99% or at least 100%. A region is "substantially complementary" to another region if they hybridize under low stringency conditions, preferably medium stringency conditions, most preferably high stringency conditions. Similarly, when the term "non-complementary" is used herein, it should be understood that said term also encompasses the term "substantially non-complementary". A region is "substantially non-complementary" to another region when the complementarity between both regions is less than 40%, less than 30%, less than 20%, preferably less than 10%, most preferably less than 5%, or even most preferably 0% complementarity. A region is "substantially non-complementary" to another region if they do not hybridize under high stringency conditions, preferably medium stringency conditions, most preferably low stringency conditions.

By "functional equivalents" is referred herein to other nucleotide sequences or amino acid sequences that differ in their nucleobase or amino acid sequences, respectively, from that of SEQ ID NO referred to in the specific embodiment, but which perform the same function and provide the same utility or technical effect as the SEQ ID NO: referred to in the specific embodiment.

The term "hybridization" is used to refer to the structure formed by 2 independent strands of nucleic acid (i.e. DNA and/or RNA) that form a double stranded structure via base pairings from one strand to the other. These base pairs are considered to be G-C, A-U/T and G-U. (A - Adenine, C -Cytosine, G- Guanine, U - Uracil, T - thymine). As in the case of the complementarity, the hybridization can be total or partial. In the context of the present invention, each uracil and thymine base can be optionally replaced, respectively, by a thymine or uracil base. In general, whether such hybridization takes place is influenced by, among other things, the length of the polynucleotides and the complementary, the pH, the temperature, the presence of mono- and divalent cations, the proportion of G and C nucleotides in the hybridizing region, the viscosity of the medium, and the presence of denaturants. Such variables influence the time required for hybridization. Thus, the preferred annealing conditions will depend upon the particular application. Such conditions, however, can be routinely determined by the person of ordinary skill in the art without undue experimentation.

"Percentage of sequence identity" for polynucleotides and polypeptides is determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the same nucleobase or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by computerized implementations of known algorithms (BLAST in the resources of the National Center for Biotechnology Information, CLUSTAL in the resources of the European Bioinformatics Institute, GAP, BESTFIT, FASTA, and TFASTA in the Wisconsin Genetics Software Package, Genetics Computer Group (GCG), 575 Science Dr., Madison, Wis.), or by inspection. It should be noted that the "percentage of sequence identity" as used herein is preferably decided in the context of a local alignment, i.e., it is based on the alignment of regions of local similarity between nucleobase sequences, contrary to a global alignment, which aims to align two sequences across their entire span. Thus, in the context of the present invention, percentage identity is calculated preferably only based on the local alignment comparison algorithm.

The term "rolling-circle amplification" or "RCA" refers to an isothermal enzymatic process where a short DNA or RNA primer is amplified to form a long single stranded DNA or RNA using a circular DNA template and phi29 polymerases. For diagnosis purposes, this circular template can be a so-called padlock probe that is circularized in the presence of a target nucleic acid molecule. The RCA product is a concatemer containing tens to hundreds of tandem repeats that are complementary to the padlock probe. In the method of the present invention, it is preferred that the DNA or RNA primer corresponds to the target nucleic acid.

The term "hyperbranched rolling-circle amplification" or "HRCA" refers to an RCA reaction coupled to an MDA reaction.

The term "strand- displacement DNA polymerase" refers to a DNA polymerase that performs a 3' end elongation reaction while melting the corresponding portion of a nucleic acid hybridized to the template DNA strand.

The expression "3' region", as used herein, refers to a region of a nucleotide strand that includes the 3' end of said strand. The term "3' end", as used herein, designates the end of a nucleotide strand that has the hydroxyl group of the third carbon in the sugar-ring of the deoxyribose at its terminus. The expression "5' region", as used herein, refers to a region of a nucleotide strand that includes the 5' end of said strand. The term "5' end", as used herein, designates the end of a nucleotide strand that has the fifth carbon in the sugar-ring of the deoxyribose at its terminus.

As used herein, the term "portion" when referred to a sequence means a nucleotide region or fragment of said sequence. Thus, a "first portion" refers to a first nucleotide region or fragment within a sequence. Similarly, a "second portion" refers to a second nucleotide region or fragment within a sequence. As used herein, "portion" and "region" are considered synonymous.

As used herein, the term "detection" refers to any of a variety of ways of determining the presence and/or quantity and/or identity of a target nucleic acid.

### DESCRIPTION OF THE EMBODIMENTS

In the present invention, an HRCA assay is improved by applying different optimization strategies. The general concept of the optimizations applied herein lies in facilitating the conditions to provide a strong amplification step to ensure that the target nucleic acid is amplified even if present at low concentrations, and then applying stringent conditions in the detection step, so that only the target nucleic acid, and not the background that may have been generated due to the amplification step, is detected. Thus, in a first step, the method applies non-stringent amplification conditions by providing short primers, that have low Tm, and that easily hybridize with the padlock probes. As a result of this amplification step, a huge amount of nucleic acid is exponentially amplified. The amplified nucleic acid may come from target-specific amplifications or from nonspecific (also called background) amplifications, where target-specific specific amplifications are those derived from padlocks that hybridized with the target nucleic acid, and non-specific amplifications derive from the promiscuous and non-specific annealing of the short primers, and/or by padlocks that have circularized in the absence of target nucleic acid.

Next, this bulk amplification is subjected to a strict detection step based on specific nucleotide sequence, so that the huge amount of amplification products generated during the bulk amplification is filtered out and only the nucleic acids resulting from the specific amplifications are detected. With this strategy, the assay of the present invention is capable of detecting low amount of a target nucleic acid or analyte in a sample. Example 6 shows an example of this HRCA method, where short primers are used, but the detection step is performed using CRISPR-Cas12a system, which specifically detects the target sequence present in the region of the S gene of the target SARS-CoV-2 viral genome. As shown in Fig. 6, even though the unspecific amplification provided high yield of amplicons that were undistinguishable in a gel, the detection step allowed for the specific detection of the target nucleic acid, reaching detection of a number of molecules in the order of 10⁵. This demonstrates that the combination of a sequence-specific method with the use of short primers leads to a synergistic effect derived from the strong amplification provided by the short primers and the high specificity of a sequence-based detection method, that allows efficient discrimination of amplicons with the correct sequence.

In view of the above, **a first aspect** of the present invention provides a method for detecting a target nucleic acid in a sample by hyperbranched rolling circle amplification (HRCA), the method comprising the steps of:
a) adding at least one padlock probe to the sample and providing a nucleic acid structure formed by the hybridization of the at least one padlock with the target nucleic acid, wherein in said nucleic acid structure the at least one padlock is in a circularizable form,
b) subjecting the circularizable padlock to a ligation reaction using at least one ligase to provide a padlock comprising sequences A-B-C in a single circular molecule,
c) subjecting the ligated circular padlock of step b) to a rolling circle amplification (RCA) reaction and, optionally, amplifying the RCA product using primers with a maximum of 10 nucleotides in length and,
d) detecting the amplification product from step c), thereby detecting the target nucleic acid, wherein the detection is preferably performed with a sequence-specific detection method.

Each of the steps of the method of the present invention is further explained below:
Step a) adding at least one padlock probe to the sample and providing a nucleic acid structure formed by the hybridization of the at least one padlock with the target nucleic acid, wherein in said nucleic acid structure the at least one padlock is in a circularizable form.

Padlocks are known molecules that play a main role in RCA-based methods. Said molecules are circularizable oligonucleotides, as they comprise, in their 3' and 5' regions, target-complementary regions that are capable of hybridizing to a target DNA or RNA sequence. Upon hybridization to the target nucleic acid, the 3' and 5' ends of the padlock probe become close to each other, and a ligase joins them, thereby providing a circular padlock probe with a region of its sequence hybridized to the target molecule. In the context of the present invention, "padlock" and "padlock probe" are considered synonymous and thus are used interchangeably. The specific structure of the padlock used herein, also called "the padlocks of the present invention" is defined below. Preferably, the padlock of the present invention is a DNA molecule.

In an embodiment, the at least one padlock is a population of padlocks that are identical in sequence, or a population of padlocks that differ in their sequence.

Step a) comprises adding at least one padlock to the sample. If the sample contains the target nucleic acid to be detected, then the at least one padlock and the target nucleic acid are contacted, and a hybridization reaction occurs which leads to the formation of a nucleic acid structure. By "nucleic acid structure" is meant herein the nucleotide configuration formed by hybridization of at least one padlock with the target nucleic acid wherein, due to said hybridization, the 3' and 5' ends of the padlock become close to each other and thus the padlock adopts a circularizable form. By "circularizable form" is meant herein a padlock conformation wherein the padlock is hybridized to the target nucleic acid and comprises ligatable 5' and 3' ends that are capable of being ligated and closed into a circle. A padlock in a circularizable form, also called herein "circularizable padlock", is thus a target-specific, ligation-dependent circularizable probe. Thus, the nucleic acid structure of step a) is a structure formed by a padlock in a circularizable conformation hybridized to a target nucleic acid.

In an embodiment, the padlock is added in the form of a single oligonucleotide molecule that becomes a circularizable probe when it hybridizes with the target nucleic acid, thereby providing the nucleic acid structure of step a). In this case, the padlock in a circularizable form is a single, target-specific, ligation dependent circularizable probe.

In another embodiment, the padlock is added as a system of two or more different oligonucleotide molecules that arrange in a circular shape when hybridized to the target nucleic acid, thereby providing the nucleic acid structure of step a). By "system of two or more different oligonucleotide molecules" is referred herein as independent oligonucleotide molecules that comprise different sequences and thus represent different parts or fragments of a padlock. In this case, the padlock in a circularizable form is a cluster of several molecules that form a target-specific, ligation dependent circularizable probe.

The nucleotide sequence of the system of two or more different oligonucleotide molecules is designed in a way that they are capable of assembling to each other and to become a circularizable padlock when one or more of them hybridize to the target nucleic acid, thereby providing the nucleic acid structure of step a). This assembly may be achieved by including complementary sequences in each of said oligonucleotide sequences, so that they arrange in a specific order by hybridization. The assembly may also be achieved by including other splint molecules that hybridize with two of said molecules, bringing them together. Thus, a "splint nucleic acid molecule" is a nucleotide sequence that serves as a bridge between two ends of the padlock molecule or system of molecules, facilitating the ligation of the ends of said molecules in step b).

The circularizable padlock, when added as a single oligonucleotide, will have one nick, located in the region where it hybridizes to the target nucleic acid, i.e., between its 5' and 3' ends. The circularizable padlock, when added as a system of two or more different oligonucleotide molecules, will have one nick in its sequence for every junction between two molecules, and one nick where it hybridizes to the target nucleic acid. Thus, the circularizable padlock added as a system of two or more molecules will present two or more nicks in its sequence. Hence, it is understood that the circularizable padlock or the padlock in a circularizable conformation provided in the nucleic acid structure of step a) is not to be interpreted only as to a continuous single molecule, but rather as one or more molecules arranged in a circular shape, presenting one or more nicks in its sequence that, upon addition of at least one ligase, will be ligated to each other in step b) to become a covalently-closed single-stranded nucleic acid circular probe. Preferably, said covalently-closed single-stranded nucleic acid circular probe is a DNA covalently-closed single-stranded nucleic acid circular probe. Hence, the padlock in a circularizable conformation of the nucleic acid structure of step a) does not need to be a single molecule, but it may be several molecules arranged in a specific order providing a circularizable structure. Figure 1 depicts preferred nucleic acid structures of the present invention, which will be further explained below.

Each of the different molecules that form the padlock system may be added to the sample simultaneously or subsequently. They may also be added pre-hybridized to each other, such as the case of the Skinlocks or dsSplitlocks that are detailed below. Suitable hybridizing conditions are known in the art. Thus, step a) of the method of the present invention comprises contacting a sample comprising a target nucleic acid with at least one padlock and providing a nucleic acid structure formed by the hybridization of at least one padlock with a target nucleic acid, wherein the padlock adopts a circularizable form.

In an embodiment, the at least one circularizable padlock of the present invention is characterized by comprising, in the 5' to 3' direction, sequences A-B-C, as represented in Fig 1A. By "sequence A" is referred herein to a nucleotide sequence located at the 5' region, preferably at the 5' end, of the circularizable padlock that is complementary (including substantially complementary, see definitions above) to a first portion of the target nucleic acid. By "sequence C" is referred herein to a nucleotide sequence located at the 3' region, preferably at the 3' end, of the circularizable padlock that is complementary (including substantially complementary, see definitions above) to a second portion of the target nucleic acid. By sequence "B" is referred herein to a nucleotide sequence located between sequences A and C and that is not complementary (including not substantially complementary, see definitions above) to the target nucleic acid. Preferably, the different sequences of the padlock are defined herein in the context of a nucleic acid structure of step a) as represented in Fig. 1, i.e., when the target nucleic acid is depicted in a 5' to 3' direction.

By "at the 5' region of the padlock" and "at the 3' region of the padlock" is referred herein that the sequences A and C, respectively, are located towards or in the vicinity of the 5' or 3' end, respectively, of the padlock (or of one of the molecules that constitute the system of two or more different oligonucleotide molecules), when arranged in a circularizable conformation. Although it is preferred that sequences A and/or C are located at the 5' and 3' ends of the padlock, respectively, in some other embodiments, they may be located close or adjacent to the 5' and 3' ends of said padlock, so that there is a further nucleotide sequence, not complementary to the target nucleic acid, that is at the 5' or 3' end. This is possible as long as the sequences A and C of the padlock are capable of hybridizing, under suitable hybridizing conditions, to the first and second portions, respectively, of the target nucleic acid, resulting in a padlock with circularizable conformation. Suitable hybridizing conditions are known in the art.

In an embodiment, sequences A and/or C of the circularizable padlock are comprised in the 30, 25, 20, preferably 15, nucleotides located at the 5' and/or 3' ends of the circularizable padlock. In an embodiment, sequence A of the circularizable padlock is located less than 50, 40, 30, 25, 20, 15, 10, 9, 8, 7, 6, 5, 4, 3, 2, or 1 nucleotides from the 5' end of said padlock. More preferably, sequence A is located exactly at the 5' end of the padlock in a circularizable conformation. In an embodiment, sequence C of the circularizable padlock is located less than 50, 40, 30, 25, 20, 15, 10, 9, 8, 7, 6, 5, 4, 3, 2, or 1 nucleotides from the 3' end of the padlock probe. More preferably, sequence C of the padlock probe is located exactly at the 3' end of the padlock in a circularizable conformation.

In an embodiment, sequences A and/or C of the circularizable padlock comprise a nucleotide sequence that is at least 70%, 75%, 80%, 85%, preferably 90%, 92%, 94%, 96%, 98% or 100% complementary to a first and a second portions, respectively, of the target nucleic acid.

In a preferred embodiment, the 5' region, preferably 5' end, of the circularizable padlock of the present invention is thus characterized by comprising sequence A. In a preferred embodiment, the 3' region, preferably 3' end, of the circularizable padlock of the present invention is thus characterized by comprising sequence C. Sequence A and/or C of the padlock may be of any suitable length as long as they allow the padlock to stably hybridise to the target nucleic acid sequence resulting in a circularization of the probe, as will be explained in detail below. For example, sequence A and/or C may be 6-20 nucleotides in length. A minimum size of 6 nucleotides is selected to ensure specificity of binding. Thus, preferably the size range of each of sequences A and/or C may be 6-20, 6-19, 6-18, 6-17, 6-16, 6-15, 6-14, 6-13, 6-12, 6-11, 10-25, 10-20 or 6-10 nucleotides in length. Preferably, sequence A and/or C are 15 nucleotides in length. The length sequences A and C may be the same or different, and thus for example a probe may have target-complementary regions of 6+6, 6+7, 7+7, 6+8, 7+8, 8+8, 8+9, 7+9, 9+9, 10+9, 10+10, 10+11, 11+11, 11+12, 12+12 etc., i.e., any combination of any integers within the above-noted ranges. Sequences A and/or C may be comprised in different molecules when the padlock is added in the form of two or more different molecules.

The total length of the hybridized region (i.e., the sum of the nucleotides forming sequences A and C) may be 12-50, more particularly 12-40 nucleotides, more particularly 12-36, 12-32 or 12-30, 12-28, 12-26, 12-24, 12-23, 12-22, 12-21 , 12-20, 12-19, 12-18, 12-17, 12-16, 10 13-30, 13-28, 13-26, 13- 24, 13-23, 13-22, 13-21 , 13-20, 13-19, 13-18, 13-17, 13-16, 14-30, 14-28, 14-26, 14-24, 12-23, 14-22, 14-21 , 14-20, 12-19, 14-18, 14-17, 14-16, 15-30, 15-28, 15-26, 15-24, 15-23, 15-22, 15-21 , 15-20, 15-19, 15-18, 15-17, or 15-16 nucleotides. Preferably, the total length of the hybridized region is between 20-40 nucleotides, preferably 30 nucleotides.

Sequence A is complementary and capable of hybridizing to a first portion of the target nucleotide. Sequence C is complementary and capable of hybridizing to a second portion of the target nucleotide. Thus, the first and second portions of the target nucleotide are complementary to sequences A and C of the padlock, respectively. This is essential because, as explained above, the padlock will adopt a circularizable form upon hybridization to the target, which will bring together its 5' and 3' ends providing the nucleic acid structure of step a). Hence, sequences A and C located at the 5' and 3' regions, preferably at the 5' and 3' ends, of the circularizable padlock are complementary to and capable of hybridising to adjacent or substantially adjacent sequences in a target nucleic acid, such that the hybridisation of the padlock (either as a single oligonucleotide molecule or as a system of two or more different oligonucleotide molecules) to the target nucleic acid in step a) results in the 5' and 3' ends of the padlock being brought into juxtaposition, forming a nucleic acid structure in which the padlock acquires a circularizable conformation, but contains at least one nick between the A and C sequences. Use of the term "adjacent" is herein intended to mean that there are no nucleotides (i.e., there are 0 nucleotides) of the target sequence left without base-pairing between the first and second portions of the target nucleic acid sequence which are base paired to sequences A and C, respectively, of the padlock. This proximity between the first and second portions of the target nucleotide enables the 5' and 3' ends of the padlocks to be close to each other. The first and second portions of the target nucleic acid are thus close to each other, but they are not overlapping. Preferably, the first and second portions are contiguous sequences, i.e., they are located directly next to each other, wherein the first portion of the target nucleic acid is located at the 5' of the second portion of the target nucleic acid or, in other words, the second portion of the target nucleic acid is located at the 3' of the first portion of the target nucleic acid. Consequently, the first portion of the target nucleic acid is located closer to the 5' end of the target nucleic acid than the second portion, and the second portion of the target nucleic acid is located closer to the 3' end of the target nucleic acid that the first portion.

As will readily be apparent to those skilled in the art, when selecting the first and second portions of the target nucleotide and designing the padlock sequences A and/or C, gaps between the first and second portions of the target nucleic acid may be introduced between nucleotides to which sequences A and/or C of the probes hybridize. In this situation the first and second portions of the target nucleic acid are said to be "substantially adjacent", because there may be some nucleotides left without base-pairing between sequences A and C of the padlock. Preferably, the term "substantially adjacent" is herein intended to mean that there is a maximum of 4 nucleotides, preferably a maximum of 3, 2 or 1 nucleotides of the target nucleic between the first and second portions left without base-pairing with the padlock. Thus, whilst it is preferred that the sequences A and C of the padlock hybridize in the target nucleic acid so as to be adjacent, that is, as to leave 0 nucleotides in between the first and second portions of the target nucleic acid, the sequences A and C of the padlocks may be separated by a maximum 4, 3, 2 or 1 nucleotides of target nucleic acid, and the term "substantially adjacent" is intended in the present invention to refer to such latter situation. In an embodiment, the first portion of the target nucleotide is located towards the 5' end of the target nucleic acid and the second portion of the target nucleic acid is located towards the 3' end of the target nucleic acid, see Fig. 1.

The padlock of the present invention further comprises a backbone sequence, not complementary or not substantially complementary to the target nucleic acid, called herein sequence B. The length of this sequence is not particularly limiting, with the only proviso that it is long enough as to allow sequences A and C hybridize to the target nucleic acid and adopt a ligatable circular configuration. Sequence B may thus be preferably longer that the sum of the lengths of sequences A and C. This allows the padlock to be flexible and hybridize to the target nucleotide in a circularizable conformation more easily. Preferably, sequence B is at least 30 nucleotides in length, preferably at least 40, 50, 60, 70, or 80, nucleotides in length, preferably between 30-50, 30-40, 30-50, 30-60, 30-70, 30-80, 30-90, or 30-100 nucleotides in length. In an embodiment, sequence B is characterized by not being complementary to the target nucleic acid and by comprising complementary sequences to the sequences of the one or more primers used in the method of the present invention. In other words, it is preferred that the one or more primers used in the present invention, which will be further defined below, hybridize or anneal in the B sequence and/or in its reverse complementary thereof. Similar as for sequences A and/or C, sequence B may be comprised in different molecules when the padlock is added in the form of two or more different molecules.

Further, as explained above, the padlock may be added together with other molecules called herein "splint nucleic acid molecule". In an embodiment, the splint nucleic acid molecule comprises a first domain that hybridizes to a region of sequences A, B, or C, and a second domain that hybridizes with another region of sequences A, B or C, thereby bringing said regions together to facilitate the formation of the circularizable arrangement of a padlock.

In order to further improve the limit of detection of the HRCA method, the authors of the present invention focused on implementing different improvements in the padlocks that could, on their own or combined, reduce background and/or increase target-specific amplification. Said improvements are different ways of "adding at least one padlock" and different ways of "providing the nucleic acid structure" in step a), as it will be further explained below.

As explained above, all circularizable padlocks comprise, in the 5' to 3' direction, sequences A-B-C. However, one improvement is to add at least a padlock comprising, either as a single or as a system of two or more different molecules, additional sequences other than A-B-C. Said additional sequences may be sequence E (skin) and/or sequence D (flap). The following embodiments define said improved padlocks:

### Skinlock

As explained above, the at least one padlock may be added in step a) as a system of two or more different oligonucleotide molecules that are designed to assemble to each other and hybridize by means of sequences A and C to the target nucleic acid. In the case of the padlock with a skin or Skinlock, the padlock is added in the form of a system of at least two different oligonucleotide molecules, wherein one of the molecules is a skin sequence, also called herein sequence E. Sequence E is complementary to sequence B or to a region of sequence B. Skinlocks are used in Example 7 and are represented in Fig. 1B. The advantage of using a padlock with sequence E in the HRCA method of the present invention can be summarised as follows:
i) since it covers, totally or partially, sequence B of the padlock, it avoids premature primer annealing that may triggers unspecific RCA or MDA amplification before the target-dependent RCA reaction is triggered. This reduces non-specific amplifications,
ii) further, the skin also limits the hybridization of remaining padlocks with the initial RCA products, which would outcompete with MDA primers, limiting the HCRA reaction. This improves the efficiency of the amplification step,
iii) lastly, if a 3' OH is added to the skin or sequence E, it may be used as a primer to start RCA, thereby increasing the efficiency of the method, without prejudice of its specificity. This also improves the efficiency of the amplification step.

Thus, the presence of a skin hybridized to the padlock results in a reduction in the background signal of the amplification step, as shown in Example 7 and figure 7.

In an embodiment, sequence E is complementary to the entire length of sequence B. In another preferred embodiment, sequence E is not complementary to the entire length of sequence B, but is complementary to a region comprised in sequence B, so that not all nucleotides of sequence B are hybridized to sequence E. Preferably, sequence E is complementary to a region of sequence B, wherein said region is the primer hybridization region of the primers used in the MDA reaction of step c), so that the primers cannot hybridize in sequence B if sequence E is hybridized to sequence B. Preferably, sequence E is complementary to a central region of sequence B, but not complementary to the 3' and 5' regions of sequence B. The fact that there are nucleotides in the 3' and 5' regions, preferably 5' and 3' ends, of sequence B that are not complementary to sequence E provides the padlock with more flexibility at the time of circularizing and hybridizing to the target nucleic acid. Thus, in an embodiment, sequence E is shorter that sequence B. In an embodiment, sequence E is complementary to a region of sequence B, wherein said region of sequence B is not the 3' nor 5' ends of sequence B. Preferably, sequence E is complementary to a region of sequence B, wherein said region of sequence B is at least 1, 2, 3, 4, 5, 6, 7, 8, 9, 10 nucleotides of distance from the 3' and 5' end of sequence B. In an embodiment, sequence E is complementary to a region of sequence B, wherein said region represents at least 50%, 60%, 70%, 80% or 90% of the length of sequence B. In an embodiment, sequence D is DNA or RNA.

In an embodiment, sequence E comprises a modified nucleotide in its 3' end, so that sequence E is not capable to prime the RCA reaction. In an embodiment, the modified nucleotide comprises a molecule that avoids the use of said nucleotide as a starting point of a polymerization, i.e., as a primer. In an embodiment, the nucleotide at the 3' end of the E sequence is bound to a molecule that prevents the use of 3' end as a primer by the polymerase of the reaction. Preferably, the 3' end of sequence E is covalently bound to biotin. In an alternative embodiment, sequence E is used as a primer in the RCA reaction of step c). In order to facilitate the priming using the 3' end of sequence E, the nucleotide located at the 3' end of sequence E comprises a 3'OH.

In a most preferred embodiment, the padlock of the present invention is a Skinlock and is added in step a) in the form of a system of two different oligonucleotide molecules, wherein the first molecule comprises, in the 5' to 3' direction, nucleotide sequences A-B-C, wherein:
- A is a nucleotide sequence located in the 5' region of the padlock that is complementary to a first portion of the target nucleic acid and is capable of hybridizing thereto,
- B is a nucleotide sequence that is not complementary to the target nucleic acid,
- C is a nucleotide sequence that is located in the 3' region of the padlock and that is complementary to a second portion of the target nucleic acid and is capable of hybridizing thereto,
and wherein the second oligonucleotide molecule comprises a nucleotide sequence E that is complementary to nucleotide sequence B or a portion thereof, and wherein the nucleic acid structure of step a) with the padlock in a circularizable form is provided by hybridizing the first molecule to the target nucleic acid and the second molecule to the first molecule. Said circularizable padlock will present at least nick in its sequence, which is located in the junction region of sequences A and C. The first and second molecules can be added simultaneously or subsequently to the sample. When the padlock comprises sequence E, it is preferrable that the padlock is added to the sample with sequence E already hybridized to sequence B, i.e., the padlock is preferably added in a pre-hybridized form.

Sequence E is compatible with and can be added to any of the padlocks defined in the present invention.

### Flaplocks

These padlocks are tested in Example 9 and represented in Fig. 1 C and D. They are added in step a) comprising an additional sequence, named herein sequence D or flap, that is placed at the 3' and/or 5' end of the padlock, and that is not complementary to the target nucleic acid. Sequence D thus overhangs from one of the padlock's ends after the padlock has hybridised to the target and will impede ligation of the 3' end of sequence C with the 5' end of sequence A until sequence D is removed by nucleolytic cleavage. Consequently, if sequence D is placed at the 3' end of sequence C and if the padlock has hybridized with the target nucleic acid, the 3' end of the padlock is not ligatable with the 5' end until sequence D is cleaved. Similarly, if sequence D is placed at the 5' end of sequence A and if the padlock has hybridized with the target nucleic acid, then the 5' end of the padlock is not ligatable with the 3' end until sequence D is cleaved. It is noted however that the presence of sequence D, either at 3' or 5' ends of the padlock, does not impede the hybridization of sequences A and C to the target nucleic acid. Sequence D impedes the formation of the nucleic acid structure of step a) because it impedes the formation of the padlock in a circularizable form. Hence, a padlock with sequence D cannot proceed with the ligation of the 5' end of sequence A with the 3' end of sequence C in step b) once the padlock has hybridized with the target nucleic acid.

In this case, the junction between the 5' end of A and 3' end of C is "created" by nucleolytic cleavage of the 3' or 5' end of sequence D. The padlock may, therefore, comprise an additional sequence D at the 5' or 3' end of the padlock that provides, once the padlock has hybridized to the target nucleic acid, a 5' flap that results in non-ligatable 5'-3' ends. Said end with said additional sequence D or flap will become ligatable once said sequence D or flap has been removed by cleavage. If sequence D is placed in the 5' end of the padlock, said padlock comprises a sequence A situated internally of the 5' end of the padlock and, once the padlock has hybridized to the target nucleic acid, the 5' end of the padlock is not ligatable to its 3' end due to the presence of sequence D. Once the internal sequence A has hybridized to the target nucleic acid, sequence D will be removed by nucleolytic cleavage, thereby creating the 5' ligatable end of sequence A of the padlock.

If sequence D is placed in the 3' end of the padlock, said padlock comprises a sequence C situated internally of the 3' end of the padlock and, once the padlock has hybridized to the target nucleic acid, the 3' end of said padlock forms a 3' flap that is not ligatable to its 5' end due to the presence of sequence D, which cannot hybridize to the target nucleic acid. Once the internal sequence C has hybridized to the target nucleic acid, sequence D will be removed by nucleolytic cleavage, thereby creating the 3' ligatable end of sequence C of the padlock.

Padlocks having sequence D (i.e. padlocks in which sequence A or C is situated internally of the 5' or 3' end, respectively, of the padlock) and hybridized to the target nucleotide are regarded as being non circularizable padlocks as they need to be activated (i.e. by nucleolytic cleavage) prior to forming the nucleic acid structure of step a). In this way, only when sequence D is cleaved after padlock hybridization with the target nucleic acid, the padlock adopts a circularizable form, the structure of step a) is provided, and step b) of the method may proceed. By "situated internally" is referred herein that sequence A or C are located not in the 5' or 3' end, respectively, of the padlock, but in a region next to sequence D.

The use of the flap sequence or sequence D in the padlock is advantageous when using a sequence-based detection method, as it allows for discriminating target-specific amplicons from unspecific amplicons: if the target nucleic acid is present in the sample and the padlock is hybridised to it, sequence D is removed by nucleolytic cleavage and the 5' end of sequence A and 3' end of sequence C of the padlock are ligated. The padlock is then amplified in step c), without comprising sequence D. Thus, target-specific amplicons will lack sequence D. However, if the padlock sequence does not hybridise with the target nucleic acid (maybe because it is not present or because there is an excess of padlocks in comparison with target nucleic acid), the ligase will join the end of sequence D with the end of sequence A or C, thereby circularizing the padlock with sequence D. This padlock including sequence D will then be amplified, resulting in nonspecific amplicons comprising sequence D. As a result, two amplification products that differ in their nucleotide sequence are obtained: one from the padlocks that hybridised to the target nucleic acid that lack sequence D, and another from nonspecific padlocks that did not hybridise to the target nucleic acid and thus comprise sequence D. The sequence-specific detection method will allow the efficient identification of those amplicons that hybridised to the target nucleic acid, disregarding the unspecific ones that differ in sequence.

The cleavage of sequence D after the padlock has hybridized with the target nucleic acid may be performed by action of a single-stranded DNA specific exonuclease that degrades sequence D until reaching the hybridized A or C sequences, respectively. If sequence D is placed in the 5' end of sequence A, an enzyme having 5' exonuclease activity may be used to cleave the 5' sequence D. If sequence D is placed in the 3' end of sequence C, an enzyme having 3' exonuclease activity may be used to cleave the 3' sequence D. These 5' or 3' exonucleases degrade a single-stranded oligonucleotide having a free 5' or 3' end by degrading the oligonucleotide into constituent mononucleotides from its 5' or 3' end. Alternatively, structure specific nucleases such as Flap endonucleases (FENS), which are a class of enzymes having endonucleolytic activity and being capable of catalysing the hydrolytic cleavage of the phosphodiester bond at the junction of single- and double-stranded DNA, may be used. As with the exonucleases, the FEN used will need to have 3' or 5' polarity depending on the location of D. It is preferred to use an exonuclease, as it will not only remove sequence D of hybridised padlocks, but degrade the excess of non-hybridized padlocks.

The length of sequence D is not limiting, as long as it allows the padlock to hybridize via its sequences A and C to the target nucleic acid, and as long as it prevents the ligation of the 5' and 3' ends of the padlock when it is hybridized to the target nucleic acid if sequence D is present. In an embodiment, sequence D is between 5-50, 5-40, 5-30, 10-40, 10-30, 5-10, 5-15, 10-15, 10-20, nucleotides in length. Further, the specific nucleotide sequence of sequence D is also not limited, as it can be any sequence but with the proviso that it does not hybridize with the target nucleic acid and preferably that it does not hybridize, or present complementarity, with any molecule present in the reaction.

In a most preferred embodiment, the padlock of the present invention added in step a) is a Flaplock that comprises, in the 5' to 3' direction, nucleotide sequences A-B-C, wherein:
- A is a nucleotide sequence located in the 5' region of the padlock that is complementary to a first portion of the target nucleic acid and is capable of hybridizing thereto,
- B is a nucleotide sequence that is not complementary to the target nucleic acid,
- C is a nucleotide sequence that is located in the 3' region of the padlock and that is complementary to a second portion of the target nucleic acid and is capable of hybridizing thereto,
wherein the Flaplock further comprises a nucleotide sequence D, located at the 3' end of sequence C and/or at the 5' end of sequence A, wherein sequence D is characterized in that it is not complementary to the target nucleic acid, and wherein the nucleic acid structure of step a) with the padlock in a circularizable form is provided by hybridizing the Flaplock, preferably in the form of a single oligonucleotide molecule, with the target nucleic acid and subsequently nucleolytically cleaving sequence D, preferably by action of a ssDNA-specific exonuclease or a FLAP endonuclease. Said circularizable padlock will present at least one nick in its sequence, which will be located in the junction region of sequences A and C.

In an embodiment, the Flaplock is added to the reaction as a single oligonucleotide molecule comprising, in the 5' to 3' direction, sequences A-B-C, wherein the padlock further comprises sequence D, and wherein sequence D is placed at the 5' end of sequence A or at the 3' end of sequence C, wherein sequence D is characterized in that it is not complementary to the target nucleic acid and wherein the nucleic acid structure of step a) with the padlock in a circularizable form is provided by hybridizing the first molecule with the target nucleic acid and subsequently nucleolytic cleaving sequence D by action of a ssDNA-specific exonuclease or a FLAP endonuclease. Said circularizable padlock will present at least one nick in its sequence, which will be located in the junction region of sequences A and C.

In another embodiment, the Flaplock is added to the reaction in the form of a system of at least two different oligonucleotide molecules, wherein a first molecule comprises, in the 5' to 3' direction, sequences A-B-C-D as represented in Fig. 1D or D-A-B-C as represented in Fig 1C, and wherein a second oligonucleotide molecule comprises sequence E, and wherein:
- the first and second molecules are added separately to the sample (in any order), and the nucleic acid structure of step a) is formed by hybridizing the first molecule with the target nucleic acid and the second molecule to the first molecule, and subsequently exonucleolytic cleaving sequence D by action of a ssDNA-specific exonuclease or a FLAP endonuclease, or
- the first and second molecules are added to the sample being hybridized to each other, and the nucleic acid structure of step a) is formed by hybridizing the first molecule with the target nucleic acid and subsequently exonucleolytic cleaving sequence D by action of a ssDNA-specific exonuclease or a FLAP endonuclease.

Said circularizable padlock will present at least one nick in its sequence, which will be located in the junction region of sequences A and C. The first and second molecules can be added prehybridized (i.e., hybridized to each other), simultaneously or subsequently to the sample. It is noted that sequence E has the features as defined in the case of Skinlock, and thus the above embodiments algo apply herein and throughout the entire document where sequence E is mentioned.

Preferably, the exonuclease is a 5' exonuclease if sequence D is located at the 5' end of sequence A. Preferably, the exonuclease is a 3' exonuclease if sequence D is located at the 3' end of sequence C. Preferably, the 3' exonuclease is selected from Exo I or the 3' exonuclease activity of phi29 polymerase.

Preferably, the at least one padlock is at least one Flaplock, and the method comprises a step a) comprising a step a.1) of adding the Flaplock to the sample and hybridizing at least one padlock with a target nucleic acid, and step a.2) comprising the 5' or 3' nucleolytic cleavage of sequence D to provide the nucleic acid structure of step a). Sequence D is compatible and can be added to any of the padlocks defined in the present invention.

Padlocks added in the form of a system of two or more different molecule, wherein sequences A-B-C are divided into two or more oligonucleotide molecules.

In an embodiment, sequences A-B-C of the at least one padlock of the present invention are added in step a) divided or comprised in different oligonucleotides molecules. If sequences A-B-C are added in one or more parts as a system of different oligonucleotide molecules, said different oligonucleotides molecules comprising sequences A and/or B and/or C should each comprise, or generate (i.e. by cleavage or extension), ligatable 5' and 3' ends, so that upon, addition of a ligase in step b), sequences A-B-C can be ligated and joined in this order (from 5' to 3') to provide a circular padlock in step b) that will be amplified in step c). Providing the padlock sequences A-B-C in two or more different molecules presents the general advantage of limiting self-circularization due to the reduced size of the molecules. Most importantly, splitting the sequences required for the different steps of the reaction (primer binding sites, complementary target sequence, etc) in different molecules, favours the amplification and detection of only the molecules that were assembled correctly, as opposed to random ligation and spurious priming events.

The padlock system comprising sequences A-B-C in the form of two or more different molecules preferably comprises at least two molecules that each comprise sequences A and/or C, so that they can hybridize to the target nucleic acid. Thus, although not a requirement, it is preferred that each of the different molecules have at least one target-specific binding site, sequence A and/or C, or a portion of sequence A and/or C. In these systems, comprising sequences A-B-C in the form of two or more different molecules, a splint nucleic acid molecule may be present to bridge between said different molecules, acting as a bridge and facilitating the circularizable conformation of the padlock. Said splint nucleic acid molecule can be the target nucleic acid, the E sequence described above, and/or other molecules. The splint molecules will guide the assembly of the A, B and C sequences of the padlock in the correct orientation, which is, in the 5' to 3' direction, sequences A-B-C and, as a result, the circularizable padlock of the nucleic acid structure of step a) will comprise two or more nick between sequences A-B-C. However, the molecules comprising sequences A-B-C and forming said circularizable padlock will comprise ligatable ends and thus will be able to be ligated in step b), forming a single circularized molecule.

In an embodiment, sequences A, B and C of the padlock are comprised in two or more different molecules, where all molecules are contacted with the sample simultaneously. In an embodiment, sequences A, B and C of the padlock are comprised in two or more different molecules, where at least two of the different molecules are contacted with the sample subsequently. In a preferred embodiment, the A- and C-containing molecules are first contacted to the sample, allowing their hybridization to the target nucleic acid (if present). Then, an additional splint nucleic acid molecule or molecules, such as sequence E, is added to the sample to further bridge sequences A and C and to provide the nucleic acid structure of step a). In another preferred embodiment, the A- and C-containing molecules are prehybridized to sequence E before adding them to the sample, so that the system of two molecules is added to the sample in a pre-hybridized form.

The following padlocks are preferred embodiments of a padlock of the present invention where sequences A-B-C are comprised in at least two, preferably two, different molecules:

### • Gaplocks:

These padlocks are described in Example 10 and represented in Fig. 1E and 1F. They are added to the reaction as a system of two or more different molecules, wherein a first molecule of the Gaplock system comprises sequence B and a portion of sequences A and C, and the second or more molecules comprise the remaining sequence of A and/or C. The hybridization of the portion of sequences A and C of the first molecule leave a non-hybridized gap in the target nucleic acid that is filled by the hybridization of the second or further molecules. Once the first and second or more molecules have hybridized to the target nucleic acid, the Gaplock probe then adopts the circularizable form to provide the nucleic acid structure of step a). In these Gaplocks, the target nucleic acid acts as a splint nucleic acid molecule for the ligations required to join the first and second or more molecules, to provide sequences A-B-C in a single molecule in step b), so additional molecules besides those comprising A, B and C may not be required in this system of two or more molecules.

The effect of these padlocks in combination with the sequence-specific detection method relies in the fact that only the Gaplocks that have hybridized will comprise the sequence provided in the second or further molecules, so that they are easily identified by the sequence-specific method of step d). Gaplock probes that do not hybridize to the target nucleic acid or that are ligated without the target nucleic acid will not present the sequence provided in the different second or further molecules. Thus, while the target-specific amplicons derived from Flaplocks are detected by the absence of the sequence D or flap, the target-specific amplicons derived from Gaplocks are detected by the presence of the sequence provided in the second or further molecules that filled the gap between 3' and 5' ends of the first molecule of the padlock system.

In an example of a Gaplock, represented in Fig. 1E, a division is performed in sequence C: In this preferred embodiment, sequences A-B-C of the Gaplock are added in the form of a system of at least two different oligonucleotide molecules, wherein a first molecule comprises, in the in the 5' to 3' direction, sequences A, B, and a first portion of sequence C (C1) and a second molecule comprises a second portion of C (C2). Consequently, in this preferred embodiment of a Gaplock, the first molecule comprises, in the 5' to 3' direction, sequences A-B-C1, and the second molecule comprises sequences C2. A third molecule comprising sequence E may be added, thereby becoming a system of three or more different oligonucleotide molecules, as represented in Fig. 1G. In the embodiment of a Gaplock having a division in sequence C, it is preferred that the first and second different molecules, comprising sequences 5'- A-B-C1-3' and 5'-C2-3', respectively, are added to the sample in step a) simultaneously. The length of C1 and C2 is not limiting as long as it is long enough to hybridize to the target nucleotide. Preferably C1 and/or C2 is at least 6, 8, 10, 12, 15 nucleotides in length. The length of sequence B and E, if present, is as defined in embodiments above. In the embodiment of a Gaplock having a division in sequence C, the circularizable padlock in step a) is formed by hybridizing the first and second molecule to the target nucleic acid. Said circularizable padlock will present at least two nicks in its sequence, one placed at the junction region of sequences C1 and C2, and the other placed between sequences A and C2. The first and second molecules can be added simultaneously or subsequently to the sample.

In another example of a Gaplock, represented in Fig. 1E, a division is performed in sequence A: In this preferred embodiment, the sequences A-B-C of the Gaplock are added in the form of a system of at least two different oligonucleotide molecules, wherein a first molecule comprises, in the 5' to 3' direction, a first portion of sequence A (A1), followed by sequences B and C, and wherein a second molecule comprises a second portion of sequence A (A2). Consequently, in this preferred embodiment of a Gaplock, the second molecule comprises sequence A2, and the first molecule comprises, in the 5' to 3' direction, sequences A1-B-C. A third molecule comprising sequence E may be added, thereby becoming a system of three or more different oligonucleotide molecules, as represented in Fig. 1G. In the embodiment of a Gaplock having a division in sequence A, it is preferred that the first and second different molecules, comprising sequences and 5'-A1-B-C-3' and 5'-A2-3', respectively, are added to the sample in step a) simultaneously. The length of A1 and A2 is not limiting as long as it is long enough to hybridize to the target nucleotide. Preferably A1 and/or A2 is at least 6, 8, 10, 12, 15 nucleotides in length. The length of sequence B and E, if present, is as defined in embodiments above. In the embodiment of a Gaplock having a division in sequence A, the circularizable padlock in step a) is formed by hybridizing the first and second molecule to the target nucleic acid. Said circularizable padlock will present at least two nicks in its sequence, one placed at the junction region of sequences A1 and A2, and the other placed between sequences A2 and C. The first and second molecules can be added simultaneously or subsequently to the sample.

In another example of a Gaplock, a division is performed in each of sequences A and C: In this preferred embodiment, sequences A-B-C of the Gaplock are added in the form of a system of at least two different oligonucleotide molecules, wherein a first molecule comprises, in the 5' to 3' direction, a first portion of sequence A (A1), followed by sequences B and a first portion of C (C1), and a second molecules comprises, in the 5' to 3' direction, a second portion of sequence C (C2) and a second portion of sequence A (A2). Consequently, in this preferred embodiment of a Gaplock, the first molecule comprises, in the 5' to 3' direction, sequence A1-B-C1, and the second molecule comprises, in the 5' to 3' direction, sequences C2-A2. A third molecule comprising sequence E may be added, thereby becoming a system of three or more different oligonucleotide molecules. In the embodiment of a Gaplock having a division in sequences A and C, it is preferred that the first and second different molecules, comprising sequences 5'-A1-B-C1-3' and 5'-C2-A2-3', respectively, are added to the sample in step a) simultaneously. The length of A1, A2, C1 and C2 is not limiting as long as it is long enough to hybridize to the target nucleotide. Preferably A1, A2, C1 and/or C2 is at least 6, 8, 10, 12, 15 nucleotides in length. The length of sequence B, and sequence E, if present, is as defined in embodiments above. In the embodiment of a Gaplock having a division in sequences A and C, the circularizable padlock in step a) is formed by hybridizing the first and second molecule to the target nucleic acid. Said circularizable padlock will present at least two nicks in its sequence, one placed at the junction region sequences of A1 and A2, and the other placed between sequences C1 and C2. The first and second molecules can be added simultaneously or subsequently to the sample.

Hence, in all Gaplocks added in the form of a system of at least two or more different oligonucleotide molecules, the nucleic acid structure of step a) is provided when the first and second molecules are hybridized to the target nucleotide, providing a padlock in a circularizable form. When the Gaplock comprises sequence E and is thus added in step a) as a system of at least three or more different oligonucleotide molecules, the first, second and third molecules may be added to the sample separately (in any order) or they may be added already pre-hybridized to each other, and the nucleic acid structure of step a) with the padlock in a circularizable form is provided by hybridizing the first and second molecules with the target nucleic acid and the third molecule with the first molecule. Hence, the first, second and third molecules can be added prehybridized, simultaneously or subsequently to the sample.

It will be apparent to the skilled person that sequences A and C can be divided in two or more molecules, as long as all their ends are ligatable to each other and provide the circularizable padlock of the nucleic acid structure of a) when the different molecule hybridize to the target nucleic acid, so that in step b) the circularizable padlocks is capable of becoming a single circular padlock molecule. The length of the first, second, third, and further molecules is not limited, as long as they comprise the minimum number of nucleotides that allow a stable hybridization between each molecule and the target nucleic acid. Under standard conditions, said length is at least 6 nucleotides. This minimum length may be reduced to 5 or 4 nucleotides if the conditions are modified to favour hybridization.

### • Splitlocks:

These padlocks are shown in Example 11 and represented in Fig. 1H, Fig.1I and Fig.1J. They are padlocks wherein at least one division is performed in sequence B. Hence, in these padlocks, sequence B is provided in at least two different oligonucleotide molecules. Since the target nucleic acid does not hybridize with B, the addition of a skin (sequence E) is required to act as splint nucleic acid molecule to bridge between the two molecules comprising B sequence of the padlock, so that they can be ligated in step b).

One advantage of the Splitlocks relies in that the two or more different oligonucleotide molecules of the padlock comprising sequence A-B-C will only be ligated together and form the circular padlock when the first and second molecules have hybridized to the target nucleotide and when at least one third molecule, i.e. sequence E, has hybridized to both the first and second molecules, thereby bringing their ends together and providing the circularizable padlock o step a). This reduces the formation of padlock dimers (5'-A-B-C-A-B-C-3') where sequence A of one padlock hybridizes in the first portion of the target nucleic acid, but sequence C of the same padlock cannot hybridize in the second portion of the target nucleic acid, because said second portion is already occupied by sequence C of a different padlock molecule. If the A-B-C padlock sequence is added in the form of a system of at least two molecules, wherein sequence A and C are provided in different molecules, the formation of dimers of padlock will be limited as the ligase will join the two molecules independently hybridized to the target molecule by the A and C sequences and to sequence E by the respective B segments, thereby providing a single padlock according to the present invention. The Splitlock has the further advantage that, if the MDA primers are designed to hybridize each in a different molecule comprising sequence B, they only start the MDA amplification during step c) when the full padlock has been successfully hybridized and adopted a circularizable form providing the nucleic acid structure of step a).

In view of the above, in a preferred embodiment, sequences A, B, C and E of the Splitlock are added in the form of a system of at least three different molecules, wherein a division is placed in the B sequence and where sequence E (comprised in the third molecule) is preferably added after the hybridization of A and C sequences with the target nucleic acid. This Splitlock is also called herein single stranded Splitlock (ssSplitlock), represented in Fig. 1H, and comprises a first molecule comprising, in the 5' to 3' direction, sequence A and a first portion of sequence B (B1), a second molecule comprising, in the 5' to 3' direction, a second portion of sequence B (B2) and sequence C, and a third molecule comprising sequence E that hybridizes with sequences B1 and B2 or a portion of sequences B1 and B2. Consequently, in this preferred embodiment of a ssSplitlock, the first molecule comprises, in the 5' to 3' direction, sequences A-B1, the second molecule comprises, in the 5' to 3' direction, sequences B2-C, and the third molecule comprises E. In the case of the ssSplitlocks, the nucleic acid structure of step a) with the padlock in a circularizable form is provided by hybridizing the first and second molecules with the target nucleic acid and by hybridizing the third molecule to the first and second molecules. The circularizable padlock, when added as a ssSplitlocks, will present at least two nicks in its sequence, one placed at the junction region of sequences B1 and B2, and the other placed between sequences A and C. Figure 11a shows that a ssSplitlock approach was efficient in reducing background signal.

In this preferred embodiment of a ssSplitlock having a division in sequence B, it is preferred that the first and second different molecules comprising sequences 5'-A-B1-3' and 5'-B2-C-3', respectively, are added to the sample in step a) simultaneously, and then the third molecule comprising sequence E is added subsequently. The third molecule is preferably added after adding the first and second molecules so that it can bridge said molecules (acting as a splint nucleic acid molecule) and provide the circularizable padlock comprised in the nucleic acid structure of step a), which is then joined by ligation in step b). This specific order of adding the different molecules with ssSplitlock is favourable, since otherwise, the structures formed will not differ significantly from regular Skinlocks, and the advantage of reducing the formation of padlock dimers with respect to circularization events will be lost. The length of B1 and/or B2 is not limited as long as they are capable of hybridizing with E, and is preferably a least 6, 7, 8, 9, 10, 12, 14, 18, 20, 22, 26, 28, or 30 nucleotides in length. The length of sequences A, C and E is as defined in embodiments above. Preferably, B1 and B2 sequences contain the hybridization and reverse complementary sequences for each of the MDA primers.

In a preferred embodiment, the third molecule comprising sequence E is also divided in at least two different molecules. This Splitlock is called herein double-stranded Splitlocks (dsSplitlock), and is represented din Fig. 1I. In the dsSplitlocks, the padlock is added in the form of a system of at least four or more different molecules, wherein:
- a first molecule comprises, in the 5' to 3' direction, sequence A and a first portion of sequence B (B1),
- a second molecule comprises, in the 5' to 3' direction, a second portion of sequence B (B2) and sequence C,
- a third molecule comprising a first portion of sequence E (E1), wherein said first portion is preferably complementary to sequence B1 of the first molecule and
- a fourth molecule comprising a second portion of sequence E (E2), wherein said second portion is preferably complementary to sequence B2 of the second molecule.

Consequently, in this preferred embodiment of a dsSplitlock, the first molecule comprises, in the 5' to 3' direction, sequences A-B1, and the second molecule comprises, in the 5' to 3' direction, sequences B2-C, the third molecule comprises sequence E1 and the fourth molecule comprises sequence E2.

In the case of the dsSplitlocks, the nucleic acid structure of step a) with the padlock in a circularizable form is provided by hybridizing the first and second molecules with the target nucleic acid and by hybridizing the third molecule to the first molecule and the fourth molecule to the second molecule. Although in the case of the dsSplitlock the four molecules can be added in any order and manner, it is preferred that they are added simultaneously, and most preferred if the first molecule is added pre-hybridized to the third molecule (i.e, sequence B1 is pre-hybridized to sequence E1), and the second molecule is added pre-hybridized to the fourth molecule (i.e, sequence B2 is hybridized to sequence E2) so that when all molecules are added simultaneously, the nucleic acid structure of step a) with the padlock in a circularizable form is provided by only hybridizing the first and second molecules with the target nucleic acid.

The circularizable padlock in the nucleic acid structure of step a), when added as a dsSplitlock, will present at least three nicks: one in the junction region of sequences E1 and E2, another in the junction region of sequences B1 and B2, and another in the junction region of sequences A and C.

The length of E1 and E2 is not limiting if they are long enough to hybridize to sequence B1 and B2, i.e., they are of a minimum of 6 nucleotides in length. Preferably, E1 and E2 are 5, 8, 10, 12, 14 or 15 nucleotides in length. This system of at least four molecules may be designed so that the division in B and E coincide, in which case the junction between B1/E1 and B2/E2 "arms" will comprise blunt dsDNA ends. Preferably, by shifting the division in B and E, reverse-complementary or "sticky" overhangs in the dsDNA junction between B1/E1 and B2/E2 are formed, so that ligation and circularization are favoured. A padlock with 5' overhangs in the B1-E1/B2-E2 junction is represented in Fig. 1I, and a padlock with a 3' overhangs in the B1-E1/B2-E2 junction is represented in Fig. 1J. Thus, in a preferred embodiment, the nucleic acid structure comprises reverse-complementary overhangs in the junction between B1/E1 and B2/E2 to favour ligation and circularization, wherein the reverse-complementary overhangs comprise at least 1, 2, 3, 4, 5, 6, 7, 8, 9 or 10 nucleotides. It is also preferred that molecules comprising sequences B1 and B2 contain the hybridization and reverse complementary sequences for each of the MDA primers.

Thus, in a preferred embodiment, the dsSplitlock comprises sequences A-B-C and E, wherein said sequences are added in step a) in the form of a system of at least four different molecules, wherein
- a first molecule comprises, in the 5' to 3' direction, sequence A and a portion of sequence B (B1),
- a second molecule comprises, in the 5' to 3' direction, a second portion of sequence B (B2) and sequence C,
- a third molecule comprises a first portion of sequence E (E1), wherein said first portion is complementary to sequence B1 of the first molecule, and
- a fourth molecule comprises a second portion of sequence E (E2), wherein said second portion is complementary to sequence B2 of the second molecule, and
wherein the first and third molecules are preferably added pre-hybridized and the second and fourth molecules are preferably added pre-hybridized, so that the nucleic acid structure of step a) with the padlock in a circularizable form is provided by hybridizing the first and second molecules with the target nucleic acid and by alignment of the B1-E1/B2-E2 junction.

It will be apparent to the skilled person that sequence B can be at divided at any point throughout its sequence, as long as each of the resulting different molecules are capable of hybridizing to E or its fragments. The length of the first, second, third, and further molecules is not limited, as long as they comprise the minimum number of nucleotides that allow a stable hybridization between each molecule and with the target nucleic acid.

It will be understood that the system of different molecules is not incompatible with the presence of sequence D as defined above. Thus, the padlocks with additional sequences (Flaplocks) are combinable with the padlocks added in the form of a system of different molecules (Skinlocks, Gaplocks and Splitlocks, including ssSplitlocks and dsSplitlocks). In these systems of two or more molecules, the discontinuities can be located simultaneously in regions A/C and in B, leading to a combined Gaplock-Splitlock.

### Nucleotide sequences present in the padlock

The sequences A and C of the padlocks are chosen or selected with respect to the sequence of the target nucleic acid molecule to which they bind. The nucleotide sequence of the other sequences (sequences E, B, or D) is not critical, although it is preferably that they not only do not hybridize with the target nucleic acid but also do not hybridize with other nucleotides present in the reaction. Hence, the sequences should be chosen to avoid the occurrence of inter- and intramolecular hybridization. Once the sequence is selected or identified, the padlocks may be synthesized using any convenient method.

The padlocks described herein, in the form of a single molecule or as a system of two or more different molecules, may comprise one or more sequences which, when amplified after step c), serve to differentiate target-specific amplifications from unspecific or background amplifications. Said sequences are called herein "detectable templates" and are useful for the sequence-based detection method, as it will be explained in further detail below. The detectable templates may be present in any sequence of the padlock, including sequences A, B, C, D, and E.

Particularly preferred is that at least one detectable template is comprised within sequences A or C, and most preferred, in the junction between sequences A and C, so that it is only reconstituted in correctly assembled and ligated padlocks. This is particularly preferred in the case of Flaplocks and Gaplocks, in which target-independent reconstitution of the A-C sequence is prevented by the inclusion of additional nucleotides adjacent to A and/or C (D sequence), or by providing A and/or C sequences in different molecules, respectively. Flaplocks or Gaplocks that have not been properly assembled, processed and ligated will therefore not contain the correct detectable template, limiting target-independent background signal. In the case of Flaplocks, unspecific ligations will contain sequence D, disrupting thus the A-C junction and preventing the sequence-specific recognition step. For Gaplocks, target-independent reconstitution events will lack sequence A1 and/or C2 from the A-C junction, also preventing subsequent sequence-specific recognition. Thus, in these approaches, only correctly assembled, target dependent events will result in perfect reconstitution of the detectable template in the A-C junction, providing further specificity to the system.

The padlocks of the present invention may also comprise one or more, preferably two or more, sites for binding of a primer or the reverse complementary sequence, so that the primers used in the RCA reaction and/or in the MDA reaction of step c) hybridize in said sites of the padlock sequence and/or the product strands of the RCA and/or MDA reactions. In an embodiment, the primer sites are placed in sequence B of the padlock. It is preferred and advantageous that, if the padlock is a Splitlock wherein sequence B is comprised in two or more different molecules, the primer binding sites are each placed in different molecules comprising sequence B and its reverse complementary, so that only target-specific, circularized and ligated padlocks will be amplified by MDA.

### The target nucleic acid and the sample

As explained above, the method of the present invention is directed to detect a target nucleic acid in a sample. In a preferred embodiment, the target nucleic acid is indicative of the presence of an analyte of interest in the sample. By "analyte" is referred herein as to any molecule of interest that wants to be detected. It may include proteins, nucleic acids, peptides, etc. Thus, the detection of the target nucleic acid in the present method is an indication of the presence of the analyte in the sample. In some embodiments, the target nucleic acid may be used to directly or indirectly tag or label a target analyte in a sample and detection of the nucleic acid molecule serves to indicate the presence of the analyte in the sample. In some methods a new nucleic acid molecule may be generated in a sample (i.e. a nucleic acid molecule that was not present in the original sample and was not one of the components added to the sample) when one or more molecules that interact with, e.g. bind to, the target analyte.

In some other embodiments, the target nucleic acid is itself the analyte of interest. In this case, the target nucleic acid or analyte may be any nucleotide sequence it may be desired to detect, analyse or amplify. The target nucleic acid or analyte may be a nucleotide sequence derived from the analyte, such as the mRNA derived from the genome of a virus. Target nucleic acids may include DNA (e.g. genomic DNA, mitochondrial DNA, plasMid DNA, viral DNA etc.) and RNA (e.g. mRNA, microRNA, rRNA, snRNA, viral RNA etc.), and synthetic and/or modified nucleic acid molecules, (e.g. including nucleic acid domains comprising or consisting of synthetic or modified nucleotides such as LNA, PNA, morpholino etc.), or fragments thereof. Thus, the target nucleic acid or analyte may be made up of ribonucleotides and/or deoxyribonucleotides as well as synthetic nucleotides that are capable of participating in Watson-Crick type or analogous base pair interactions. Thus, the target nucleic acid or analyte may be or may comprise, e.g. bi-sulphite converted DNA, LNA, PNA or any other derivative containing a non-nucleotide backbone.

The target nucleic acid or analyte may thus be coding or non-coding DNA, for example genomic DNA or a sub-fraction thereof, or may be derived from genomic DNA, e.g. a copy or amplicon thereof, or it may be cDNA or a sub-fraction thereof, or an amplicon or copy thereof etc.

As noted above, the target nucleic acid or analyte may be a target RNA molecule. The target nucleic acid or analyte may, for example, be an RNA molecule in a pool of RNA or other nucleic acid molecules or nucleotide sequences, for example genomic nucleic acids, whether human or from any source, from a transcriptome, or any other nucleic acid (e.g. organelle nucleic acids, i.e. mitochondrial or plastid nucleic acids), whether naturally occurring or synthetic. The target RNA or analyte may thus be or may be derived from coding (i.e. pre-mRNA or mRNA) or non-coding RNA sequences (such as tRNA, rRNA, snoRNA, miRNA, siRNA, snRNA, exRNA, piRNA and long ncRNA). The target RNA or analyte may typically be an RNA molecule it is desired to detect in a sample, in the sense of being the target of the assay, i.e. an analyte. In one preferred embodiment, the target nucleic acid or analyte is a micro RNA (miRNA). In another preferred embodiment, the target RNA or analyte is 16S RNA, preferably wherein the 16S RNA is from and identificatory of a microorganism (e.g. a pathogenic microorganism) in a sample.

Alternatively, the target RNA or analyte may be genomic RNA, e.g. ssRNA or dsRNA of a virus having RNA as its genetic material. Such viruses include Ebola, HIV, SARS, influenza, hepatitis C, West Nile fever, polio and measles. Accordingly, the target RNA or analyte may be positive sense RNA, negative sense RNA, or double-stranded RNA from a viral genome, or positive-sense RNA from a retroviral RNA genome. Preferably, the target nucleic acid or analyte is the genome of a virus, preferably the genome of SARS-CoV-2 virus.

The target nucleic acid or analyte may be comprised in a sample. The sample may be any sample which contains the target nucleic acid or analyte, and includes both natural and synthetic samples, that is materials which occur naturally or preparations which have been made. All biological and clinical samples are included, e.g. any cell or tissue sample of an organism, or any body-fluid or preparation derived therefrom, as well as samples such as cell cultures, cell preparations, cell lysates etc. The samples may be freshly prepared, or they may be prior-treated in any convenient way e.g. for storage.

The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells. Exemplary biological samples include tissue samples (such as tissue sections and needle biopsies of a tissue); cell samples (e.g., cytological smears (such as Pap or blood smears) or samples of cells obtained by microdissection); samples of whole organisms (such as samples of yeasts or bacteria); or cell fractions, fragments or organelles (such as obtained by lysing cells and separating the components thereof by centrifugation or otherwise). Other examples of biological samples include blood, serum, urine, semen, fecal matter, cerebrospinal fluid, interstitial fluid, mucous, tears, sweat, pus, biopsied tissue (e.g., obtained by a surgical biopsy or needle biopsy), nipple aspirates, milk, vaginal fluid, saliva, swabs (such as buccal swabs), or any material containing biomolecules that is derived from a said biological sample. In some embodiments, the biological sample can be a body fluid, which can be fluid isolated from the body of an individual. For example, "body fluid" may include blood, plasma, serum, mucus, bile, saliva, nasopharyngeal swabs, urine, tears, perspiration, or washings from bodily cavities. Preferably, the sample is a biological sample, preferably saliva.

The biological sample may be obtained from a subject in need of the analysis. A "subject" may be a human (i.e., male or female of any age group, for example, pediatric subject (e.g., infant, child, or adolescent) or adult subject (e.g., young adult, middle-aged adult, or senior adult). Alternatively, the subject may be a non-human animal. In certain embodiments, the non-human animal is a mammal (e.g., primate, for example, cynomolgus monkey or rhesus monkey), commercially relevant mammal (e.g., cattle, pig, horse, sheep, goat, cat, or dog), or bird (e.g., commercially relevant bird, such as chicken, duck, goose, or turkey). In other examples, the non-human animal is a fish, reptile, or amphibian. The non-human animal may be a transgenic animal or genetically engineered animal. In some examples, the subject may also be a plant.

In one particular embodiment the sample comprises microbial cells or viruses which have been isolated from a clinical sample or from a culture of a clinical sample, such as saliva. In one particular embodiment the sample comprises microbial cells or viruses which have been isolated from a food or water sample or from a culture of a food or water sample. In such a sample the target nucleic acid sequence for a padlock may be a nucleotide sequence present in a microbial cell or virus, e.g. a nucleotide sequence which is characteristic for, or discriminatory or identificatory of a microbial cell or virus, at any level, e.g. at type, group, class, genus, species or strain level. In an embodiment the target nucleic acid or analyte is the genome of an infectious agent. By "infectious agent" is meant herein a pathogen that can produce a disease in the subject in need of the analysis, preferably in humans. Preferably, the target nucleic acid or analyte is a viral genome, preferably a single-stranded viral genome, preferably RNA single-stranded viral genome. In an embodiment according to the first aspect or any of its embodiments, the viral genome is SARS-CoV-2 virus genome.

In an embodiment, the nucleic acid structure of a) may be DNA-RNA structure if target is RNA and the padlock is DNA. It may also be a DNA-RNA hybrid if sequences A-B-C are provided in the form of DNA, but sequence E is a RNA molecule.

In an embodiment, the padlock of the present invention comprises, consists, or consists essentially of SEQ ID NO: 8, 16, 17, 18, 19, 20, 23, 24, 25, 26, 27 or 32 or a sequence with at least 85%, 86%, 87%, 88%, 89%, 90%, 91%, 92%, 93%, 94%, 95%, 96%, 97%, 98%, 99% or 100% sequence identity to SEQ ID NO: 8, 16, 17, 18, 19, 20, 23, 24, 25, 26, 27 or 32.

### Step b) of the method of the present invention comprises or consists of circularising the hybridised padlock

Step b) comprises subjecting the circularizable padlock comprised in the nucleic acid structure of step a) to a ligation reaction using at least one ligase to provide a circular padlock comprising sequences A-B-C in a single circular molecule that does not comprise any nick in its sequence. A circular padlock is a covalently-closed single-stranded DNA circular probe. The ligation step preferably occurs after step a), that is, after the nucleic acid structure is provided. However, it is noted that reaction a) and b) may be overlapping, i.e., ligation step may start before step a) if fully completed.

In all padlocks, the target nucleic acid acts as a splint nucleic acid molecule to facilitate ligation between sequences A and/or C. If the padlock is contacted with the target nucleic acid comprising sequences A-B-C in a single molecule (i.e., without any division), the padlock will hybridise to the target nucleic acid and its 3' and 5' ends will be directly juxtaposed for ligation to each other using the target nucleic acid as a ligation template (i.e., as a splint nucleic acid molecule), without any further modification or processing (e.g., cleavage or further hybridization) of the padlock. Preferably, in the case where sequences A-B-C are provided as a single molecule, step b) of the present invention comprises or consists of a ligation step comprising ligating the 5' end of sequence A with the 3' end of sequence C, without any further steps being required to provide a ligated circularized probe comprising sequences A-B-C in a single circular molecule.

However, for the formation of the nucleic acid structure of step a), it is not required that sequences A-B-C are provided in a single molecule, since it is possible that one or both of the ligatable ends of sequences A and/or C are generated in one or more processing or hybridization steps, as explained above. In some embodiments, sequences A-B-C are provided in two or more different molecules or parts, e.g., two, three, four, five or more different molecules, which create two or more ligation junctions. This is the case of, for example, Gaplocks, ssSplitlocks and dsSplitlocks. Each molecule of the system may provide (i.e., form or comprise) 3' and/or 5' ligatable ends which may be juxtaposed for ligation and be ligated in step b) of the methods of the present invention as outlined above. In certain embodiments, step b) may, therefore, comprise two or more different ligation reactions, i.e., between adjacent ligatable ends provided by the two or more different molecules. In this case, the ligation step comprises more than one ligation reaction to join the two or more different molecules comprising sequences A, B, and/or C in a single circular molecule. As explained above, the different molecules comprising sequences A-B-C will comprise ligatable ends and thus will be able to be ligated in step b). There will be one ligation reaction for each nick between the molecules comprising sequences A, B, and/or C.

Preferably, when the padlock is added in the form of a system of two different molecules wherein sequences A-B-C are divided in said two molecules (such as in the case of Gaplocks and Splitlocks), and once the nucleic acid structure of step a) is provided, the ligation step b) comprises or consists of providing a padlock with sequences A-B-C in a single circular molecule by ligating the 5' end of the first molecule with the 3' end of the second molecule, and by ligating the 5' end of the second molecule with the 3' end of the first molecule, using at least one ligase. In the case of padlocks where sequences A-B-C are added in different oligonucleotide molecules, the ligation to provide sequences A-B-C in a single circular molecule may be performed simultaneously or subsequently, preferably after the molecules have hybridized to the target nucleic acid and have formed a nucleic acid structure of step a).

As mentioned above, the only requirement for the system of different molecules to work is that A-B-C sequences are reconstituted as a single circular molecule in the presence of the target nucleic acid and by the action of a ligase in step b). Hence, sequence E, if present (i.e. Skinlocks and Splitlocks), does not have to be circularized, reconstituted or ligated to any other sequence, even if E is added divided in different molecules (i.e. dsSplitlocks).

In the particular case of Flaplocks, with or without skin, the target nucleic acid acts as a splint nucleic acid molecule for the ligation between sequences A and C. However, ligation only happens after sequence D has been removed from the padlock and thus the padlock in a circularizable form has been formed.

In the case of Gaplocks, with and without skin or sequence E, the target nucleic acid acts as a splint nucleic acid molecule for the ligation of sequences A1 and A2 and/or C1 and C2. Alternatively, in some embodiments of Gaplocks, the space in between the free ends of the first molecule may be "filled-in" by extending the free 3' end, e.g. in a polymerase reaction, using the target nucleic acid molecule as an extension template. Once the free 3' end has been extended to be adjacent to the free 5' end, the two ends may be joined by a ligation reaction.

In the case of ssSplitlocks, sequence E will act as splint nucleic acid molecule for ligating the nick between B1 and B2, and the target nucleic acid will act as splint nucleic acid molecule for ligating the nick between A and C.

Also, in dsSplitlocks, the target nucleic acid acts as a splint nucleic acid molecule for ligation of sequences A and C, and sequence E acts to allow alignment of B1 and B2 sequences in the B1-E1/B2-E2 junction. However, the third (E1) and fourth (E2) molecules may be or may not be ligatable to each other. If they are ligated, then the third and fourth molecules are joined by ligation of the 5' end of the third molecule (E1) with the 3' end of the fourth (E2) molecule, thereby providing sequence E as a single molecule that is hybridized to sequence B, or a region thereof, of the padlock. However, as depicted in Fig. 1J, it may be advantageous to prevent ligation of the third (E1) and fourth (E2) molecules of sequence E, and provide a third molecule (E1) with a modified 3' end so it is not capable to prime the RCA reaction of step c). This way, only the dsSplitlocks that hybridize to the target nucleic acid, and in which B1 and B2 are ligated, contain the 3' end of E2 to trigger the RCA reaction, thereby providing a further level of specificity. Thus, ligation of sequences E1 and E2 is not mandatory for the method to work. Ligation of the 5' end of the third molecule with the 3' end of the fourth molecule may be prevented by removing the phosphate group from the 5' end of E1 or by including a non-ligatable modification, and or by providing E1 and E2 molecules that are not juxtaposed (i.e. they leave a gap of at least 1 nucleotide) when arranged in the circularizable form.

Hence, in the case of dsSplitlocks, once the nucleic acid structure of step a) with the circularizable padlock is provided, step b) comprises ligation of the 5' end of the first molecule with the 3' end of the second molecule, and the 3' end of the first molecule with the 5' end of the second molecule, wherein:
the third and fourth molecules are also joined by ligation of the 5' end of the third molecule with the 3' end of the fourth molecule, thereby forming a padlock comprising sequences A-B-C in a single circular molecule and further comprising sequence E in the form of a single molecule hybridized to sequence B or a region thereof, or wherein
the third and fourth molecules are not joined by ligation of the 5' end of the third molecule with the 3' end of the fourth molecule due to the presence of a non-ligatable 5' end in the third molecule, thereby forming a padlock comprising sequences A-B-C in a single circular molecule and further comprising sequence E in the form of two molecules hybridized to sequence B or a region thereof. By non-ligatable is referred in the context of the present invention as a 5' or 3' end that cannot form a phosphodiester bond between adjacent nucleotides by action of a ligase, and/or that the 5' and 3' ends are not adjacent, or sufficiently adjacent for the ligase to operate. In a preferred embodiment, the non-ligatable 5' of the third molecule lacks a phosphate group.

In an embodiment, the ligation of the 5' and 3' ends of the molecule or molecules comprising sequences A, B and C of the padlock of the present invention is carried out by action of at least one ligase. As is known in the art, in template-directed ligation, ligases catalyse the formation of a phosphodiester bond between juxtaposed 3'-hydroxyl and 5'- phosphate termini of two immediately adjacent nucleic acids when they are annealed or hybridized to a third nucleic acid sequence to which they are complementary (i.e. a ligation template or splint nucleic acid molecule). As it is also known in the art, there may be ligations that are not template-directed, such as the ligation happening in case of dsSplitlocks where the division in E1 and E2 is placed in the same location within the padlock as the division in B1 and B2, thereby creating blunt ends that are ligated by ligases, such as T4 ligase, capable of template-independent ligation.

Any convenient ligase may be employed, where representative ligases of interest include, but are not limited to: temperature sensitive and thermostable ligases. Temperature sensitive ligases include, but are not limited to, bacteriophage T4 DNA ligase, bacteriophage T7 ligase, and E. coli ligase. Thermostable ligases include, but are not limited to, Taq ligase, Tth ligase, Ampligase^{®} and Pfu ligase. Thermostable ligase may be obtained from thermophilic or hyperthermophilic organisms, including but not limited to, prokaryotic, eukaryotic, or archaeal organisms. Certain RNA ligases may also be employed in the methods of the invention.

A suitable ligase and any reagents that are necessary and/or desirable may be combined with the reaction mixture and maintained under conditions sufficient for ligation of the hybridized oligonucleotides to occur. Ligation reaction conditions are well known to those of skill in the art.

In a representative embodiment, the ligation reaction mixture includes 50 mM Tris pH7.5, 10 mM MgCl2, 10 mM DTT, 1 mM ATP, 25 mg/ml BSA, 0.25 units/ml RNase inhibitor, and T4 DNA ligase at 0.2 units/ml.

It will be evident that the ligation conditions may depend on the ligase enzyme used in the methods of the invention. Hence, the above-described ligation conditions are merely a representative example and the parameters may be varied according to well-known protocols. Such manipulation of the assay methods is routine in the art.

In a preferred embodiment, when the target is single-stranded RNA, the ligase preferably used is the DNA Ligase from *Paramecium bursaria Chlorella Virus-1* (also known as SplintR), represented by SEQ ID NO: 1. In an embodiment, the ligase is the DNA Ligase from *Paramecium bursaria Chlorella Virus-1* comprising or consisting of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1 and is capable of ligating the 5' end of A with the 3' end of C once the padlock molecule or system of two or more different molecules has hybridized to the target ribonucleic acid. Thus, it is preferred that SplintR ligase is used in step b) of the method of the present invention. This ligase is also capable of ligating additional nicks in the sequence of the circularizable padlock probe when it is added in the form of a system of different molecules, provided that they are templated by an RNA splint nucleic acid molecule. The SplitR ligase is also preferably used to ligate sequences B1 and B2 when sequence E is provided as a RNA molecule.

Hence, when the splint nucleic acid molecule (such as the target nucleic acid or sequence E) is an RNA molecule, a SplintR ligase is preferably used in the ligation reaction of step b). However, if the splint nucleic acid molecule is a DNA molecule (for instance, when sequence E is provided as a DNA molecule), a T4 DNA ligase is preferably used in the ligation reaction of step b). If the reaction comprises both DNA and RNA splint nucleic acid molecules (for instance, a target nucleic acid that is a RNA molecule and a sequence E that is a DNA molecule), two ligases may be added, preferably SplinR and T4 DNA ligases.

Step c) of the method of the present invention comprises or consists of subjecting the ligated circularized padlock to rolling circle amplification (RCA).

The ligated circular padlock (or part thereof) obtained in step b) is then amplified by rolling circle amplification (RCA). Step c) thus comprises at least an RCA reaction. Said amplification is in order to increase the copy number of the ligation product in a sample, which increases the sensitivity of the detection method of the present invention. In certain embodiments, only a part or portion of the ligated circular padlock is amplified.

Rolling circle amplification using a circular template provides a concatenated RCA product comprising multiple tandem repeats, each having a sequence complementary to the circular template oligonucleotide (i.e. the circular padlock). RCA may be triggered directly from the hybridized nucleic acid of interest, provided that a hybridized 3' end is provided. This can occur directly, by designing the padlock so it hybridizes with the 3' end of the nucleic acid of interest, or by a nucleolytic treatment, preferably by a 3' exonucleolytic activity, which can be linked to the polymerase used for the RCA reaction, or by endonucleolytic nicking of the target molecule hybridized to the padlock, as for example with mild treatment with RNAse H in the case of target RNA molecules. Alternatively, RCA may occur by contacting the sample with at least one RCA primer complementary to and capable of hybridising to the circularized padlock, so the 3' end of said at least one primer may be extended to provide a concatenated RCA product. Said RCA primer(s) is/are characterized by having between 6-80 nucleotides, preferably between 10-50, 10-40, 10-30, 10-20 nucleotides in length. Preferably, said primer or primers hybridize to a region of sequence B.

In an embodiment, the padlock comprises sequence E and the RCA reaction uses the 3' free end of sequence E, or a fragment thereof (e.g. E2), as a primer. In an embodiment, the padlock comprises sequence E, but the RCA reaction is triggered by the target nucleic acid or an additional primer; preferably, this primer anneals to a part of sequence B not hybridized with E.

Amplification of the ligated circular padlock is performed using a DNA polymerase enzyme, i.e. a polymerase enzyme capable of synthesising a DNA oligonucleotide from dNTPs. The term "DNA polymerase" as used herein includes any enzyme capable of incorporating dNTPs, and hence includes enzymes which have both DNA and RNA polymerase activity. In particular, the DNA polymerase may be an enzyme whose principal activity or function is to synthesise DNA, but which is also capable of incorporating RNA nucleotides.

The RCA reaction of step c) is preferably carried out by a bacteriophage phi29 DNA polymerase. Bacteriophage phi29 DNA polymerase shows unique properties that enable its application in numerous DNA amplification and DNA sequencing technologies and platforms: highly processive DNA synthesis; exceptional strand-displacement, which allows polymerization coupled to the unwinding of double-stranded DNA, in the absence of helicase-type enzymes; high fidelity of synthesis, with very low error insertion rates and efficient proofreading of inserted errors, which collectively enhance fidelity.

By "a bacteriophage phi29 DNA polymerase" is understood herein to include the wild-type Phi29 DNA polymerase, represented by SEQ ID NO: 2, but also any variant, chimera, truncated version of said polymerase. Thus, the term "a bacteriophage phi29 DNA polymerase" includes polymerases which have been genetically modified, as well as those which are substantially identical to a naturally-occurring phi29-type DNA polymerase or a modified polymerase thereof, or to the equivalent enzymes. By substantially identical is meant that the enzyme may contain amino acid substitutions which do not affect the above-mentioned properties of the enzyme.

In a preferred embodiment, the bacteriophage phi29 polymerase is an improved chimeric phi29 polymerase that comprises the entire amino acid sequence of phi29 wildtype of SEQ ID NO: 3, but it further comprises a M. kandleri Topo V (HhH)₂ domain H (residues 696-751) or H and I (residues 696-802) fused to the C terminus of phi29 DNA polymerase. This chimeric phi29 DNA polymerase, also named Qualiphi polymerase, presents enhanced DNA binding and thus is a preferred phi29 DNA polymerase to be used in the method of the present invention. The Qualiphi polymerase is represented by SEQ ID NO: 3.

In another preferred embodiment, the bacteriophage phi29 DNA polymerase is improved by several amino acid substitutions as described in Fig. 3 of Povilaitis, T., et al., In vitro evolution of phi29 DNA polymerase using isothermal compartmentalized self-replication technique, Protein Eng. Des. Select. 29, 617-628, 2016, also called herein Equiphi.

Thus, in an embodiment, the bacteriophage phi29 DNA polymerase used in step c) of the method of the present invention is a bacteriophage phi29 enzyme comprising or consisting of SEQ ID NOs: 2 or 3 or a sequence with at least 75%, 80%, 85%, 89%, 90%, 91%, 92%, 95%, 97%, 98%, 99%, or 100% identity to the nucleotide sequence as defined in SEQ ID NOs: 2 or 3 respectively. Preferably, the bacteriophage phi29 DNA polymerase used in step c) of the method of the present invention is a bacteriophage phi29 DNA polymerase enzyme comprising or consisting of SEQ ID NO: 3. Of note, a certain % of variation over SEQ ID NOs: 2 or 3 are included and allowed in the method of the present invention provided that the variant of the a bacteriophage phi29 DNA polymerase maintains or improves the 3'-5' exonuclease, processivity and strand-displacement DNA polymerase activity of the corresponding phi29 of SEQ ID NOs 2 or 3.

In an embodiment, the bacteriophage phi29 DNA polymerase used in step c) of the method of the present invention is a bacteriophage phi29 DNA polymerase comprising or consisting of SEQ ID NO: 2, or a sequence with at least 75%, 80%, 85%, 89%, 90%, 91%, 92%, 95%, 97%, 98%, 99%, or 100% identity to the nucleotide sequence as defined in SEQ ID NOs: 2, wherein said sequence comprises at least the amino acid substitutions F526L, M97T, G197D, E221K, Q497P, V51A, K512E, M8R, L123S, K209E, E515A, E239G. It is noted that, when referred to the amino acid substitutions in the context of a sequence with certain % of identity to SEQ ID NO: 2, it is to be understood that said % of identity allows for modifications of the SEQ ID NO: 2, as long as the specific amino acid substitutions mentioned herein are maintained. It is also noted that here and throughout the whole document, the positions or location of the amino acid residues of a protein or a polypeptide sequence are numbered sequentially starting from the first amino acid residue at the N-terminal end which would then be located at position 1. For example, a protein of 137 amino acids will have those residues numbered 1 (first amino acid residue located in the N-terminal end of the protein) until 137 (the last amino acid residue, located in the C-terminal end of the protein). Thus, the numbering preferably starts from the first amino acid residue at the N-terminal end of the protein or polypeptide and ends at the C-terminal end of the protein or polypeptide. Preferably, the positions of the amino acid residues are numbered using the amino acid sequence of the translated mature protein.

Step c) comprises or consist of performing at least one RCA reaction using as a template the circularized padlock and preferably using a bacteriophage phi29 DNA polymerase as defined above.

The RCA amplification comprises at least a linear RCA reaction using the RCA template(s) formed in step (b) to form first rolling circle amplification product(s) (RCA product), wherein a first RCA product is a concatemer comprising monomers which are complementary to the circularised padlock which templated its formation. Suitable conditions to carry out said RCA reaction are known in the art. The RCA product may be the product of a primary (i.e. initial) RCA reaction, or it may be the product of a further or later RCA reaction. The second RCA reaction may be a secondary or further, or later, RCA reaction. It will thus be understood that the method of the invention may involve multiple, successive rounds of RCA, e.g. two, three, four or more, wherein in each round a probe is used which is hybridised, directly or indirectly, to the reaction product of a previous round of RCA. Expressed in other words, the method of the invention may comprise repeating steps (a), (b) and (c) one or more times, and/or performing steps (a), (b) and/or (c) simultaneously.

In some embodiments, e.g. in situ assays or other assays in which the target nucleic acid is immobilised, wash steps may be included between the addition of probe and ligation and/or amplification of the ligation product. In other words, a target nucleic acid molecule may be captured or immobilised on a solid support or substrate, which may be washed to remove unbound or non-specifically bound probe. In some embodiments, wash steps may be included between ligating the probe and amplification of the ligation product, to remove unligated probes. In other representative embodiments a wash step may be included before ligation takes place. Where the probe is a multi-part padlock, the backbone oligonucleotide a may be contacted with the sample and allowed to hybridise, and the gap oligonucleotide(s) may then be added, if used, and allowed to hybridise.

The resulting product of the RCA reaction described above is a linear RCA product that is made of concatemers of the complementary sequence of the padlock of the present invention. This linear RCA product may be optionally used as a template for a further amplification reaction (MDA reaction).

Hence, in an embodiment, step c) comprises an RCA reaction followed by a MDA reaction, where the product of the RCA reaction is exponentially amplified. For the MDA reaction, at least two primers are used. Said primers, herein referred to as MDA primers, are characterized by having a maximum of 15 nucleotides in length, but preferably have less than 14, 13, 12, 11, 10, 9, 8, 7, 6 nucleotides in length. The advantage of providing short MDA primers relies in favouring the strong amplification characteristic of this method, so that the target nucleic acid, even if present in very low amount, is amplified. At least one MDA primer (MDA1) is reverse complementary, and hybridizes to, the A-B-C sequence of the circularized padlock, preferably to region B. This primer may be the same one used to trigger the RCA reaction, as described above. At least one MDA primer (MDA2) contains a sequence comprised in the A-B-C circularized padlock, preferably in region B, and is therefore reverse complementary, and hybridizes to, the RCA product. If the B sequence is split in two or more different molecules, it is preferable that each MDA primer anneals or hybridizes to a different molecule of the B sequence or its reverse complementary, such as one primer annealing in sequence B1 (MDA1) and the other primer annealing in the reverse complementary of sequence B2 (MDA2). Thus, in a preferred embodiment, step c) comprises amplifying the RCA product by using two primers having a maximum length of 15, preferably of 10 or less nucleotides, wherein the padlock is added in the form of a system of two different molecules, wherein a division is performed in sequence B, and wherein one of the primers hybridizes in portion B1, and the other primer hybridizes in a reverse complementary sequence of B2.

This MDA reaction may be performed subsequent to, or more preferably, simultaneously to the RCA reaction. Thus, in a preferred embodiment, step c) comprises a HRCA reaction, which is an amplification reaction which combines a linear RCA reaction with a MDA reaction (HRCA is also known as strand displacement cascade reaction). Typically, HRCA is performed by producing a first-generation RCA product primed from either the hybridized target molecule or a primer that anneals to the padlock (e.g. the MDA1 primer or the skin, as described above). The RCA product, being a concatemeric ssDNA molecule, contains multiple in-tandem priming sites for the MDA2 primer, so an MDA reaction is triggered in which each polymerase extending from a given primer displaces and releases all the products that are being produced downstream of it, converting the RCA product into dsDNA and resulting in a second-generation of ssDNA molecules with the same polarity of the padlock, each containing a variable number of A-B-C sequences, from one to the number of repeats contained in the RCA product (i.e. 1, 2, 3, ... n). Furthermore, each of these ssDNA molecules contain priming sites for MDA1, so a further MDA reaction is triggered as described above, converting the second-generation ssDNA molecules into dsDNA and releasing a set of third-generation ssDNA molecules, again, as in the first-generation RCA product, with the reverse polarity of the padlock. This can go on in multiple cycles, alternating priming by MDA2 and MDA1, while the RCA reaction is active and/or ssDNA is being released by MDA. The final result is an exponentially amplified mixed dsDNA and ssDNA product.

In an embodiment, the polymerase carrying out the MDA reaction is the same as used in the RCA reaction, which was defined above.

Thus, in the methods of the present invention, the RCA reaction of step c) may be an HRCA assay, wherein the linear RCA reaction takes place as part of the HRCA reaction and wherein said HRCA reaction takes place prior to detection step d).

Step d) of the method of the present invention comprises or consists of detecting the amplified product, thereby detecting the target nucleic acid.

Step d) comprises the detection of the amplified product. It is noted that the molecule that is detected is the amplified product of step c), and not the target nucleic acid. However, the detection of the amplified product indicates that the target nucleic acid or analyte is present in the sample. This step may be performed subsequently to step c), or at the same time, as the amplified products are produced. Steps a) to c) are preferably carried out for a suitable amount of time until a detectable signal is obtained.

The term "detecting" is used broadly herein to include any means of determining the presence or absence of the target nucleic acid (i.e. if it is present or not) or any form of measurement. Since the amplification in step c) was strong, and may have given rise to unspecific amplicons, the detection step is preferably based on sequence, i.e., is a sequence-specific detection step. A sequence-specific method will therefore allow the specific detection of the amplicons that are target-specific, thereby increasing the specificity of the method and disregarding the background that may have been generated during the strong amplification with short primers of step c). The region specifically identified by the sequence-specific detection method is referred herein as "detectable sequence" and is the complementary sequence of a sequence located in the padlock probe, also called herein "detectable template". The detectable template is included, as a whole or in fragments, in the padlock sequence, so that the product of correctly assembled padlocks amplified during step c) will comprise its reverse complementary sequence, and therefore the detectable sequence. One or more detectable templates can be included in the padlock sequence.

In these embodiments, the signal producing system may include a nucleic acid or oligonucleotide that specifically binds to a sequence found in the amplified product (i.e. the detectable sequence), where the nucleic acid/oligonucleotide may be labelled with a directly or indirectly detectable label.

A directly detectable label is one that can be directly detected without the use of additional reagents, while an indirectly detectable label is one that is detectable by employing one or more additional reagents, e.g., where the label is a member of a signal producing system made up of two or more components.

In many embodiments, the label is a directly detectable label, where directly detectable labels of interest include, but are not limited to: fluorescent labels, radioisotopic labels, chemiluminescent labels, and the like. In many embodiments, the label is a fluorescent label, where the labelling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labelled probe nucleic acids (i.e. detection probes) include, but are not limited to: fluorescein, the cyanine dyes, such as Cy3, Cy5, Alexa 555, Bodipy 630/650, and the like. Other labels, such as those described above, may also be employed as are known in the art.

In an embodiment, the sequence-specific detection step d) uses a labelled nucleic acid that binds to the detectable sequence and emits a detectable signal. In certain embodiments, the specifically labelled nucleic acids (detection probes) are labelled with "energy transfer" labels. As used herein, "energy transfer" refers to the process by which the fluorescence emission of a fluorescent group is altered by a fluorescence-modifying group. Energy transfer labels are well known in the art, and such labelled oligonucleotide probes include the TaqMan^{®} type probes, Scorpion probes, Sunrise probes , and conformationally assisted probes , among others.

In another embodiment, the sequence-specific detection step uses a Crispr/Cas system, where a guide RNA that specifically binds to the detectable sequence and said specific binding results in the emission of a detectable signal. Preferably, the Cas protein is a Cas12 protein, more preferably Cas12a, and most preferably LbaCas12a (Cas12a (Cpf1) from *Lachnospiraceae bacterium* ND20006). In a preferred embodiment, the detectable template contains a protospacer adjacent motif (PAM) sequence, so that the detectable sequence resulting from step c) can be recognized in both ssDNA and dsDNA forms. Preferably, said PAM sequence within the detectable template is included in sequences A, B and/or C of the padlock probe, and the sequence specific detection method is CRISPR/Cas, preferably CRISPR/Cas12a, and most preferably CRISPr/LbaCas12a. Preferably, at least one detectable template comprising a PAM sequence is located in regions A and/or C.

As a general rule, the detectable template is preferably placed in the junction region of sequence A and C, so that it is only reconstituted upon the ligation of sequences A and C that characterizes the presence of the target nucleic acid. By "junction region" is referred herein as the region of the sequence of the padlock where there is a nick; for instance, by "junction region of sequence A and C" is referred herein as a nucleotide sequence comprising the 5' end of region A and the 3' end of region C, so that the junction region is only formed when regions A and C are joined by the action of a ligase. In other words, the junction region of sequences A and C is the region where sequences A and C ligate. Since the padlock sequences may have more than one nick, there may be more than one junction region.

If the padlock is a Flaplock, it is preferred that the detectable template is placed in the junction region of sequences A and C, so that only the padlocks whose sequence D has been removed allow ligation between A and C and result in the production of detectable sequences during step c).

If the padlock is a Gaplock, the detectable template is preferably placed in at least one of the junction regions of sequence A and/or C, more preferably in at least one the junction regions of the sequences that have been provided in different molecules. For example, in the case of Gaplocks where a division is performed in sequence A, it is preferable that a detectable template is placed in the junction region of sequences A1 and A2, or in the junction region of sequences A2 and C, or both. In the case of Gaplocks where a division is performed in sequence C, it is preferable that a detectable template is placed in the junction region of sequences C1 and C2, or in the junction region of sequences C2 and A, or both. In the case of Gaplocks where a division is performed in sequence A and C, it is preferable that a detectable template is placed in the junction region of sequences C1 and C2, or in the junction region of sequences A1 and A2, or both. Since said sequences (A1 and A2, C1 and C2, or A1 and C2) are placed in different molecules, i.e., in the first and second molecule, respectively, a detectable template in the case of Gaplocks is preferably placed in the junction region of the first and second molecules, that is formed by ligation of the first and second molecules in step b). This way, only the amplification of those padlocks that have been completed and circularized upon hybridization of the target nucleic acid will result in the production of detectable sequences during step c). Since Gaplocks have at least two nicks, they will have at least two junction regions where a detectable sequence can be placed.

If the padlock is a Splitlock, including both ssSplitlocks and dsSplitlocks, with a division in sequence B, a detectable template is still preferably placed in the junction region of sequences A and C, as their ligation is the event that is most directly linked with the presence of the target nucleic acid. Since said sequences (A and B) are placed in different molecules, i.e., in the first and second molecule, respectively, a detectable template in the case of Gaplocks is preferably placed in the junction region of the first and second molecules, that is formed by ligation of the first and second molecules in step b).

Alternatively, other detection methods that are not sequence-specific may be used. This would be particularly useful when an optimized padlock as the ones defined herein is used. Not sequence-specific methods for detecting nucleic acids that are known in the art, such as the use of intercalating agents that label the amplified product and release a detectable signal.

The detection step may include the measurement of the signal released from the detection and determining the presence of the target nucleic acid in the sample based on the intensity of the signal. Detectable said signal may be a fluorogenic or colorimetric signal, among others. The detection step may include a quantification step in which the levels (concentration) of the target nucleic acid are obtained. To measure the concentration of a target nucleic acid in a sample, a calibration curve may be developed using samples containing known concentrations of the target nucleic acid molecule. The concentration of the target nucleic acid in a sample may be determined by comparison of a measured parameter to a calibration standard. In some cases, a calibration curve may be prepared, wherein the total measured signal is determined for a plurality of samples comprising the target nucleic acid at a known concentration using a substantially similar assay format. For example, the total intensity of the array may be compared to a calibration curve to determine a measure of the concentration of the target nucleic acid in the sample. The calibration curve may be produced by completing the method with a plurality of standardized samples of known concentration under similar conditions used to analyse test samples with unknown concentrations. A calibration curve may be used to relate the detected signal of the target nucleic acid complex with a known concentration of the target nucleic acid. The assay may then be completed on a sample containing the target nucleic acid or fragment in an unknown concentration, and the signals detected may be compared to the calibration curve, (or a mathematical equation fitting the same) to determine a measure of the concentration of the target nucleic acid in the sample.

The method described herein can also be used to detect several targets present in a sample or measure the levels of two or more targets (multiplex assay) of a sample. In this case, the two or more target nucleic acids may then be detected using different padlocks. In an embodiment, two or more padlocks are used, wherein each of the padlock used is capable of hybridising to a different nucleic acid of interest. In other words, each of the padlocks have different sequences A and C in its 5' and 3' regions which direct it to hybridise (bind) to a different target nucleic acid sequence present in the sample (thus to indicate the presence thereof). The use of two or more padlocks in a multiplexed detection method of the present invention accordingly allows the detection of two or more different target nucleic acid in a sample. As each target nucleic acid is different, each padlock may thereby be considered to be 'for' detecting that target nucleic acid, or the analyte for which the target nucleic acid is representative.

Multiplex assays may involve the detection of tens, hundreds, thousands or even tens of thousands of target nucleic acids or analytes in a sample. In this case, it is preferred that each padlock used to detect each target nucleic acid or analyte comprises in its sequence a tag sequence, e.g. a barcode or identificatory motif, or a binding site for a detection probe or primer. Such a tag sequence may be found, for example, at a 3' or a 5' end of a padlock or in one of the different molecules or may be found intermediate to an end of a padlock or different molecules, for example, in a portion of a circularisable backbone oligonucleotide not hybridised to a target nucleic acid molecule. Tags such as barcodes may be designed with different needs/purposes, for example, to introduce a universal or common sequence to enable different ligated padlocks in a multiplex setting to be processed together. This would enable different ligated padlocks to be amplified together, e.g. in a library amplification by RCA. Alternatively, or additionally, a tag/barcode sequence may be used to "label" different amplified ligated padlocks so that they may be readily distinguished from one another (i.e. a "target" tag or marker), or to tag different samples etc. so that they may be pooled prior to a common/universal amplification step (i.e. a "sample" tag or marker). Thus, in a multiplex setting, different padlocks (i.e. padlocks for different target nucleic acid) may be provided with different tag sequences (e.g. different marker or detection sequences) and/or they may be provided with the same tag sequence(s) e.g. for the introduction of a common or universal sequence.

In a multiplexed reaction, for example, in a sample suspected of containing three different target nucleic acid or analytes, the detection step may comprise (i) measuring a first signal released from a first amplification product derived of the amplification of a first padlock and determining the presence or a level of a first target nucleic acid in the sample based on the intensity of the first signal; (ii) measuring a second signal released from a second amplification product derived from the amplification of a second padlock and determining the presence or a level of the second target nucleic acid in the sample based on the intensity of the second signal; and (iii) measuring a third signal released from a third amplification product derived from the amplification of a third padlock and determining the presence or a level of the third target nucleic acid in the sample based on the intensity of the second signal.

In other embodiments, a single target nucleic acid or analyte of interest may be represented by two or more different target nucleic acid sequences, such that detection of any one or more of the target nucleic acid sequences that are representative of that target nucleic acid or analyte will indicate the presence of that nucleic acid of interest in the sample.

The amplified ligated probe may be detected using any of the well- established methods for analysis of nucleic acid molecules known from the literature including liquid chromatography, electrophoresis, mass spectrometry, microscopy, real-time PCR, fluorescent probes, microarray, colorimetric analysis such as ELISA, flow cytometry, lateral flows, mass spectrometry (CyTOF) etc. This may require immobilisation of the target nucleic acid molecule, for example in in situ detection procedures, or the inmobilization of the padlock probe or the amplified product. The use of solid phase assays offers advantages, particularly for the detection of difficult samples: washing steps can assist in the removal of unbound and/or unligated probes etc., inhibiting components, and target molecules can be enriched from an undesirably large sample volume. Higher concentrations and greater amounts of probes can be used, as unbound probes and RNA molecules can be removed by washing.

### Other Improvements

The authors of the present invention also found improvements of the conditions to carry out the method. In an embodiment, the method of the invention is an isothermal method, so that all the reactions described herein can potentially be achieved at a single temperature. Preferably, said temperature is between 20-37°C, preferably 25-30°C.

As discussed in Example 3, the presence of ammonium sulphate was clearly detrimental to the amplification reaction with Qualiphi enzyme, as molecules in the order of 10⁷ could be clearly detected when this compound was removed from the reaction. Thus, in an embodiment, the method of the present invention is performed in the absence of ammonium salt, preferably ammonium sulphate, when Qualiphi polymerase, as set forth in SEQ ID NO: 3, is used in step c) of the method.

Since RCA and MDA, and therefore HRCA methods, are known in the art, the assay conditions and steps are known by a skilled person and can be used herein.

In a second aspect, the present invention provides a padlock suitable for the method of the present invention. Preferably, the padlock is one of the padlocks defined above, preferably a Gaplock, Flaplock, ssSplitlocks or dsSplitlocks, with or without skin, or any padlock derived from them. Thus, all embodiments related to the padlocks of the present invention defined in the first aspect or any of its embodiments, are also included in this second aspect. In an embodiment, the padlocks are provided as a composition, comprising other reagents or additives such as stabilizers, etc. The sequence of the padlocks may be modified to improve their stability. Modifications include chemical modification such as 2'-O-methyl (2'OMe), 2'-O-Methoxyethyl (2' MOE), an extra bridge connecting the 2' oxygen and 4' carbon (LNA), or phosphorothioate linkages, or a combination thereof.

Preferably the padlock probe comprises, consists, or consists essentially of SEQ ID NO: 8, 16, 17, 18, 19, 20, 23, 24, 25, 26, 27 or 32, or a sequence with at least 85%, 86%, 87%, 88%, 89%, 90%, 91%, 92%, 93%, 94%, 95%, 96%, 97%, 98%, 99% or 100% sequence identity to SEQ ID NO: 8, 16, 17, 18, 19, 20, 23, 24, 25, 26, 27 or 32. More preferably, the padlock of the present invention comprises, consists, or consists essentially of SEQ ID NO: 24-27 or 32 or a sequence with at least 85%, 86%, 87%, 88%, 89%, 90%, 91%, 92%, 93%, 94%, 95%, 96%, 97%, 98%, 99% or 100% sequence identity to SEQ ID NO: 24-27 or 32 wherein the backbone region (that is, sequence B) is indicated with "N", and preferably comprises between 20-160, preferably between 40-150 nucleotides that are not complementary to the target nucleic acid. Uses of the padlock probe in the method of the present invention or in any RCA or HRCA methos are also included herein.

In **a third aspect**, the present invention relates to the use of the method of the first aspect or the use of the padlocks of the second aspect for diagnostic and/or prognostic purposes. The applications of the method of the present invention and the padlocks thereof include detecting the presence and/or measuring the level of a target nucleic acid of interest in a suitable sample. In one particular the method is used to detect a target nucleic acid in a food or water sample, such as for instance to detect the presence of human pathogens in said samples. In other embodiments, the assay methods and padlocks described herein can be used in research settings for detecting presence or measuring the level of a target nucleic acid in a sample. The method and the padlocks may be applied in a diagnostic/prognostic setting to detect the presence or measure the level of a nucleic acid biomarker that is associated with a target disease. For example, the methods and padlocks may be used to detect/measure the presence of an infectious agent, which may be associated with a specific disease, e.g., covid or flu. The methods and padlocks can be used in detecting such a nucleic acid biomarker in subjects that are absent of any symptom of the disease for early-stage diagnosis. The assay methods and padlocks can also be used to detect nucleic acids of microorganisms for determining whether a subject has been infected by such microorganisms, for example, viruses (e.g., HBV, HCV, HPV, HIV, Influenza, coronavirus). Preferably, the method and padlocks of the present invention are used for detecting the presence of a virus, preferably a coronavirus, most preferably SARS-CoV-2 virus, in a sample.

In a **fourth aspect**, the present invention relates to a system or device to implement the method of the present invention. Several known systems or devices may be used for said purpose. They may include, but are not limited to, HRCA-based point-of-care (POC) tests for the rapid detection of the at least one target polynucleotide. Detection methods in POC tests are known in the art, and may include immunochromatographic assays, colorimetric assays, electrochemical assays, etc. The POC tests are usually designed as easy-to-use membrane-based test strips, often enclosed by a plastic test cassette. An example of a POC test is a test based on immunochromatographic assays, particularly Lateral flow tests (LFTs). In essence, these the LFTs run the test sample along the surface of a pad with reactive molecules that show a positive or negative result. The result may be displayed visually or upon stimulation with, for in-stance, a laser. Lateral flow-based biosensors usually comprise a backing card, a sample pad, nitrocellulose membrane, and absorbent pad.

In the context of the present invention "lateral flow system" or "lateral flow assay" is understood as a system comprising a lateral flow device implementing a methodology to detect, in a specific manner, the presence or absence of a target nucleic acid in a given matrix sample. This type of assay eliminates the need for specialized and costly equipment used in conventional laboratories. Lateral flow technology is used as point of care tool (fast and in situ), in a large range of applications, from the widely used home pregnancy test to more specialized tests for clinical, food safety, environmental and industrial applications.

In the context of the present invention "a backing card" is understood as the material where all the membranes (sample, conjugate, membrane and wicking pads) are mounted and connected to each other. At the same time, the backing card provides better stability and handling to the lateral flow device.

In the context of the present invention "a sample pad" is understood as the element of the device where the test sample is introduced or deposited and acts as a sponge that holds the sample fluid. Subsequently, the sample fluid is transported to the membrane by capillary action.

In the context of the present invention "a membrane" is understood as the material (e.g. nitrocellulose) that transports the test sample and provides support to the capture molecules (antibodies, aptamers etc.) and allows or enhances their binding capabilities. Two different capture molecules areas are defined on the membrane, test line area which provides the lateral flow assay result (positive or negative) and control line area which probes the correct performance of the lateral flow assay.

In the context of the present invention "a wicking pad" is understood as element in the device that retains all the assay material acting as a waste container.

Further, the method disclosed herein can also be implemented in other devices or systems, such as microfluidic devices, microarrays or electrochemical biosensors. The term "microfluidic chip or device" refers to a set of micro-channels etched or molded into a material (such as glass, silicon, a thermoplastic material or a polymer such as PDMS, for PolyDimethylSiloxane). The micro-channels forming the microfluidic chip are connected together in order to achieve the desired features (mix, pump, sort, control biochemical environment). This network of micro-channels trapped into the microfluidic chip is connected to the outside by inputs and outputs pierced through the chip, as an interface between the macro- and micro-world. It is through these holes that the liquids are injected and removed from the microfluidic chip (through tubing, syringe adapters or even simple holes in the chip) with external active systems (pressure controller, pushsyringe or peristatic pump) or passive ways (e.g. hydrostatic pressure).

A microfluidic chip or device comprises a support or substrate, wherein said support or substrate comprises at least one channel in the substrate, the channel comprising an inlet, an outlet, and a flow-path connecting the inlet and outlet, wherein the inlet and outlet together define a midplane; and a portion of the flowpath travels transversely across the midplane, wherein the portion of the flowpath that travels transversely across the midplane includes a recognition site or sensing area for detecting, for instance, a target nucleic acid.

In the context of the present invention "DNA microarray chip system" is understood as a system comprising a DNA microarray chip or device that in turn comprises a solid surface onto which DNA molecules, such as the padlocks, have been chemically bonded. Complementary base pairing between the target nucleic acid present in a test sample, preferably an isolated biological sample, and the DNA molecules attached on the chip produces light that is measured. In this particular system, the detection is produced when a hybridization complex comprising the at least one padlock and the target nucleic acid is formed and is further amplified. Areas on the chip producing light identify the target nucleotide that is present in the test sample. The microarray technology is a platform for the simultaneous analysis of different target sequences in complex fluids, in a high throughput manner. The microarray technology also allows multiplexing (detection of different target polynucleotides at the same time).

In the context of the present invention "electrochemical system" is understood as a system comprising an electrochemical device that provides an analytical signal in the form of an electrical current, which is directly proportional to the concentration of an analyte or, in this case, a target nucleic acid. Thus, the electrochemical system or device may act as biosensor. Detection of the at least one target polynucleotide is achieved due to production of a faradaic current produced due to the binding of the target nucleic acid to the padlock, which triggers the method of the present invention. The appearance of the RCA products or the RCA reaction itself (e.g., by means of a change in pH) produces an electroactive chemical compound. The redox process of the produced electroactive compound taking place on the electrochemical device pro-duces a current upon application of a voltage that is directly proportional to the concentration of the target nucleic acid.

In a **fifth aspect**, the present invention provides a kit comprising the necessary rea-gents to carry out the method of the present invention. Preferably, the kit also comprises instructions for such method. In an embodiment, the kit comprises:
a) at least one bacteriophage phi29 polymerase, preferably a polymerase defined in the first aspect of the present invention, or any of their embodiments,
b) at least one ligase, preferably SplintR if the target nucleic acid is a ribonucleotide,
c) optionally, at least one RNAase if the target nucleic acid is a ribonucleotide,
c) suitable MDA and/or RCA primers, preferably MDA and RCA primers defined in the present invention,
d) dNTPs,
e) suitable buffer for the amplification of a target nucleic acid, preferably in isothermal conditions.

Preferably, the kit comprises the padlock probes defined in the first aspect or any of the embodiments.

The following are preferred embodiments of the method of the present invention:
Method using a padlock where sequences A-B-C are provided in a single molecule, as represented in Fig. 1A: In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,
   wherein the first and the second portions of the target nucleic acid are located adjacent to each other and wherein the at least one padlock is added in the form of a single molecule comprising said sequences A-B-C, and wherein the nucleic acid structure of step a) comprising the padlock in a circularizable form is provided by hybridizing said at least one padlock in the form of a single molecule with the target nucleic acid,
b) ligating the 5' end of A sequence with the 3' end of sequence C using at least one ligase to provide a padlock comprising sequences A-B-C in a covalently closed circular molecule,
c) subjecting the ligated padlock probe of step b) to rolling circle amplification (RCA), wherein the RCA is carried out by a bacteriophage phi29 polymerase, and amplifying the RCA product in an MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in sequences A, B, and/or C of the padlock probe, preferably a sequence that is located in the junction region of sequences A and C and that is formed when sequences A and C are ligated in step b).

Method using a padlock where sequences A-B-C are provided in a single molecule and that further comprises a skin (SkinLock), as represented in Fig. 1B: In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,
   wherein the first and the second portions of the target nucleic acid are located adjacent to each other, wherein the at least one padlock is added in the form of a system of two different oligonucleotide molecules, wherein a first molecule comprises, in the 5' to 3' direction, nucleotide sequences A-B-C, and wherein the second molecule comprises sequence E, wherein sequence E is complementary and capable of hybridizing to sequence B or a region thereof, and wherein the nucleic acid structure of step a) with the padlock in a circularizable form is provided by hybridizing the first molecule to the target nucleic acid and the second molecule to the first molecule,
b) ligating the 5' end of A sequence with the 3' end of C sequence using at least one ligase to provide a padlock comprising sequences A-B-C in a covalently closed circular molecule,
c) subjecting the ligated padlock probe of step b) to rolling circle amplification (RCA), wherein the RCA is carried out by a bacteriophage phi29 polymerase, and amplifying the RCA product in a MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in sequences A, B, and/or C of the padlock probe, preferably a sequence that is located in the junction region of sequences A and C and that is formed when sequences A and C are ligated in step b).

Method using a Flaplock probes as represented in Fig. 1C: In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,

   wherein the first and the second portions of the target nucleic acid are located adjacent to each other,
   wherein the at least one padlock is added in the form of a single molecule comprising, in the 5' to 3' direction, nucleotide sequences D-A-B-C, or nucleotide sequences A-B-C-D,
   wherein sequence D is not complementary to the target nucleic acid and prevents sequences A and C to be ligated to each other,
   and wherein the nucleic acid structure of step a) with the padlock in a circularizable form is provided by first hybridizing the padlock in the form of a single oligonucleotide molecule with the target nucleic acid and subsequently exonucleolytically cleaving sequence D using a ssDNA-specific exonuclease or a FLAP endonuclease,
b) ligating the 5' end of A sequence with the 3' end of C sequence using at least one ligase to provide a padlock comprising sequences A-B-C in a covalently closed circular molecule,
c) subjecting the ligated padlock probe of step b) to rolling circle amplification (RCA), wherein the RCA is carried out by a bacteriophage phi29 polymerase, and amplifying the RCA product in a MDA reaction using at least two MDA primers with a maximum of 15 nucleotides in length, preferably a maximum of 10 nucleotides in length, and
d) detecting the amplification product from step c), thereby detecting the target nucleic acid, wherein the detection method is preferably a sequence-specific detection method, and wherein said sequence-specific detection method detects a sequence that is the complementary sequence of a sequence located in the junction region of sequence A and C and that is formed when sequences A and C are ligated in step b).

Method using a Flaplock with skin as represented in Fig. 1D: In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,

   wherein the first and the second portions of the target nucleic acid are located adjacent to each other,
   wherein the at least one padlock is added in the form of a system of two different oligonucleotide molecules, wherein:
      - a first molecule comprises, in the 5' to 3' direction, sequences D-A-B-C or sequences A-B-C-D, wherein sequence D is characterized by not being complementary to the target nucleic acid prevents sequences A and C to be ligated to each other; and
      - a second molecule comprises a sequence E, wherein sequence E is complementary to sequence B or a region thereof,
   and wherein the nucleic acid structure of step a) with the padlock in a circularizable form is provided by hybridizing the first molecule with the target nucleic acid and the second molecule to the first molecule, and subsequently exonucleolytically cleaving sequence D using a ssDNA-specific exonuclease or a FLAP endonuclease,
b) ligating the 5' end of A sequence with the 3' end of C sequence using at least one ligase, to provide a padlock comprising sequences A-B-C in a covalently closed circular molecule,
c) subjecting the ligated circular padlock probe of step b) to rolling circle amplification (RCA), wherein the RCA is carried out by a bacteriophage phi29 polymerase, and amplifying the RCA product in a MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in the junction region of sequence A and C and that is formed when sequences A and C are ligated in step b).

Method using a Gaplock where sequences A-B-C are provided in two different oligonucleotide molecules with a division in sequence C (as represented in Fig. 1E), or a division in sequence A (as represented in Fig. 1F), or with a division in both A and C: In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,

   wherein the first and the second portions of the target nucleic acid are located adjacent to each other,
   wherein the at least one padlock is added in the form of a system of two different oligonucleotide molecules, wherein:
      - a first molecule comprises, in the in the 5' to 3' direction, sequences A, followed by sequence B and a first portion of sequence C (C1) and a second molecule comprises a second portion of sequence C (C2), or
      - a first molecule comprises, in the 5' to 3' direction, a first portion of sequence A (A1) followed by sequence B and C, and a second molecule comprises a second portion of sequence A (A2),
         or
      - a first molecule comprises, in the 5' to 3' direction, a first portion of sequence A (A1) followed by sequence B and a first portion of C (C1), and a second molecule comprises, in the 5' to 3' direction, a second portion of sequence C (C2) followed by and a second portion of sequence A (A2),
   and wherein the nucleic acid structure of step a) with the padlock in a circularizable form is provided by hybridizing the first and second molecules with the target nucleic acid, and
b) providing a padlock with sequences A-B-C in a single covalently closed circular molecule by ligating the 5' end of the first molecule with the 3' end of the second molecule, and by ligating the 5' end of the second molecule with the 3' end of the first molecule, using at least one ligase,
c) subjecting the ligated circular padlock probe of step b) to rolling circle amplification (RCA), wherein the RCA is carried out by a bacteriophage phi29 polymerase, and amplifying the RCA product in a MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in one of the junction regions of the first and second molecules and that is formed by ligation of the first and second molecules in step b).

Method using a Gaplock where sequences A-B-C are provided in two different oligonucleotide molecules and wherein the padlock comprises sequence E, as represented in Fig. 1G: In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,

   wherein the first and the second portions of the target nucleic acid are located adjacent to each other,
   wherein the at least one padlock is added in the form of a system of three different oligonucleotide molecules, wherein:
      - a first molecule comprises, in the 5' to 3' direction, sequences A, followed by sequence B and a first portion of sequence C (C1); a second molecule comprises a second portion of C (C2); and a third molecule comprises a sequence E,
         or
      - a first molecule comprises, in the 5' to 3' direction, a first portion of sequence A (A1) followed by sequences B and C; a second molecule comprises a second portion of sequence A (A2); and a third molecule comprises a sequence E,
         or
      - a first molecule comprises, in the 5' to 3' direction, a first portion of sequence A (A1) followed by sequence B and a first portion of sequence C (C1); a second molecule comprises, in the 5' to 3' direction, a second portion of sequence C (C2) and a second portion of A (A2); and a third molecule comprises a sequence E,
   wherein sequence E is complementary to sequence B or a region thereof, and wherein the nucleic acid structure of step a) with the padlock in a circularizable form is provided by hybridizing the first and second molecules with the target nucleic acid, and
b) providing a padlock with sequences A-B-C in a single covalently closed circular molecule by ligating the 5' end of the first molecule with the 3' end of the second molecule, and by ligating the 5' end of the second molecule with the 3' end of the first molecule, using at least one ligase,
c) subjecting the ligated circular padlock probe of step b) to rolling circle amplification (RCA), wherein the RCA is carried out by a bacteriophage phi29 polymerase, and amplifying the RCA product in a MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in one of the junction regions of the first and second molecules and that is formed by ligation of the first and second molecules in step b).

Method using a ssSplitclock where sequences A-B-C are provided in two different oligonucleotide molecules with a division in sequence B, and further comprising sequence E, as represented in Fig. 1H. In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,

   wherein the first and the second portions of the target nucleic acid are located adjacent to each other,
   wherein the padlock probe is added as a system of three different oligonucleotide molecules, wherein:
      - a first molecule comprises, in the 5' to 3' direction, sequence A followed by a first portion of sequence B (B1),
      - a second molecule comprises, in the 5' to 3' direction, a second portion of B (B2) followed by sequence C, and
      - a third molecule comprises, in the 5' to 3' direction, sequence E, wherein sequence E is complementary and capable of hybridizing to sequence B or a region thereof, preferably to a region of both B1 and B2, and wherein the nucleic acid structure of step a) with the padlock in a circularizable form is provided by hybridizing the first and second molecules with the target nucleic acid and by hybridizing the third molecule to the first and second molecules, and
b) providing a padlock with sequences A-B-C in a single covalently closed circular molecule by ligating the 5' end of the first molecule with the 3' end of the second molecule, and by ligating the 5' end of the second molecule with the 3' end of the first molecule, using at least one ligase,
c) subjecting the ligated circular padlock probe of step b) to rolling circle amplification (RCA), wherein the RCA is carried out by a bacteriophage phi29 polymerase, and amplifying the RCA product in an MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in one of the junction regions of the first and second molecules and that is formed by ligation of the first and second molecules in step b), wherein preferably the sequence is located in the junction region of sequences A and C.

Method using a dsSplitclock where sequences A-B-C are provided in two different oligonucleotide molecules with a division in sequence B, and further comprising sequence E provided in two different oligonucleotide molecules, as represented in Fig. 1I and 1J. In an embodiment, the method comprises the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
   - A is a nucleotide sequence located in the 5' end of the padlock that is complementary to a first portion of the target nucleic acid and is hybridized thereto,
   - B is a nucleotide sequence that is not complementary to the target nucleic acid,
   - C is a nucleotide sequence that is located in the 3' end of the padlock and that is complementary to a second portion of the target nucleic acid and is hybridized thereto,

   wherein the first and the second portions of the target nucleic acid are located adjacent to each other,
   wherein the padlock probe is added as a system of four different oligonucleotide molecules, wherein:
      - a first molecule comprises, in the 5' to 3' direction, sequence A (A1) and a first portion of sequence B (B1),
      - a second molecule comprises, in the 5' to 3' direction, a second portion of B (B2) followed by sequence C,
      - a third molecule comprises a first portion of sequence E (E1), wherein sequence E1 is complementary and capable of hybridizing to a region of sequence B containing at least a part of the first portion of sequence B (B1) and
      - a fourth molecule comprises a second portion of sequence E (E2), wherein sequence E is complementary and capable of hybridizing to a region of sequence B containing at least a part of the second portion of sequence B (B2), and
   wherein the nucleic acid structure of step a) with the padlock in a circularizable form is provided by hybridizing the first and second molecules with the target nucleic acid and the third and fourth molecules with the first and second molecules, respectively, and
   wherein, preferably, said nucleic acid structure comprises reverse-complementary overhangs in the junction between sequences B1/E1 and B2/E2,
b) providing a padlock with sequences A-B-C in a single covalently closed circular molecule by ligating the 5' end of the first molecule with the 3' end of the second molecule, and by ligating the 5' end of the second molecule with the 3' end of the first molecule, using at least one ligase,
c) subjecting the ligated circular padlock probe of step b) to rolling circle amplification (RCA), wherein the RCA is carried out by a bacteriophage phi29 polymerase, and amplifying the RCA product in an MDA reaction using at least two MDA primers with a maximum of 15 nucleotides in length, preferably a maximum of 10 nucleotides in length, and,
d) detecting the amplification product from step c), thereby detecting the target nucleic acid, wherein the detection method is preferably a sequence-specific detection method, and wherein said sequence-specific detection method detects a sequence that is the complementary sequence of a sequence located in one of the junction regions of the first and second molecules and that is formed by ligation of the first and second molecules in step b), wherein preferably the sequence is located in the junction region of sequences A and C..

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

### SEQUENCE LISTING

**SEQ ID NO: 1 SplintR: DNA Ligase from Paramecium bursaria Chlorella Virus-1** 
MAITKPLLAATLENIEDVQFPCLATPKIDGIRSVKQTQMLSRTFKPIRNSVMNRLLTELL
PEGSDGEISIEGATFQDTTSAVMTGHKMYNAKFSYYWFDYVTDDPLKKYIDRVEDMKNYI
TVHPHILEHAQVKIIPLIPVEINNITELLQYERDVLSKGFEGVMIRKPDGKYKFGRSTLK
EGILLKMKQFKDAEATIISMTALFKNTNTKTKDNFGYSKRSTHKSGKVEEDVMGSIEVDY
DGVVFSIGTGFDADQRRDFWQNKESYIGKMVKFKYFEMGSKDCPRFPVFIGIRHEEDR
**SEQ ID NO: 2 Phi29 wildt-ype polymerase** 
MPRKMYSCDFETTTKVEDCRVWAYGYMNIEDHSEYKIGNSLDEFMAWVLKVQADLYFHNL
KFDGAFIINWLERNGFKWSADGLPNTYNTIISRMGQWYMIDICLGYKGKRKIHTVIYDSL
KKLPFPVKKIAKDFKLTVLKGDIDYHKERPVGYKITPEEYAYIKNDIQIIAEALLIQFKQ
GLDRMTAGSDSLKGFKDIITTKKFKKVFPTLSLGLDKEVRYAYRGGFTWLNDRFKEKEIG
EGMVFDVNSLYPAQMYSRLLPYGEPIVFEGKYVWDEDYPLHIQHIRCEFELKEGYIPTIQ
IKRSRFYKGNEYLKSSGGEIADLWLSNVDLELMKEHYDLYNVEYISGLKFKATTGLFKDF
IDKWTYIKTTSEGAIKQLAKLMLNSLYGKFASNPDVTGKVPYLKENGALGFRLGEEETKD
PVYTPMGVFITAWARYTTITAAQACYDRIIYCDTDSIHLTGTEIPDVIKDIVDPKKLGYW
AHESTFKRAKYLRQKTYIQDIYMKEVDGKLVEGSPDDYTDIKFSVKCAGMTDKIKKEVTF
ENFKVGFSRKMKPKPVQVPGGVVLVDDTFTIK
**SEQ ID NO: 3 Qualiphi polymerase** 
MPRKMYSCDFETTTKVEDCRVWAYGYMNIEDHSEYKIGNSLDEFMAWVLKVQADLYFHNL
KFDGAFIINWLERNGFKWSADGLPNTYNTIISRMGQWYMIDICLGYKGKRKIHTVIYDSL
KKLPFPVKKIAKDFKLTVLKGDIDYHKERPVGYKITPEEYAYIKNDIQIIAEALLIQFKQ
GLDRMTAGSDSLKGFKDIITTKKFKKVFPTLSLGLDKEVRYAYRGGFTWLNDRFKEKEIG
EGMVFDVNSLYPAQMYSRLLPYGEPIVFEGKYVWDEDYPLHIQHIRCEFELKEGYIPTIQ
IKRSRFYKGNEYLKSSGGEIADLWLSNVDLELMKEHYDLYNVEYISGLKFKATTGLFKDF
IDKWTYIKTTSEGAIKQLAKLMLNSLYGKFASNPDVTGKVPYLKENGALGFRLGEEETKD
PVYTPMGVFITAWARYTTITAAQACYDRIIYCDTDSIHLTGTEIPDVIKDIVDPKKLGYW
AHESTFKRAKYLRQKTYIQDIYMKEVDGKLVEGSPDDYTDIKFSVKCAGMTDKIKKEVTF
ENFKVGFSRKMKPKPVQVPGGVVLVDDTFTIKGTGSGAWKEWLERKVGEGRARRLIEYFG
SAGEVGKLVENAEVSKLLEVPGIGDEAVARLVPGYKTLRDAGLTPAEAERVLKRYGSVSK
VQEGATPDELRELGLGDAKIARILG
**SEQ ID NO: 24 padlock (similar to SEQ ID NO: 16)**
   [Phos]-TGCGGTGGACGATGG**N***CCGCTAGAAGCATT*
   wherein the 5' end of the padlock is phosphorylated (indicated with [Phos]) to facilitate the ligation of the 5' and 3' ends of the hybridized padlock, and wherein the sequence underlined represents sequence "A", sequence "N" in bold represents sequence "B", and sequence in *italics* represent sequence "C".
**SEQ ID NO: 25 padlock (similar to SEQ ID NO: 8)**
   [Phos]AAAGTCCTAGGTTGA**N***TTATATTTTAATAG*
   wherein the 5' end of the padlock is phosphorylated (indicated with [Phos]) to facilitate the ligation of the 5' and 3' ends of the hybridized padlock, and wherein the sequence underlined represents sequence "A", sequence "N" in bold represents sequence "B", and sequence in *italics* represent sequence "C".
**SEQ ID NO: 26 padlock (similar to SEQ ID NO: 23)**
   [Phos]AAAGTCCTAGGTTGA**N***TTATATTTTAATA*GAAAAAAAAAAAAAAAA
   wherein the 5' end of the padlock is phosphorylated (indicated with [Phos]) to facilitate the ligation of the 5' and 3' ends of the hybridized padlock, and wherein the sequence underlined represents sequence "A", sequence "N" in bold represents sequence "B", sequence in *italics* represent sequence "C", and highlighted font represents sequence "D".
**SEQ ID NO: 27 padlock (similar to SEQ ID NO: 17** + **18)**
   [Phos]AGATAACCCACATAA**N***TTATATTTTAATAGAAAA*GTCCTAGGTTGA
   wherein the 5' end of the padlock is phosphorylated (indicated with [Phos]) to facilitate the ligation of the 5' and 3' ends of the hybridized padlock, and wherein the sequence underlined represents sequence "A", sequence "N" in bold represents sequence "B", sequence in *italics* represent sequence "C1" and highlighted font indicates sequence "C2".
**SEQ ID NO: 32 padlock (similar to SEQ ID NOs: 19** + **20 and 28+29)**
   [Phos]AAAGTCCTAGGTTGA**N***TTATATTTTAATAG*
   wherein the 5' end of the padlock is phosphorylated (indicated with [Phos]) to facilitate the ligation of the 5' and 3' ends of the hybridized padlock, and wherein the sequence underlined represents sequence "A", sequence "N" in bold represents sequence "B", and sequence in *italics* represent sequence "C".

The invention is described below by the following examples, which must be considered as merely illustrative and in no case limiting of the scope of the present invention.

### EXAMPLES

### Example 1. Proof of principle

As proof of principle for phi29pol-mediated hyperbranched rolling-circle amplification (HRCA) detection of RNA sequences at low temperature (30°C) (Figure 2a), we designed a padlock probe against human *ACTB* mRNA. Then, HRCA reactions were carried out with Qualiphi and SplintR-ligase enzymes, as indicated below. Amplification was only observed when both SplintR ligase and the corresponding *ACTB* synthetic RNA was included in the reaction (Figure 2b). This proves that Qualiphi can be used for HRCA -mediated RNA detection at low temperatures.

### Oligonucleotides:

| Name | Sequence 5'-3' |
|---|---|
| Padlock-*ACTB* (B region underlined) | SEQ ID NO: 16: |
| | [Phos]TGCGGTGGACGATGGAAAAATGTAAAACGACGGCCGA ATTCATAGCTGTTTCCTGAAAAACCGCTAGAAGCATT |
| *GFP* RNA target | SEQ ID NO: 33: |
| | rCrArArCrArUrCrCrUrGrGrGrCrArArUrArArGrArUrGrGrArGrUrArC rArArCrUrArCrArArCrGrCrCrCrArCrArArUrGrUrGrU |
| *ACTB* RNA target | SEQ ID NO: 34: |
| | rUrCrCrGrGrCrCrCrCrUrCrCrArUrCrGrUrCrCrArCrCrGrCrArA rArUrGrCrUrUrCrUrArGrGrCrGrGrArCrUrArUrGrArCrUrU |
| MDA1 15nt primer | SEQ ID NO: 4: TGTAAAACGACG*G*C*C |
| MDA2 15nt primer | SEQ ID NO: 5: CAGGAAACAGCT*A*T*G |

| | |
|---|---|
| * indicates phosphorothioate linkages and r means the molecule is RNA | |

### Reaction:

- Annealing: 1h 25°C

| **Reagent** | **Final concentration** |
|---|---|
| SplintR Buffer 1X | 50 mM Tris-HCl pH 7.5, 10 mM MgCl₂, 1mM ATP, 10 mM DTT |
| Padlock | 15nM |
| RNA target | 15nM |

- Ligation: 1h 25°C

| **Reagent** | **Final concentration** |
|---|---|
| SplintR Buffer 1X | 50 mM Tris-HCl pH 7.5, 10 mM MgCl2, 1mM ATP, 10 mM DTT |
| Padlock | 15nM |
| RNA target | 15nM |
| SplintR Ligase | 1.25U/ul |

- Amplification: 2h 30°C

| **Reagent** | **Final concentration** |
|---|---|
| Qualiphi | 0.5U/ul |
| Qualiphi Buffer 1X | 50 mM Tris-HCl pH 7.5, 10 mM MgCl2, 1 mM DTT, 50 mM KCI, 10mM (NH₄)₂SO4 |
| dNTPs | 500nM each |
| MDA1/2 | |
| 15nt primer | 800nM each |
| Tween 20 | 0.025% |

### Example 2. Target titration

We then decided to test the level of sensitivity of this method, for which we performed the HRCA reactions, as indicated in Example 1, but with decreasing concentrations of *ACTB* synthetic RNA. As can be seen in Figure 2c, the sensitivity of the reactions was limited to a number of target molecules above 10⁷.

### Example 3. Ammonium sulphate

In order to check whether the lack of sensitivity was due to a problem in the ligation or in the amplification steps of the reaction, we annealed and ligated target and padlock probe in a 1:1 ratio, and then this ligation was serially diluted to test amplification of the generated circles. Furthermore, we decided to test whether ammonium sulphate, which is included in the commercial reaction buffer of Qualiphi, could be somewhat limiting amplification in these conditions. First, we observed that there was a clear problem of amplification, as the sensitivity for detecting diluted circles (Figure 3) was similar to that of target titration experiments (Figure 2c). Furthermore, the presence of ammonium sulphate was clearly detrimental to the amplification reaction with Qualiphi enzyme, as molecules in the order of 10⁷ could be clearly detected when this compound was removed from the reaction. The experiment was carried out as described in Example 1, in the presence and absence of ammonium sulphate in the Qualiphi buffer, as indicated.

### Example 4. Primers

Since ammonium sulphate is known to affect primer annealing and is used to increase specificity and sensitivity of PCR reactions, we decided to test the effect of the length of the primers in the reaction, and used shorter (10-nt) primers with a lower Tm (Figure 4). We observed a dramatic change, with strong amplification in all the dilutions of circles tested, but at the cost of an important level of unspecific amplification in all samples containing linear padlocks, both in the absence of target and/or ligase. We conclude that the problem for Qualiphi in performing HRCA is not related to an intrinsic characteristic of the enzyme, but to the behaviour of nucleic acids at the low temperatures used in the assay (30°C), and it should be therefore achievable to obtain conditions with the required sensitivity. The experiment was carried out as described in Example 3, without ammonium sulphate in the Qualiphi buffer, and with the following MDA1/2 primers.

### Oligonucleotides:

| Name | Sequence 5'-3' |
|---|---|
| MDA1 10nt primer | SEQ ID NO: 6: TGTAAAA*C*G*A |
| MDA2 10nt primer | SEQ ID NO: 7: AACAGCT*A*T*G |

| | |
|---|---|
| * indicates phosphorothioate linkages | |

### Example 5. Wild-type phi29pol

Qualiphi is an engineered version of phi29pol fused with DNA binding domains that confer it with enhanced substrate affinity and processivity. We decided to test whether this optimized method could also operate with wild-type enzyme. As can be seen in Figure 5, the use of wild-type phi29pol significantly delayed amplification, reducing sensitivity with 2h incubation times, but this could be circumvented by extending incubation time to 6h. The method, therefore, works with wild-type phi29pol, although enhanced versions like Qualiphi or others may be required to reduce the time of diagnostic tests. The experiment was carried out essentially as described in Example 4, but with the amplification conditions described below. Please note that ammonium sulphate was beneficial for performance of the wild-type enzyme.

### Reaction:

- Amplification: 2h/6h 30°C

| Name | Final concentration |
|---|---|
| Phi29 polymerase (NEB) | 0.5U/ul |
| Phi29 polymerase Buffer 1X | 50mM Tris-HCl ph 7.5, 10mM MgCl2, 10mM (NH4)2SO4, 4mM DTT |
| dNTPs | 500nM each |
| MDA1/2 15nt primer | 800nM each |
| Tween 20 | 0.025% |

With all these approaches, we optimized amplification conditions, but at the cost of losing specificity. The examples below are aimed at reducing the unspecific background signal, while maintaining a high level of sensitivity.

### Example 6. CRISPR-Cas12a detection

Considering that target-specific (i.e., hybridized padlocks that are circularized and amplified) and unspecific (i.e., non-hybridized padlocks) amplification events are unlikely to result in the same sequence, especially in the breakpoint region of the padlock, we reasoned that one possibility to circumvent the unspecificity of the assay, while maintaining high yield of amplification, would be to link it to a sequence-specific detection step, such as CRISPR-Cas12a-mediated detection (Figure 6a). We therefore designed a new padlock probe annealing to a region of the *S gene* of the SARS-CoV-2 viral genome, and containing a Cas12a PAM motif to allow detection of the amplified dsDNA. We also designed the corresponding gRNA specifically targeting this sequence in the (+) viral strand, so that only the amplified material, and not the padlock itself can be detected. We then performed HRCA-Cas12a detection of decreasing concentrations of *in vitro* transcribed *S gene* RNA, in the conditions described below (Figure 6b). In these reactions, nucleolytic activity of Cas12a ribonucleoparticles specifically activated by the amplified target is detected by fluorescence emission of a reporter substrate. Interestingly, specific and unspecific amplification material, undistinguishable in a gel, could be discriminated when coupled to CRISPR-Cas12a sequence-specific detection, reaching detection of a number of molecules in the order of 10⁵.

| **Name** | **Sequence 5'-3'** |
|---|---|
| Padlock-*S gene* (B region is underlined) | SEQ ID NO: 8: |
| | [Phos]AAAGTCCTAGGTTGAAAAAATGTAAAACGACG |
| | GCCGAATTCATAGCTGTTTCCTGAAAAATTATATTTT AATAG |
| IVT DNA template SARS-CoV-*Gene S* (T7 promoter is underlined) | SEQ ID NO: 9: |
| | TAATACGACTCACTATAGGTAACATCACTAGGTTTCA AACTTTACTTGCTTTACATAGAAGTTATTTGACTCCT GGTGATTCTTCTTCAGGTTGGACAGCTGGTGCTGCA GCTTATTATGTGGGTTATCTTCAACCTAGGACTTTTC TATTAAAATATAATGAAAATGGAACCATTACAGATGC TGTAGACTGTGC |
| MDA1 10nt primer | SEQ ID NO: 10: TGT AAA A*C*G* A |
| MDA2 10nt primer | SEQ ID NO: 11: AAC AGC T*A*T* G |
| Guide LbaCas12a SARS-CoV-2 *S gene* | SEQ ID NO: 12: |
| | rUrArArUrUrUrCrUrArCrUrArArGrUrGrUrArGrArUrArUrA rGrArArArArGrUrCrCrUrArGrGrUrUrGrA |
| Cas12a reporter | SEQ ID NO: 13: /56-FAM/T*TAT*T*/3IABkFQ/ |

| | |
|---|---|
| * indicates phosphorothioate linkages and r means the molecule is RNA | |

### Reaction:

- Annealing: 1h 25°C

| Name | Final concentration |
|---|---|
| SplintR Buffer 1X | 50 mM Tris-HCl pH 7.5, 10 mM MgCl₂, 1mM ATP, 10 mM DTT |
| Padlock | 15nM |
| RNA target | 15nM |

- Ligation: 1h 25°C

| Name | Final concentration |
|---|---|
| SplintR Buffer 1X | 50 mM Tris-HCl pH 7.5, 10 mM MgCl₂, 1mM ATP, 10 mM DTT |
| Padlock | 15nM |
| RNA target | 15nM |
| SplintR Ligase | 1.25U/ul |

- Amplification: 2h 30°C

| Name | Final concentration |
|---|---|
| Qualiphi | 0.5U/ul |
| Qualiphi Buffer 1X | 50 mM Tris-HCl pH 7.5, 10 mM MgCl2, 1 mM DTT, 50 mM KCl |
| dNTPs | 500nM each |
| MDA1/2 10nt primer | 800nM each |
| Tween 20 | 0.025% |

- Cas12a detection: 30°C for the indicated time

| Name | Final concentration |
|---|---|
| Buffer 2.1 1X (NEB) | 50 mM NaCl, 10mM Tris-HCl, 10 mM MgCl2, 100 µg/ml BSA. pH 7.9 |
| Cas12a RNP | 50nM |
| Reporter | 200nM |

With this in mind, we focused on implementing different improvements in the padlock that could, on their own or combined, reduce background and/or increase target-specific amplification. Both wild-type and Qualiphi versions of the enzyme were indistinctly used to assess the benefit of the different approaches tested.

### Example 7. Padlock with skin (Skinlock)

Since the problems in amplification and background likely result from unspecific annealing events, we decided to cover the backbone of the padlock with a complementary DNA strand (named herein skin or sequence E). This should both, protect from unspecific priming events (reducing background) and limit hybridization of remaining padlocks with the initial RCA products, which would outcompete with MDA primers, limiting the HCRA reaction. This skin can either contain a 3'-OH end to serve as an initial point of DNA synthesis, or have a blocked 3' end (e.g. conjugated biotin), so synthesis would need to be triggered from the annealed RNA. When we tested these possibilities with phi29pol (Figure 7), we found that the blocked skin clearly reduced background signal. The amplification was further improved by including a 3'-OH skin, while the benefit in terms of specificity was maintained. We therefore conclude that a skin, reverse complementary to the padlock backbone, can limit background amplification. Depending on the sensitivity needs, this skin can harbor, or not, an extensible 3'-OH end. The experiment was performed as described in Example 6, but with the amplification conditions described in Example 5. In addition, the padlock was pre-annealed with the skins described below.

| | |
|---|---|
| Skin 3'OH | SEQ ID NO: 14: CAGGAAACAGCTATGAATTCGGCCGTCGTTTTACA |
| Skin-Biotin | SEQ ID NO: 15: CAGGAAACAGCTATGAATTCGGCCGTCGTT*T*T*A* C*A[Biot] |

| | |
|---|---|
| * indicates phosphorothioate linkages | |

### Example 8. Padlock with flap (Flaplock) and exonuclease treatment

In order to improve the specific detection of the amplified product derived from the hybridization of the padlocks in the presence of target nucleic acid, we thought on approaches that could force the hybridized and amplified padlock to be as different as possible from the padlocks that are ligated in the absence of target. One of the possibilities is to include some sequence in the breakpoint junction of the padlock, so that it is only specifically removed when it is annealed to the target. We decided to include a poly-A extension on the 3' end of the S-gene padlock, so that it forms a flap when annealed to the target RNA (Figure 8a). Some approaches have used 5'-flap padlocks and structure-specific endonucleases to eliminate this flap, but we decided to use a 3'-flap and treatment with *E. coli* exonuclease I (Exol), which is a 3' ssDNA-specific exonuclease, since it allows us to fulfill two purposes: removing the flap of annealed padlocks and removing the excess of non-annealed padlocks. The latter may be relevant as an excess of padlock may interfere with the reaction by annealing with the initial RCA product and result in some level of background amplification. It is worth noting that the remaining padlock can also serve as MDA primer if not removed from the reaction. As seen in Figure 8b, this flap-Exol approach using Qualiphi enzyme clearly reduced background signal, allowing detection of down to 10³ target molecules, which was easily discernible from samples not containing target RNA. The experiment was performed as described in Example 6 but with the padlock-flap described below, and including an Exol degradation step after annealing, and before ligation, as indicated below.

### Oligonucleotides:

| **Name** | **Sequence 5'-3'** |
|---|---|
| Padlock-*S gene*-FLAP | SEQ ID NO: 23: |
| | [Phos]AAAGTCCTAGGTTGAAAAAATGTAAAACGACGGCCGAATTC ATAGCTGTTTCCTGAAAAATTATATTTTAATAGAAAAAAAAAAAAAA AA |

### Reaction:

- Exol degradation: 1h 25°C

| Name | Final concentration |
|---|---|
| SplintR Buffer 1X | 50 mM Tris-HCl pH 7.5, 10 mM MgCl₂, 1mM ATP, 10 mM DTT |
| Name | Final concentration |
| Padlock | 15nM |
| RNA target | 15nM |
| Exol | 1U/ul |

### Example 9. Padlock with flap (Flaplock) without exonuclease

Phi29pol, and its derivatives such as Qualiphi, also harbor strong 3' exonuclease activity. We therefore tested whether this approach with padlock flap could also work in the absence Exol treatment. Indeed, we observed that the Exol treatment step was not required (Figure 9), reaching similar levels of detection and background signal, regardless of including or not an Exol treatment step in reactions with flap padlocks. The experiment was performed as described in Example 6, with the padlock described in Example 8.

### Example 10. Padlock with gap (Gaplock)

Another possibility to differentiate hybridized and amplified padlocks from those padlocks that did not hybridize to the target sequence is to remove a number of nucleotides from the breakpoint junction of the padlock (sequences A or C), creating a gap that is then filled-in by additionally providing a short oligonucleotide to cover it, so the padlock is circularized by a double ligation event, reconstituting the correct sequence only when the molecules are perfectly aligned with the target RNA (Figure 10a). As can be seen in Figure 10b, the use of this Gaplock approach completely removed background signal. The experiment was performed as described in Example 6 but with the amplification conditions of Example 5 (wild-type phi29pol), and with the following padlock (A-B-C1 sequences) and gap fragment (C2 sequence).

### Oligonucleotides:

| Name | Sequence 5'-3' |
|---|---|
| Padlock-*S gene*-GAP (B region underlined) | SEQ ID NO: 17: |
| | [Phos]AGATAACCCACATAAAAAAATGTAAAACGACGG- CCGAATTCATAGCTGTTTCCTGAAAAATTATATTTTAATAGAAAA |
| GAP fragment | SEQ ID NO:18: [Phos]GTCCTAGGTTGA |

### Example 11. Padlock with separated arms (Splitlock)

Finally, we thought that separating sequence B of the padlock in two different molecules or arms (B1 and B2, i.e., splitting the padlock into two fragments), should also strongly reduce the background signal, since the annealing regions for the MDA primers would be located in different molecules, unless the padlock is fully circularized (Figure 1H-J).

In one embodiment (ssSplitlock; Figure 1H), once the two arms individually find its target region, the skin (sequence E) is added to act as a splint between the two arms. Breakpoints in the target RNA and skin are ligated by SplintR and T4 ligases, respectively. In addition to decreasing the background signal, this approach is also likely to favour productive circularization events, as opposed to dimerization of padlocks, something that is likely to be an important limitation, especially in conditions of low target availability, as is the case of most diagnostic approaches.

Another embodiment (dsSplitlock; Figure 1I) is to provide padlock arms that are already double-stranded in the backbone breakpoint region (pre-hybridized with a split skin), which can be designed to result in reverse-complementary overhangs to favour ligation. In this case, we designed padlock arms with 5-bp 5' overhangs, but other configurations may be used. Figure 11a and 11b show that both ssDNA and dsDNA Splitlock approaches, respectively, were efficient in reducing background signal.

Finally, this dsSplitlock approach can be designed so that E1 and E2 sequences cannot be ligated, so that the 3' OH end of E2 can be used for priming the RCA reaction only in the ligated padlocks (dsSplitlock-nick; Figure 1J). In this case it is preferable to use a design leaving 3' overhangs, to prevent them from being filled in by the polymerase, and to leave at least one nucleotide gap between E1 and E2 when they are annealed in order to facilitate the action of the polymerase on the ligated template. In this case we designed a 5-bp 3' overhang with a one-nucleotide gap and an E1 fragment lacking 5' phosphate. An important advantage of this arrangement is that padlocks are not modified by the DNA polymerase until they are appropriately assembled and circularized, which should allow 1-step reaction conditions. Furthermore, the circularization of padlocks using the RCA product is favoured, and they are self-sufficient for triggering a subsequent RCA reaction, possibly resulting in exponential amplification, even in the absence of MDA. Figure 11c shows how this approach further enhances sensitivity without an increase in background signal.

The ssSplitlock experiment was performed as described in Example 6, but including the following additional components in the ligation step.

### Oligonucleotides:

| Name | Sequence 5'-3' |
|---|---|
| Padlock-*S gene-5'* (B1 region underlined) | SEQ ID NO: 19: |
| | [Phos]AAAGTCCTAGGTTGAAAAAATGTAAAACGACGGCCGA |
| Padlock-S *gene-3'* (B2 region underlined) | SEQ ID NO: 20: |
| | [Phos]ATTCATAGCTGTTTCCTGAAAAATTATATTTTAATAGA |
| Skin Biotin | SEQ ID NO: 15: |
| | CAGGAAACAGCTATGAATTCGGCCGTCGTT*T*T*A* C*A[Biot] |

### Reaction:

- Ligation: 1h 25°C

| Name | Final concentration |
|---|---|
| SplintR Buffer 1X | 50 mM Tris-HCl pH 7.5, 10 mM MgCl₂, 1mM ATP, 10 mM DTT |
| Padlock 3' | 15nM |
| Padlock 5' | 15nM |
| Skin Biotin | 15nM |
| RNA target | 15nM |
| SplintR Ligase | 1.25U/ul |
| T4 Ligase | 20U/ul |

The dsSplitlock experiment was performed as described above, but each split arm of the padlock was pre-hybridized with the corresponding reverse complementary (skin) oligonucleotide before the annealing step of the reaction.

### Oligonucleotides:

| Name | Sequence 5'-3' |
|---|---|
| Padlock-*S gene*-5' arm (B1 sequence underlined) | SEQ ID NO: 19: |
| | [Phos]AAAGTCCTAGGTTGAAAAAATGTAAAACGACGGCCGA |
| Padlock-*S gene*-3' arm (B2 sequence underlined) | SEQ ID NO: 20: |
| | [Phos]ATTCATAGCTGTTTCCTGAAAAATTATATTTTAATAGA |
| Skin 5' arm | SEQ ID NO: 21: |
| | [Phos]GAATTCGGCCGTCGTT*T*T*A*C*A/3Bio/ |
| Skin 3' arm | SEQ ID NO: 22: [Phos]CAGGAAACAGCTAT |

The dsSplitlock-nick experiment was performed with the following oligonucleotides. In this case, the RCA reaction was performed with Equiphi29 DNA polymerase for 3h.

### Oligonucleotides:

| Name | Sequence 5'-3' |
|---|---|
| Padlock-*S gene-5'* arm (B1 sequence underlined ) | SEQ ID NO: 28: |
| | [Phos]AAAGTCCTAGGTTGAAAAAATGTAAAACGACGGCCGAA*T*T* C |
| Padlock-*S gene-3'* arm (B2 sequence underlined ) | SEQ ID NO: 29: |
| | [Phos]ATAGCTGTTTCCTGAAAAATTATATTTTAATAGA |
| Skin 5' arm | SEQ ID NO: 30: |
| | [Phos]CAGGAAACAGCTATGAA*T*T*C |
| Skin 3' arm | SEQ ID NO: 31: |
| | GCCGTCGTT*T*T*A*C*A[Bio] |

### Example 12. Combination of approaches

The implementation of each of these improvements, and their combination, may depend on the particular needs of the diagnostic test to be developed. As an example, we combined flap padlocks and Exol treatment with a 3'-OH skin, obtaining the results shown in Figure 12. In these conditions as little as 10³ molecules were detected with minimal background signal. The experiment was performed as described in Example 7 and including the Exol treatment described in Example 8.

Although we used real-time measurement of fluorescence in all the examples above, one advantage of CRISPR-Cas12a detection is that it can be easily adapted to qualitative diagnosis in lateral flow devices, as previously described. The high level of signal obtained in our approaches is perfectly compatible with this type of detection.

## Claims

1. A method of detecting the presence of a target nucleic acid in a sample by hyperbranched rolling circle amplification (HRCA), the method comprising the steps of:
a) adding at least one padlock probe to the sample and providing a nucleic acid structure formed by the hybridization of the at least one padlock with the target nucleic acid, wherein in said nucleic acid structure the at least one padlock is in a circularizable form and comprises, in the 5' to 3' direction, nucleotide sequences A-B-C, wherein:
- A is a nucleotide sequence located in the 5' end of the padlock that is complementary to a first portion of the target nucleic acid and is hybridized thereto,
- B is a nucleotide sequence that is not complementary to the target nucleic acid, and
- C is a nucleotide sequence that is located in the 3' end of the padlock and that is complementary to a second portion of the target nucleic acid and is hybridized thereto, and
wherein the first and the second portions of the target nucleic acid are located adjacent to each other, and
b) subjecting the circularizable padlock to a ligation reaction using at least one ligase to provide a padlock comprising sequences A-B-C in a single covalently closed circular molecule,
c) subjecting the ligated circular padlock of step b) to a rolling circle amplification (RCA) reaction and amplifying the RCA product in an MDA reaction using MDA primers with a maximum of 10 nucleotides in length and,
d) detecting the amplification product from step c) with a sequence-specific detection method, thereby detecting the target nucleic acid.

2. The method according to claim 1, wherein the at least one padlock is added in step a) in the form of a system of at least two different oligonucleotide molecules, wherein a first molecule comprises, in the 5' to 3' direction, nucleotide sequences A-B-C, and a second molecule comprises nucleotide sequence E, wherein sequence E is complementary to sequence B or a portion thereof,
wherein the nucleic acid structure of step a) with the padlock in a circularizable form is provided by hybridizing the first molecule to the target nucleic acid and the second molecule to the first molecule,
wherein the single covalently closed circular molecule in step b) is provided by ligating the 5' end of A sequence with the 3' end of sequence C using at least one ligase and wherein the sequence-specific detection method of step d) detects a sequence that is the complementary sequence of a sequence located in the junction region of sequences A and C and that is formed when sequences A and Care ligated in step b).

3. The method according to claim 1, wherein the at least one padlock is added in step a) in the form of a single oligonucleotide molecule comprising, in the 5' to 3' direction, sequences D-A-B-C or sequences A-B-C-D, wherein sequence D is located at the 3' end of sequence C or at the 5' end of sequence A and is **characterized by** not being complementary to the target nucleic acid,
wherein the nucleic acid structure of step a) with the padlock in a circularizable form is provided by first hybridizing the padlock in the form of a single oligonucleotide molecule with the target nucleic acid and subsequently exonucleolytically cleaving sequence D using a ssDNA-specific exonuclease or a FLAP endonuclease,
wherein the single covalently closed circular molecule in step b) is provided by ligating the 5' end of A sequence with the 3' end of sequence C using at least one ligase and wherein the sequence-specific detection method detects a sequence that is the complementary sequence of a sequence located in the junction region of sequences A and C and that is formed when sequences A and C are ligated in step b).

4. The method according to claim 1, wherein the at least one padlock is added in step a) in the form of a system of two different oligonucleotide molecules, wherein:
- a first molecule comprises, in the 5' to 3' direction, sequences D-A-B-C or sequences A-B-C-D, wherein sequence D is located at the 3' end of sequence C or at the 5' end of sequence A and is **characterized by** not being complementary to the target nucleic acid,
- a second molecule comprises a sequence E, wherein sequence E is complementary to sequence B or a region thereof, and
wherein the nucleic acid structure of step a) with the padlock in a circularizable form is provided by first hybridizing the first molecule with the target nucleic acid and with the second molecule, and subsequently exonucleolytically cleaving sequence D by action of a ssDNA-specific exonuclease or a FLAP endonuclease;
wherein the single covalently closed circular molecule in step b) is provided by ligating the 5' end of A sequence with the 3' end of sequence C using at least one ligase and wherein the sequence-specific detection method detects a sequence that is the complementary sequence of a sequence located in the junction region of sequences A and C and that is formed when sequences A and C are ligated in step b).

5. The method according to claim 1, wherein the at least one padlock is added in step a) in the form of a system of two different oligonucleotide molecules, wherein:
- a first molecule comprises, in the 5' to 3' direction, sequences A, B, and a first portion of sequence C (C1) and a second molecule comprises a second portion of C (C2), or
- a first molecule comprises, in the 5' to 3' direction, a first portion of sequence A (A1), sequence B and C, and a second molecule comprises a second portion of sequence A (A2), or
- a first molecule comprises, in the 5' to 3' direction, a first portion of sequence A (A1), sequence B and a first portion of C (C1), and a second molecule comprising, in the 5' to 3' direction, a second portion of sequence C (C2) and a second portion of sequence A (A2), and
wherein the nucleic acid structure of step a) with the padlock in a circularizable form is provided by hybridizing the first and second molecules with the target nucleic acid;
wherein the single covalently closed circular molecule in step b) is provided by ligating of the 3' end of the first molecule with the 5' end of the second molecule, and ligating the 5' end of the first molecule with the 3' end of the second molecule, and wherein the sequence-specific detection method of step d) detects a sequence that is the complementary sequence of a sequence located in one of the junction regions of the first and the second molecules and that is formed when said first and second molecules are ligated in step b).

6. The method according to claim 1, wherein the at least one padlock is added in step a) in the form of a system of three different oligonucleotide molecules, wherein:
- a first molecule comprises, in the 5' to 3' direction, sequences A, B, and a first portion of sequence C (C1); a second molecule comprises, in the 5' to 3' direction, a second portion of C (C2); and a third molecule comprises a sequence E, or
- a first molecule comprises, in the 5' to 3' direction, a first portion of sequence A (A1), sequence B and C; a second molecule comprises, in the 5' to 3' direction, a second portion of sequence A (A2); and a third molecule comprises a sequence E, or
- a first molecule comprises, in the 5' to 3' direction, a first portion of sequence A (A1) sequence B and a first portion of C (C1); a second molecule comprising, in the 5' to 3' direction, a second portion of sequence C (C2) and a second portion of A (A2); and a third molecule comprises a sequence E, and
wherein sequence E is complementary to sequence B or a region thereof, wherein the nucleic acid structure of step a) with the padlock in a circularizable form is provided by hybridizing the first and second molecules with the target nucleic acid and with the third molecule,
wherein the single covalently closed circular molecule in step b) is provided by ligating of the 3' end of the first molecule with the 5' end of the second molecule, and ligating the 5' end of the first molecule with the 3' end of the second molecule and wherein the sequence-specific detection method of step d) detects a sequence that is the complementary sequence of a sequence located in one of the junction regions of the first and the second molecules and that is formed when said first and second molecules are ligated in step b).

7. The method according to claim 1, wherein the at least one padlock is added in step a) in the form of a system of three different oligonucleotide molecules, wherein:
- a first molecule comprises, in the 5' to 3' direction, sequence A and a portion of sequence B (B1);
- a second molecule comprises, in the 5' to 3' direction, a second portion of sequence B (B2) and sequence C; and
- a third molecule comprises sequence E, wherein sequence E is complementary to the first (B1) and/or the second (B2) portions of sequence B, and
wherein the nucleic acid structure of step a) with the padlock in a circularizable form is provided by hybridizing the first and second molecules with the target nucleic acid and with the third molecule,
wherein the single covalently closed circular molecule in step b) is provided by ligating of the 3' end of the first molecule with the 5' end of the second molecule, and ligating the 5' end of the first molecule with the 3' end of the second molecule and wherein the sequence-specific detection method of step d) detects a sequence that is the complementary sequence of a sequence located in the junction region of sequences A and C and that is formed when sequences A and C are ligated in step b).

8. The method according to claim 1, wherein the at least one padlock is added in step
a) in the form of a system of four different oligonucleotide molecules, wherein:
- a first molecule comprises, in the 5' to 3' direction, sequence A and a first portion of sequence B (B1),
- a second molecule comprises, in the 5' to 3' direction, a second portion of sequence B (B2) and sequence C,
- a third molecule comprises a first portion of sequence E (E1), wherein said first portion of sequence E (E1) is complementary to the first portion of sequence B (B1) and is hybridized thereto, and
- a fourth molecule comprises a second portion of sequence E (E2), wherein said second portion of sequence E (E2) is complementary to the second portion of sequence B (B2) and is hybridized thereto, and
wherein the nucleic acid structure of step a) with the padlock in a circularizable form is provided by hybridizing the first and second molecules with the target nucleic acid and with the third and fourth molecules, respectively,
wherein the single covalently closed circular molecule in step b) is provided by ligating of the 3' end of the first molecule with the 5' end of the second molecule, and ligating the 5' end of the first molecule with the 3' end of the second molecule and wherein the sequence-specific detection method of step d) detects a sequence that is the complementary sequence of a sequence located in the junction region of sequences A and C and that is formed when sequences A and C are ligated in step b).

9. The method according to any of claims 1 to 9, wherein the ligation reaction in step b) is carried out by at least one DNA ligase.

10. The method according to any of the previous claims, wherein the sequence specific detection method is CRISPR/Cas, preferably CRISPR/Cas12a, wherein the at least one padlock comprises at least one protospacer adjacent motif (PAM), and wherein the guide RNA (gRNA) hybridizes with, and therefore targets, a sequence that is the reverse complementary to a region of the padlock adjacent to said PAM.

11. The method according to any of the previous claims, wherein target nucleic acid is a single stranded nucleic acid.

12. The method according to any of the previous claims, wherein the target nucleic acid is the genome of a virus.

13. The method according to any of the previous claims, wherein the method is performed in the absence of ammonium salt, preferably ammonium sulphate.

14. The method according to any of the previous claims, wherein the method is performed isothermally, preferably at a temperature range of 20-30°C.

15. A composition comprising at least one padlock as defined in claims 1 to 8.
